(19) Europäisches Patentamt
European Patent Office
Office européen des brevets

(11) **EP 4 114 925 B1**

(12) **EUROPEAN PATENT SPECIFICATION**

(45) Date of publication and mention
of the grant of the patent:
**05.11.2025 Bulletin 2025/45**

(51) International Patent Classification (IPC):
*C12N 5/0783* (2010.01)   *A61K 35/13* (2015.01)
*A61K 35/17* (2025.01)

(21) Application number: **21714522.6**

(22) Date of filing: **03.03.2021**

(52) Cooperative Patent Classification (CPC):
**C12N 5/0646; A61K 40/10; A61K 40/42;**
C12N 2710/24132

(86) International application number:
**PCT/US2021/020746**

(87) International publication number:
**WO 2021/178591 (10.09.2021 Gazette 2021/36)**

(54) **METHODS OF MANUFACTURING ACTIVATED IMMUNE CELLS**

VERFAHREN ZUR HERSTELLUNG AKTIVIERTER IMMUNZELLEN

PROCÉDÉS DE FABRICATION DE CELLULES IMMUNITAIRES ACTIVÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.03.2020 US 202062985237 P**

(43) Date of publication of application:
**11.01.2023 Bulletin 2023/02**

(73) Proprietor: **Bioeclipse Therapeutics, Inc.**
**Mountain View, California 94043 (US)**

(72) Inventors:
• **CONTAG, Pamela Reilly**
**San Jose, California 95129 (US)**
• **WALKER, Cameron**
**San Francisco, California 94117 (US)**
• **BHARDWAJ, Pooja**
**Sunnyvale, California 94086 (US)**

(74) Representative: **J A Kemp LLP**
**80 Turnmill Street**
**London EC1M 5QU (GB)**

(56) References cited:
**WO-A1-2020/014029    US-A1- 2013 028 875**

• **MULLER-STEINHARDT M ET AL: "Impact of storage at 22 degrees C and citrate anticoagulation on the cytokine secretion of mononuclear leukocytes", VOX SANGUINIS, S KAGER AG BASEL, CH, vol. 75, no. 1, 1 January 1998 (1998-01-01), pages 12 - 17, XP002998499, ISSN: 0042-9007**
• **BREMM MELANIE ET AL: "Improving Clinical Manufacturing of IL-15 Activated Cytokine-Induced Killer (CIK) Cells", FRONTIERS IN IMMUNOLOGY, vol. 10, 31 May 2019 (2019-05-31), CH, pages 1 - 12, XP055809778, ISSN: 1664-3224, DOI: 10.3389/fimmu.2019.01218**
• **BONANNO GIUSEPPINA ET AL: "Thymoglobulin, interferon-Î3 and interleukin-2 efficiently expand cytokine-induced killer (CIK) cells in clinical-grade cultures", JOURNAL OF TRANSLATIONAL MEDICINE, BIOMED CENTRAL, vol. 8, no. 1, 7 December 2010 (2010-12-07), pages 129, XP021088847, ISSN: 1479-5876, DOI: 10.1186/ 1479-5876-8-129**
• **THORNE S. H.: "Synergistic Antitumor Effects of Immune Cell-Viral Biotherapy", SCIENCE, vol. 311, no. 5768, 1 September 2017 (2017-09-01), US, pages 1780 - 1784, XP055810173, ISSN: 0036-8075, Retrieved from the Internet <URL:https://media.nature.com/original/ magazine-assets/d43747-020-00339-2/ d43747-020-00339-2.pdf> DOI: 10.1126/ science.1121411**

**Description**

BACKGROUND OF THE INVENTION

The complicated and coveted goal of cancer therapy is the eradication of malignant cells with only minimal effect on healthy cells. Achievement of this goal is in part due to cancer's stifling effect on the immune system that is associated with the tumor's ability to reprogram the immune system creating an immune barrier to many cancer therapies. Treating only a portion of sensitive cells within a tumor is a major cause of drug resistance and subsequent relapse of disease. High mortality rates associated with cancer are caused by the metastatic spread of tumor cells from the tissue of origin to secondary sites. Metastases are the cause of approximately 90% of cancer-associated deaths worldwide. Cancer cells are able to progress to metastatic sites by evading common immune surveillance. Immune therapies are thus intended to restore the immune system function, especially within the tumor microenvironment, to allow the identification of cancerous cells as foreign. The caveat is that most immune therapies are directed at a specific defect of our complex immune systems and the ability to precede therapy with a complete diagnosis of the specific patient immune system defect is very limited. Thus, most immune directed therapies, such as, checkpoint inhibitors, and small molecule immune modulators, have entered the arena with success only in certain welldefined groups of patients. This paradigm shift from broadly effective (but toxic) therapies to "precision medicine" was also driven in part by the success of chimeric antigen receptor (CAR) T-cell (CAR T) therapy in acute lymphocytic leukemia (ALL). This therapy is an adoptive transfer of autologous cells with an engineered T cell receptor directed to a specific ALL tumor antigen, CD19. Adoptive cell therapies including CAR T typically rely on the major histocompatibility complex (MHC) to present tumor-associated antigens that may also only be weakly expressed only on a subset of tumor cells. CAR T therapy success is an indication of the power of immune cells to drive the memory immune response. A benefit of this success was the concept of combination therapies with a CAR T and other immune molecules such as checkpoint inhibitors, renewing a focus on T, NK and T cell adoptive cell transfer as immune therapy for cancer. However, both CAR T and other immune cell therapies have been limited in widespread use mainly due to the requirement for autologous cells or tissue matched donor and expensive good manufacturing practices (GMP) manufacturing scale, and high costs to patients. These factors all seem to contribute to the limitations of the current cancer therapies to benefit patients.

Many studies have demonstrated that cytokine-induced killer (CIK) cell therapy, may be a more potent approach for killing a broad rather than narrow collection of cancer types in an MHC-independent manner. CIKs are a heterogenous cell population that retain various characteristics of effector memory T cell subpopulations, while also expressing the non-MHC-restricted killing properties of NK cells. CIK cells are derived from human peripheral blood mononuclear cells (PBMCs) during ex-vivo expansion with IFN-$\gamma$, anti-CD3 antibody, and IL-2. CIK cells express the NKG2D receptor, which directly binds to a class of stress-associated ligands expressed on the tumor cell surface (NKG2D ligands) to mediate lysis of malignant cells. Due to the heterogeneity of these stress-associated ligands, CIK cells display anti-tumor cytotoxicity against a variety of cancer types. Moreover, numerous studies have demonstrated that CIK cells also possess potent intra-tumoral homing capabilities. Indeed, pre-clinical imaging studies have demonstrated that CIK signals were principally found in the tumor site 72 hours post-intravenous delivery. Due to their homing capabilities, and MHC-unrestricted affinity for a variety of tumor types, CIK cells have immense potential as a cancer treatment and vehicle to combine therapies.

One such combination therapy utilizes CIK cells as a vehicle for the delivery of oncolytic viruses (OVs), which selectively kill cancerous cells (Thorne and Contag, 2006). In recent years, OVs have garnered significant attention as a method of cancer treatment due to their bimodal mechanism for killing tumor cells. First, the virus preferentially replicates inside tumor cells, ultimately resulting in the lysis of cancerous cells without harming healthy tissue. Additionally, OVs convert an immune suppressed tumor microenvironment into an inflamed one, as lysed cells release tumor associated antigens (TAAs) and danger-associated molecular patterns (DAMPs). While oncolytic viruses have been shown to have promising effects for directly lysing tumor cells, administration of oncolytic viruses alone have not shown any therapeutic effect in animal models as they are quickly cleared by the immune system. Due to their prolonged eclipse period when infected, CIK cells provide a vehicle to deliver oncolytic viruses payload directly to the tumor site without alerting a patient's immune system (see US Patent Nos. 9,101,658 and 10,064,893).

While adaptive cell biotherapies hold tremendous potential for treatment of a broad variety of cancers, ex-vivo expansion of patient-derived cells add complexity and cost to the generation, storage, and shipment of combination cell therapies. Considering the urgent need for treatment of patients, expanded CIK cells derived from PBMCs must be shipped from the laboratory to location of administration to the patient in a time-sensitive manner while maintaining high viability and phenotype. During this process, CIK may cells remain in a gas-impermeable fluid transfer bag. The historical view on this practice led to the belief that CIK cells must be infused in under 6 hours and thus manufactured in close proximity (Laport, GG et al., 2012, Biol Blood Marrow Transplant. 17(11):1679-1687). That viewpoint was then transferred over to the clinical setting as we prepare to infuse CIK cells that are infected with an attenuated oncolytic virus, such as vaccinia virus, which then must be immediately back to the patient. Given the personalized nature of autologous cell therapy for cancer treatment, storage and shipment methods are critically important to ensure that expanded CIK cells and oncolytic virus reach the patient in a time- and cost-effective manner. Various studies have indicated that even small temperature fluctuations during storage affect viability and activity of immune cells stored at low temperature. Moreover, it has been shown that storage of oncolytic viruses is also highly temperature-dependent, as storage at room temperature directly decreased titer of a vaccinia-virus vaccine.

Adaptive cell therapies hold enormous potential for the treatment of a broad set of cancer types. However, high costs associated with clinical storage and shipping threaten to increase cost and time associated with delivering the therapy to patient. There exists a need for optimized storage and delivery conditions of expanded immune cells to allow for distant manufacturing and greater manufacturing options. The present invention addresses each of these currently unmet needs.

Bremm et al., Front. Immunol., 10:1218 (2019) discusses cryopreservation of CIK cells.
Bonanno et al., J. Transl. Med., 8:129 (2010) discloses the use of thymoglobulin in the generation of CIK cells.
US 2013/028875 A1 discloses compositions and methods for the treatment of cancer involving immune effector cells pre-infected with oncolytic virus.
Thorne et al., Science, 311(5768):1780-4 (2006) discusses targeted biological therapies including CIK cells pre-infected with an oncolytic virus for the treatment of tumors.
WO 2020/014029 A1 discloses methods and compositions for culturing CIK cells.

BRIEF SUMMARY OF THE INVENTION

The invention is defined by the claims. Further aspects and preferred embodiments are defined in the dependent claims. Any aspects, embodiments and examples of the present disclosure which do not fall under the scope of the appended claims do not form part of the claimed invention and are merely provided for illustrative purpose.

The references to methods of treatment in paragraphs [0026] - [0029], [0078], [0090], [0093], [0101], [0108], [0115], [0121], [0122], [0139], [0151] - [0172], and [0178] of this description are to be interpreted as references to the compounds, pharmaceutical compositions and medicaments of the present invention for use in a method for treatment of the human (or animal) body by therapy (or diagnosis).

The invention provides a method of producing a stabilized population of immune cells comprising cytokine-induced killer (CIK) cells, wherein the immune cells comprise an oncolytic virus, the method comprising

a) culturing immune cells under conditions to select and enhance cytokine-induced killer cells,
b) contacting the cells with the oncolytic virus, and
c) maintaining the cells at 2°C to 8°C for more than 6 hours and up to 120 hours.

The stabilized population of immune cells comprise cytokine-induced killer (CIK) cells. In some embodiments, the CIK cells are CD3+/CD56+ cells. In some embodiments, the percentage of CIK cells in the population is maintained or enhanced. In some embodiments, the stabilized population of immune cells comprise CD3+/CD56+ cells. In some embodiments, the percentage of CD3+/CD56+ cells in the population is maintained or enhanced. In some embodiments, the stabilized population of immune cells comprise NKG2D+ cells. In some embodiments, the percentage of NKG2D+ cells in the population is maintained or enhanced. In some embodiments, the percentage of CD3+/CD56+/NKG2D+ cells is maintained or enhanced. In some embodiments, the viability of the stable population of immune cells is greater than 70%. In some embodiments, the viability of cells is measured by trypan blue exclusion or by flow cytometry.

In some embodiments of the invention, the immune cells are derived from peripheral blood mononuclear cells (PBMCs). In some embodiments, the immune cells of step a) are cultured in 1 liter to 10 liters of culture media. In some embodiments, the immune cells of step a) are cultured in 1 liter or 5 liters of culture media. In some embodiments, the immune cells of step a) are cultured for 28 days, 32 days, 36 days or 40 days.

In some embodiments not forming part of the claimed invention, the cells of step c) are maintained at room temperature. In some embodiments, room temperature is about 22°C. In some embodiments, the cells of step c) are maintained at 2°C to 8°C (*e.g.,* 4°C). In some embodiments, the cells of step c) are maintained in a bag. In some embodiments, the bag is a plastic bag. In some embodiments, the bag is not gas-permeable. In some embodiments, the cells of step c) are maintained in <1% $CO_2$. In some embodiments, the cells of step c) are maintained in a salt solution. In some embodiments, the cells of step c) are maintained in a Plasma-Lyte©. In some embodiments, the salt solution is essentially free of serum and/or growth factors. In some embodiments, the cells of step c) are maintained in the dark.

Cells of the stabilized population of immune cells comprise an oncolytic virus. In some embodiments, the oncolytic virus is a vaccinia virus. In some embodiments, the vaccinia virus comprises a mutation in the viral thymidine kinase (TK) gene and/or the viral growth factor (VGF) gene. In some embodiments, the vaccinia virus comprises a mutation in the viral thymidine kinase gene. In some embodiments, the vaccinia virus comprises a mutation in the viral growth factor (VGF) gene. In some embodiments, the oncolytic virus is replication competent. In some embodiments, the vaccinia virus is an EEV virus.

In some aspects, the invention provides a method of producing a stabilized population of immune cells, wherein the immune cells comprise an oncolytic virus, the method comprising a) culturing immune cells under conditions to select and enhance natural killer T (NKT) cells, and/or natural killer (NK) cells, and/or T cells at 37°C, b) contacting the cells with the oncolytic virus, and c) removing the cells from the culture and maintaining the cells at 2°C to 8°C for up to 120 hours. In some aspects, the invention provides a method of producing a stabilized population of immune cells, wherein the immune cells comprise an oncolytic virus, the method comprising a) culturing immune cells under conditions to select and enhance natural killer T (NKT) cells, and/or natural killer (NK) cells, and/or T cells at 37°C, b) removing the cells from the culture and maintaining the cells at 2°C to room temperature for 12 hours to 36 hours, c) contacting the cells with the oncolytic virus, and d) maintaining the cells at 2°C to 8°C for 12 hours to 36 hours. In some embodiments, the cells of step b) are maintained at 2°C to room temperature for 24 hours.

Cells of the stabilized population of immune cells comprise an oncolytic virus. In some embodiments, the oncolytic virus is a vaccinia virus. In some embodiments, the vaccinia virus comprises a mutation in the viral thymidine kinase (TK) gene and/or the viral growth factor (VGF) gene. In some embodiments, the vaccinia virus comprises a mutation in the viral thymidine kinase gene. In some embodiments, the vaccinia virus comprises a mutation in the viral growth factor (VGF) gene. In some embodiments, the oncolytic virus is replication competent. In some embodiments, the vaccinia virus is an EEV virus. In some embodiments, the cells are infected with the oncolytic virus at a multiplicity of infection (MOI) of 0.1 to an MOI of 10.

The stabilized population of immune cells comprise cytokine-induced killer (CIK) cells. In some embodiments, the CIK cells are CD3+/CD56+ cells. In some embodiments, the percentage of CIK cells in the population is maintained or enhanced. In some embodiments, the stabilized population of immune cells comprise CD3+/CD56+ cells. In some embodiments, the percentage of CD3+/CD56+ cells in the population is maintained or enhanced. In some embodiments, the stabilized population of immune cells comprise NKG2D+ cells. In some embodiments, the percentage of NKG2D+ cells in the population is maintained or enhanced. In some embodiments, the percentage of CD3+/CD56+/NKG2D+ cells is maintained or enhanced. In some embodiments, the viability of the stable population of immune cells is greater than 70%. In some embodiments, the viability of cells is measured by trypan blue exclusion or by flow cytometry.

In some embodiments of the invention, the immune cells derived from peripheral blood mononuclear cells (PBMCs). In some embodiments, the immune cells of step a) are cultured in 1 liter to 10 liters of culture media. In some embodiments, the immune cells of step a) are cultured in 1 liter or 5 liters of culture media. In some embodiments, the immune cells of step a) are cultured for 7 days, 14 days, 28 days, 32 days, 36 days or 40 days.

In some embodiments of the invention, the cells are maintained at room temperature. In some embodiments, room temperature is 22°C. In some embodiments, the cells are maintained at 2°C to 8°C (*e.g.,* 4°C). In some embodiments, the cells are maintained in a bag. In some embodiments, the bag is a plastic bag. In some embodiments, the bag is not gas-permeable. In some embodiments, the cells are maintained in <1% $CO_2$. In some embodiments, the cells are maintained in a salt solution. In some embodiments, the cells are maintained in a Plasma-Lyte©. In some embodiments, the salt solution is essentially free of serum and/or growth factors. In some embodiments, the cells are maintained in the dark.

Cells of the stabilized population of immune cells comprise an oncolytic virus. In some embodiments, the oncolytic virus is a vaccinia virus. In some embodiments, the vaccinia virus comprises a mutation in the viral thymidine kinase (TK) gene and/or the viral growth factor (VGF) gene. In some embodiments, the vaccinia virus comprises a mutation in the viral thymidine kinase gene. In some embodiments, the vaccinia virus comprises a mutation in the viral growth factor (VGF) gene. In some embodiments, the oncolytic virus is replication competent. In some embodiments, the vaccinia virus is an EEV virus. In some embodiments, the cells are infected with the oncolytic virus at a multiplicity of infection (MOI) of 0.1 to an MOI of 10.

In some aspects not forming part of the claimed invention, the present disclosure provides a method of enhancing the efficacy of therapeutic immune cells, the method comprising a) culturing immune cells under conditions to select and enhance natural killer T (NKT) cells, natural killer (NK) cells and/or T cells at about 37°C, b) removing the cells from the culture and maintaining the cells at about 2°C to room temperature for up to about 120 hours to produce the enhanced immune cells. In some aspects not forming part of the claimed invention, the present disclosure provides a method of enhancing the efficacy of therapeutic immune cells, wherein the immune cells comprise an oncolytic virus, the method comprising a) culturing peripheral blood mononuclear cells (PBMCs) under conditions to select and enhance natural killer T (NKT) cells, natural killer (NK) cells and T cells at about 37°C, b) contacting the cells with the oncolytic virus, and c) removing the cells from the culture and maintaining the cells at about 2°C to room temperature for up to about 120 hours to produce the enhanced immune cells. In some aspects not forming part of the claimed invention, the present disclosure provides a method of enhancing the efficacy of therapeutic immune cells, wherein the immune cells comprise an oncolytic virus, the method comprising a) culturing peripheral blood mononuclear cells (PBMCs) under conditions to select and enhance natural killer T (NKT) cells, natural killer (NK) cells and T cells at about 37°C, b) removing the cells from the culture and maintaining the cells at about 2°C to room temperature for up to about 12-36 hours, c) contacting the cells with the oncolytic virus, and d) removing the cells from the culture and maintaining the cells at about 2°C to room temperature for up to about 12-36 hours to produce the enhanced immune cells. In some embodiments, the cells of step b) or step d) are maintained at about 2°C to about 8°C (e.g., about 4°C) for up to about 120 hours.

In some embodiments of the methods, not forming part of the claimed invention, of enhancing the efficacy of therapeutic immune cells, the oncolytic virus is a vaccinia virus. In some embodiments, the oncolytic virus is a vaccinia virus. In some embodiments, the vaccinia virus comprises a mutation in the viral thymidine kinase (TK) gene and/or the viral growth factor (VGF) gene. In some embodiments, the vaccinia virus comprises a mutation in the viral thymidine kinase gene. In some embodiments, the vaccinia virus comprises a mutation in the viral growth factor (VGF) gene. In some embodiments, the oncolytic virus is replication competent. In some embodiments, the vaccinia virus is an EEV virus. In some embodiments, the cells are infected with the oncolytic virus at a multiplicity of infection (MOI) of about 0.1 to an MOI of about 10.

In some embodiments of the methods, not forming part of the claimed invention, of enhancing the efficacy of therapeutic immune cells, the stabilized population of immune cells comprise cytokine-induced killer (CIK) cells. In some embodiments, the CIK cells is a CD3+/CD56+ cell. In some embodiments, the percentage of CIK cells in the population is maintained or enhanced. In some embodiments, the stabilized population of immune cells comprise CD3+/CD56+ cells. In some embodiments, the percentage of CD3+/CD56+ cells in the population is maintained or enhanced. In some embodiments, the stabilized population of immune cells comprise NKG2D+ cells. In some embodiments, the percentage of NKG2D+ cells in the population is maintained or enhanced. In some embodiments, the percentage of CD3+/CD56+/NKG2D+ cells is maintained or enhanced. In some embodiments, the viability of the stable population of immune cells is greater than about 70%. In some embodiments, the viability of cells is measured by trypan blue exclusion or by flow cytometry.

In some embodiments of the methods, not forming part of the claimed invention, of enhancing the efficacy of therapeutic immune cells, the immune cells are derived from peripheral blood mononuclear cells (PBMCs). In some embodiments, the immune cells of step a) are cultured in about 1 liter to about 10 liters of culture media. In some embodiments, the immune cells of step a) are cultured in about 1 liter or about 5 liters of culture media. In some embodiments, the immune cells of step a) are cultured for about 7 days, about 14 days, about 28 days, about 32 days, about 36 days or about 40 days.

In some embodiments of the methods, not forming part of the claimed invention, of enhancing the efficacy of therapeutic immune cells, the cells are maintained at room temperature. In some embodiments, room temperature is about 22°C. In some embodiments, the cells are maintained at about 2°C to about 8°C (e.g., about 4°C). In some embodiments, the cells of step b) are maintained in a bag. In some embodiments, the bag is a plastic bag. In some embodiments, the bag is not gas-permeable. In some embodiments, the cells are maintained in <1% $CO_2$. In some embodiments, the cells are maintained in a salt solution. In some embodiments, the cells are maintained in a Plasma-Lyte©. In some embodiments, the salt solution is essentially free of serum and/or growth factors. In some embodiments, the cells are maintained in the dark.

In some aspects not forming part of the claimed invention, the present disclosure provides a composition of stable immune cells prepared by the methods described herein. In some embodiments, the composition comprises one of more pharmaceutically accepted excipients.

In some aspects not forming part of the claimed invention, the present disclosure provides a method of treating cancer in an individual, the method comprising administering to the individual an effective amount of a stabilized population of immune cells comprising an oncolytic virus, wherein the stabilized population of immune cells is prepared by a method comprising a) culturing immune cells under conditions to select and enhance natural killer T (NKT) cells, and/or natural killer (NK) cells, and/or T cells at about 37°C, b) contacting the cells with the oncolytic virus, and c) removing the cells from the culture and maintaining the cells at about 2°C to room temperature for up to about 120 hours. In some aspects not forming part of the claimed invention, the present disclosure provides a method of treating cancer in an individual, the method comprising administering to the individual an effective amount of a stabilized population of immune cells comprising an oncolytic virus, wherein the stabilized population of immune cells is prepared by a method comprising a) culturing immune cells under conditions to select and enhance natural killer T (NKT) cells, natural killer (NK) cells and T cells at about 37°C, b) removing the cells from the culture and maintaining the cells at about 2°C to about room temperature about 12 hours to about 120 hours, c) contacting the cells with the oncolytic virus, and d) removing the cells from the culture and maintaining the cells at about 2°C to room temperature (e.g., about 4°C) for up to about 120 hours. In some embodiments, the oncolytic virus remains in the immune cell until the immune cell interacts with a tumor in the individual. In some embodiments, the stabilized population of immune cells is administered to the individual during the eclipse period of the oncolytic virus. In some embodiments, the immune cells are autologous cells. In some embodiments, the immune cells are allogeneic cells. In some embodiments, the stabilized population of immune cells is administered by intravascular, subcutaneous, intraperitoneal, or intratumor injection. In some embodiments, the cells of step b) are maintained at about 2°C to about room temperature for about 24 hours.

In some embodiments of the methods of treatment not forming part of the claimed invention, the oncolytic virus is a vaccinia virus. In some embodiments, the vaccinia virus comprises a mutation in the viral thymidine kinase (TK) gene and/or the viral growth factor (VGF) gene. In some embodiments, the vaccinia virus comprises a mutation in the viral thymidine kinase gene. In some embodiments, the vaccinia virus comprises a mutation in the viral growth factor (VGF) gene. In some embodiments, the oncolytic virus is replication competent. In some embodiments, the vaccinia virus is an EEV virus. In some embodiments, the cells are infected with the oncolytic virus at a multiplicity of infection (MOI) of about 0.1 to an MOI of about 10.

In some embodiments of the methods of treatment not forming part of the claimed invention, the stabilized population of immune cells comprise cytokine-induced killer (CIK) cells. In some embodiments, the CIK cells is a CD3+/CD56+ cell. In some embodiments, the percentage of CIK cells in the population is maintained or enhanced. In some embodiments, the stabilized population of immune cells comprise CD3+/CD56+ cells. In some embodiments, the percentage of CD3+/CD56+ cells in the population is maintained or enhanced. In some embodiments, the stabilized population of immune cells comprise NKG2D+ cells. In some embodiments, the percentage of NKG2D+ cells in the population is maintained or enhanced. In some embodiments, the percentage of CD3+/CD56+/NKG2D+ cells is maintained or enhanced. In some embodiments, the viability of the stable population of immune cells is greater than about 70%. In some embodiments, the viability of cells is measured by trypan blue exclusion or by flow cytometry.

In some embodiments of the methods of treatment not forming part of the claimed invention, the cells are maintained at room temperature. In some embodiments, room temperature is about 22°C. In some embodiments, the cells are maintained at about 2°C to about 8°C (e.g., about 4°C). In some embodiments, the cells are maintained in a bag. In some embodiments, the bag is a plastic bag. In some embodiments, the bag is not gas-permeable. In some embodiments, the cells are maintained in <1% $CO_2$. In some embodiments, the cells are maintained in a salt solution. In some embodiments, the cells are maintained in a Plasma-Lyte©. In some embodiments, the salt solution is essentially free of serum and/or growth factors. In some embodiments, the cells are maintained in the dark.

BRIEF DESCRIPTION OF THE DRAWINGS

FIG. 1 shows a manufacturing process plan.

FIG. 2 is a graph showing a CIK cell substance hold study at room temperatures. The Trypan Blue viability (circles), Sytox Blue flow cytometry viability (diamonds), and viable cell density (squares) for cells stored at room temperature.

FIG. 3 is a graph showing a CIK cell substance hold study at 37°C. The Trypan Blue viability (circles), Sytox Blue flow cytometry viability (diamonds), and viable cell density (squares) for cells stored at 37°C.

FIG. 4 is a graph showing a CIK cell substance hold study at room temperature. Viability was measured by Trypan Blue staining and counting in a hemocytometer.

FIG. 5 is a graph showing a CIK cell substance hold study at room temperature. Viability was measured by flow cytometry.

FIG. 6 is a graph showing a CIK cell substance hold study at 37°C.

FIG. 7 is a graph showing a CIK cell substance hold study at room temperature. Cells were infected with a low dose of vvDD. Viability was measured by Trypan Blue staining.

FIG. 8 is a graph showing a CIK cell substance hold study at room temperature. Cells were infected with a high dose of vvDD. Viability was measured by Trypan Blue staining.

FIG. 9 is a graph showing a CIK cell substance hold study at 37°C. Cells were infected with a high dose of vvDD. Viability was measured by flow cytometry.

FIG. 10 is a graph showing percentage of CIK cell substance positive for the presence of CD107a surface marker at 0 and 24 hours incubation at 37°C after overnight shipment in a gas impermeable bag. Degranulation marker (CD107a) was assessed as a marker for cytotoxicity.

FIGs. 11A-11C are graphs showing cell growth in culture bags over a 20-day period. FIG. 11A shows cell concentration. FIG. 11B shows % viability. FIG. 11C shows total number of cells.

FIGs. 12A-12C are graphs showing the percentage of cells in culture bags over a 20-day period that are CD3+ cells (FIG. 12A), CD3+/CD56+ cells (CIK cells; FIG. 12B), and CD3-/CD56+ cells (NK cells; FIG. 12C).

FIGs. 13A-13D are graphs showing the percentage of CD314+ cells among various cell types in culture bags over a 20-day period. FIG. 13A is a graph showing the percentage of CD314+ cells among all lymphocytes. FIG. 13B is a graph showing the percentage of CD314+ cells among CD3+/CD56- T cells. FIG. 13C is a graph showing the percentage of CD314+ cells among CIK CD3+/CD56+ cells. FIG. 13D is a graph showing the percentage of CD314+ cells among CD3-/CD56+ NK cells.

FIGs. 14A and 14B are graphs showing viability of cells during holds at 4°C, room temperature or 37°C. FIG. 14A shows viability up to 48 hours of hold. FIG. 14B shows viability up to 115 hours of hold.

FIGs. 15A and 15B are graphs showing cell concentrations during holds at 4°C, room temperature or 37°C. FIG. 15A shows cell concentrations up to 48 hours of hold. FIG. 15B shows cell concentrations up to 115 hours of hold.

FIGs. 16A-16D are graphs showing the percentage of cells following 115 hours of hold at 4°C, room temperature or 37°C. Cells monitored were CD3+ cells (FIG. 16A), CD3+/CD56+ cells (CIK cells; FIG. 16B), CD3+/CD56- cells (FIG. 16C), and CD3-/CD56+ cells (NK cells; FIG. 16D).

FIGs. 17A-17D are graphs showing the percentage of CD314+ cells among various cell types over 115 hours hold. FIG. 17A is a graph showing the percentage of CD314+ cells among all lymphocytes. FIG. 17B is a graph showing the percentage of CD314+ cells among CD3+/CD56- T cells. FIG. 17C is a graph showing the percentage of CD314+ cells among CIK CD3+/CD56+ cells. FIG. 17D is a graph showing the percentage of CD314+ cells among CD3-/CD56+ NK cells.

FIGs. 18A and 18B show the percent viability (FIG. 18A) and cell concentration (cells/mL) (FIG. 18B) assessment of CIK and CRX100 bags at 4°C and 22°C. Assessment was made using a TC-20 automated cell counter. Percent viability is determined as: (Viable Cell Count ÷ Total Cell Count) × 100%.

FIG. 19 shows the extrapolated total cell count. Cell concentration (FIG. 18B) was multiplied by a volume of 350 mL to obtain an extrapolated total cell population.

FIGs. 20A-20D show cell subpopulation characterization of CIK and CRX100 at 4°C and 22°C temperatures by flow cytometry. FIG. 20A is the % of total CD3+ cells, FIG. 20B is the % of CD3-/CD56+ NK cells, FIG. 20C is the % of CD3+/CD56- T cells, and FIG. 20D % of CD3+/CD56+ CIK cells.

FIGs. 21A-21D show CD314 characterization by flow cytometry. FIG. 21A shows % CD314+ of CD3+/CD56- cells, FIG. 21B shows % CD314+ of CD3+ T cells, FIG. 21C shows % CD314+ of CD3+/CD56+ CIK Cells, and FIG. 21D shows % CD314+ of CD3-/CD56+ NK Cells.

FIGs. 22A-22C show total cell counts segregated by flow subpopulations. FIG. 22A shows total CD3+ Cell Count. FIG. 22B shows total CD3+/CD56+ CIK cell count. FIG. 22C shows total CD314+/CD3+/CD56+ cell count.

FIGs. 23A and 23B show viral shedding profiles of cell-associated (cell pellet) and cell-free virus (supernatant) as assessed by plaque assay on Vero E6 cells at 4°C (FIG. 23A) and 22°C (FIG. 23B).

FIGs. 24A and 24B show the potency assessment of CRX100 & CIK Cells on OVCAR-3 tumor cells. Percent viability of OVCAR-3 is shown in FIG. 24A. Viable cell count of OVCAR-3 is shown in FIG. 24B.

FIG. 25 shows a potency comparison of CRX100 freshly washed vs. held at 4°C for 120 hours vs. held at 22°C for 120 hours.

FIG. 26 is a graph indicating the potency of CRX100 and CIK cells formulated within GMP facilities from human patient apheresis product on OVCAR-3 tumor cells. Potency is shown by percent of tumor cells killed. The E:T ratio for CRX100 and CIK cells describes the ratio of effector cells to tumor cells. The E:T ratio for vvDD describes the ratio of virus pfu to target cells.

FIG. 27 is a graph indicating the potency of CRX100 and CIK cells formulated within GMP facilities from human patient apheresis product on OVCAR-3 cells. Potency is shown by percent of tumor cells killed. The E:T ratio for CIK cells (top axis) describes the ratio of effector cells to tumor cells. The E:T ratio for vvDD and CRX100 describes the ratio of virus pfu to target cells. vvDD pfu in CRX100 was determined to be the product of MOI, cell count, and percent of cell-associated virus as determined by plaque assay (75%).

FIG. 28 is a graph indicating the cell-associated viral titer profile of CRX100 cells held at different temperatures (37°C, 22°C, and 4°C) in Plasma-Lyte©.

FIG. 29 is a graph indicating the cell-free viral titer profile of CRX100 cells held at different temperatures (37°C, 22°C, and 4°C) in Plasma-Lyte©.

DETAILED DESCRIPTION OF THE INVENTION

The present disclosure provides methods not forming part of the claimed invention to extend the viability of immune cells that have been pre-infected with an oncolytic virus. In some embodiments not forming part of the claimed invention, the effector cells are T cells but may include other subsets including but not limited to NK cells, and NKT cells. Immune cells are infected with an oncolytic virus and stored in a gas-impermeable bag and stored in the dark at ambient room temperature (22°C ± 5°C) or at 2-8°C. Oncolytic viruses of interest may be replication-selective or tropism-modified viruses, that are only capable of completing a successful infection in transformed cells. Pre-infection of immune cells prior to storage at room temperature 2-8°C or pre-infection of immune cells 24 hours after storage at room temperature or 2-8°C resulted in sustained viability of expanded immune cells and a higher percentage of viable CD3+/CD56+ T cells compared to cells stored at 37°C.

Pre-infection of immune cells, specifically CD3+/CD56+ CIK cells, with an oncolytic virus and prolonged storage at room temperature (22°C ± 5°C) or at 2-8°C resulted in the upregulation of NKG2D (CD314), which enhances their ability to bind and lyse tumor cells. Expanded immune cell populations were observed to have a greater percentage of CD314+ cells after storage in a gas impermeable bags at room temperature (22°C ± 5°C).

In some aspects not forming part of the claimed invention, the present disclosure provides methods of producing a stabilized population of immune cells, the method comprising a) culturing immune cells under conditions to select and enhance natural killer T (NKT) cells, natural killer (NK) cells and/or T cells at about 37°C, b) removing the cells from the culture and maintaining the cells at room temperature or 2-8°C for up to about 72 hours, thereby generating the stabilized population of immune cells. In some aspects, the invention provides a method of producing a stabilized population of immune cells, wherein the immune cells comprise an oncolytic virus, the method comprising a) culturing peripheral blood mononuclear cells (PBMCs) under conditions to select and enhance natural killer T (NKT) cells, natural killer (NK) cells and T cells at 37°C, b) contacting the cells with the oncolytic virus, and c) removing the cells from the culture and maintaining the cells at 2-8°C for up to 72 hours. In some aspects, the invention provides a method of producing a stabilized population of immune cells, wherein the immune cells comprise an oncolytic virus, the method comprising a) culturing peripheral blood mononuclear cells (PBMCs) under conditions to select and enhance natural killer T (NKT) cells, natural killer (NK) cells and T cells at 37°C, b) removing the cells from the culture and maintaining the cells at room temperature or 2-8°C for 24 hours, c) contacting the cells with the oncolytic virus, and d) maintaining the cells at 2-8°C for up to 24-48 hours. In some embodiments, the oncolytic virus is a vaccinia virus. In some embodiments, the vaccinia virus comprises a mutation in the viral thymidine kinase (TK) gene and/or the viral growth factor (VGF) gene.

In some aspects not forming part of the claimed invention, the present disclosure provides a method of enhancing the efficacy of therapeutic immune cells, the method comprising a) culturing immune cells under conditions to select and enhance natural killer T (NKT) cells, natural killer (NK) cells and/or T cells at about 37°C, b) removing the cells from the culture and maintaining the cells at room temperature or 2-8°C for up to about 72 hours. In some aspects not forming part of the claimed invention, the present disclosure provides a method of enhancing the efficacy of therapeutic immune cells, wherein the immune cells comprise an oncolytic virus, the method comprising a) culturing peripheral blood mononuclear cells (PBMCs) under conditions to select and enhance natural killer T (NKT) cells, natural killer (NK) cells and T cells at about 37°C, b) contacting the cells with the oncolytic virus, and c) removing the cells from the culture and maintaining the cells at room temperature for up to about 72 hours. In some aspects not forming part of the claimed invention, the present disclosure provides a method of enhancing the efficacy of therapeutic immune cells, wherein the immune cells comprise an oncolytic virus, the method comprising a) culturing peripheral blood mononuclear cells (PBMCs) under conditions to select and enhance natural killer T (NKT) cells, natural killer (NK) cells and T cells at about 37°C, b) removing the cells from the culture and maintaining the cells at room temperature or 2-8°C for about 24 hours, c) contacting the cells with the oncolytic virus, and d) maintaining the cells at room temperature or 2-8°C for up to about 24-48 hours. In some embodiments, the oncolytic virus is a vaccinia virus. In some embodiments, the vaccinia virus comprises a mutation in the viral thymidine kinase (TK) gene and/or the viral growth factor (VGF) gene.

The practice of the present invention will employ, unless otherwise indicated, conventional techniques of

EP 4 114 925 B1

molecular biology (including recombinant techniques), microbiology, cell biology, biochemistry and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Handbook of Experimental Immunology" (D. M. Weir & C. C. Blackwell, eds.); "Gene Transfer Vectors for Mammalian Cells" (J. M. Miller & M. P. Calos, eds., 1987); "Current Protocols in Molecular Biology" (F. M. Ausubel et al., eds., 1987); "PCR: The Polymerase Chain Reaction", (Mullis et al., eds., 1994); and "Current Protocols in Immunology" (J. E. Coligan et al., eds., 1991).

It is understood that aspect and embodiments of the invention described herein include "consisting" and/or "consisting essentially of" aspects and embodiments. As used herein, the singular form "a", "an", and "the" includes plural references unless indicated otherwise.

In this application, the use of "or" means "and/or" unless expressly stated or understood by one skilled in the art. In the context of a multiple dependent claim, the use of "or" refers back to more than one preceding independent or dependent claim.

As is understood by one skilled in the art, reference to "about" a value or parameter herein includes (and describes) embodiments that are directed to that value or parameter per se. For example, description referring to "about X" includes description of "X".

As used herein, a "target cell" is a tumor cell in which the virus replicates. Usually a target cell is a mammalian cell; for example, a human cell.

A "host cell" includes an individual cell or cell culture which can be or has been a recipient of any virus and/or virus vector(s) of this invention. Host cells include progeny of a single host cell, and the progeny may not necessarily be completely identical (in morphology or of total DNA complement) to the original parent cell due to natural, accidental, or deliberate mutation and/or change. A host cell includes cells transfected or infected in vivo with a polynucleotide and/or a vector of this invention.

The term "gene" is well understood in the art and is a polynucleotide encoding a polypeptide. In addition to the polypeptide coding regions, a gene includes non-coding regions including, but not limited to, introns, transcribed but untranslated segments, and regulatory elements upstream and downstream of the coding segments.

"Replication" and "propagation" are used interchangeably and refer to the ability of a virus to reproduce or proliferate. This term is well understood in the art. For purposes of this invention, replication involves production of virus proteins and is generally directed to replication of the virus genome. Replication can be measured using assays standard in the art and described herein, such as a burst assay or plaque assay. "Replication" and "propagation" include any activity directly or indirectly involved in the process of virus manufacture, including, but not limited to, viral gene expression, production of viral proteins, nucleic acids or other components, packaging of viral components into complete viruses, and cell lysis.

The terms "nucleic acid molecule", "nucleic acid" and "polynucleotide" may be used interchangeably, and refer to a polymer of nucleotides. Such polymers of nucleotides may contain natural and/or non-natural nucleotides, and include, but are not limited to, DNA, RNA, and PNA. "Nucleic acid sequence" refers to the linear sequence of nucleotides that comprise the nucleic acid molecule or polynucleotide.

The terms "polypeptide" and "protein" are used interchangeably to refer to a polymer of amino acid residues and are not limited to a minimum length. Such polymers of amino acid residues may contain natural or non-natural amino acid residues, and include, but are not limited to, peptides, oligopeptides, dimers, trimers, and multimers of amino acid residues. Both fulllength proteins and fragments thereof are encompassed by the definition. The terms also include post-expression modifications of the polypeptide, for example, glycosylation, sialylation, acetylation, phosphorylation, and the like. Furthermore, for purposes of the present invention, a "polypeptide" refers to a protein which includes modifications, such as deletions, additions, and substitutions (generally conservative in nature), to the native sequence, as long as the protein maintains the desired activity. These modifications may be deliberate, as through site-directed mutagenesis, or may be accidental, such as through mutations of hosts which produce the proteins or errors due to PCR amplification.

As used herein, "cytotoxicity" is a term well understood in the art and refers to a state in which one or more of a cell's usual biochemical or biological functions are perturbed. These activities include, but are not limited to, metabolism, cellular replication, DNA replication, transcription, translation, and uptake of molecules. "Cytotoxicity" includes cell death and/or cytolysis. Assays are known in the art which indicate cytotoxicity, such as dye exclusion, $^3$H-thymidine uptake, and plaque assays. The term "selective cytotoxicity", as used herein, refers to the cytotoxicity conferred by a virus on a target cell, compared to the cytotoxicity conferred by a virus on a non-permissive cell. Such cytotoxicity may be measured, for example, by plaque assays, reduction or stabilization in size of a tumor comprising target cells, or the reduction or stabilization of serum levels of a marker characteristic of the tumor cells or a tissue-specific marker, *e.g.,* a cancer marker such as prostate specific antigen.

A "biological sample" encompasses a variety of sample types obtained from an individual and can be used in a diagnostic or monitoring assay. The definition encompasses blood and other liquid samples of biological origin, solid tissue samples such as a biopsy specimen or tissue cultures or cells derived therefrom and the progeny thereof. The definition

9

also includes samples that have been manipulated in any way after their procurement, such as by treatment with reagents, solubilization, or enrichment for certain components, such as proteins or polynucleotides. The term "biological sample" encompasses a clinical sample, and also includes cells in culture, cell supernatants, cell lysates, serum, plasma, biological fluid, and tissue samples.

The terms "individual" or "subject" are used interchangeably herein to refer to an animal; for example, a mammal. In some embodiments, methods of treating mammals, including, but not limited to, humans, rodents, simians, felines, canines, equines, bovines, porcines, ovines, caprines, mammalian laboratory animals, mammalian farm animals, mammalian sport animals, and mammalian pets, are provided. In some examples, an "individual" or "subject" refers to an individual or subject in need of treatment for a disease or disorder.

A "disease" or "disorder" as used herein refers to a condition where treatment is needed.

"Cancer" and "tumor," as used herein, are interchangeable terms that refer to any abnormal cell or tissue growth or proliferation in an animal. As used herein, the terms "cancer" and "tumor" encompass solid and hematological/lymphatic cancers and also encompass malignant, pre-malignant, and benign growth, such as dysplasia. Examples of cancer include but are not limited to, carcinoma, lymphoma, blastoma, sarcoma, and leukemia.

As used herein, "treatment" is an approach for obtaining beneficial or desired clinical results. "Treatment" as used herein, covers any administration or application of a therapeutic for disease in a mammal, including a human. For purposes of the present disclosure, beneficial or desired clinical results include, but are not limited to, any one or more of: alleviation of one or more symptoms, diminishment of extent of disease, preventing or delaying spread (*e.g.,* metastasis, for example metastasis to the lung or to the lymph node) of disease, preventing or delaying recurrence of disease, delay or slowing of disease progression, amelioration of the disease state, inhibiting the disease or progression of the disease, inhibiting or slowing the disease or its progression, arresting its development, and remission (whether partial or total). Also encompassed by "treatment" is a reduction of pathological consequence of a proliferative disease. The methods of the present disclosure, not forming part of the claimed invention, contemplate any one or more of these aspects of treatment.

In the context of cancer, the term "treating" includes any or all of: inhibiting growth of cancer cells, inhibiting replication of cancer cells, lessening of overall tumor burden and ameliorating one or more symptoms associated with the disease.

An "effective amount" of an agent refers to an amount effective, at dosages and for periods of time necessary, to achieve the desired therapeutic or prophylactic result.

A "therapeutically effective amount" of a substance/molecule of the present disclosure, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A therapeutically effective amount may be delivered in one or more administrations.

A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount will be less than the therapeutically effective amount.

The terms "pharmaceutical formulation" and "pharmaceutical composition" refer to a preparation which is in such form as to permit the biological activity of the active ingredient(s) to be effective, and which contains no additional components which are unacceptably toxic to a subject to which the formulation would be administered. Such formulations may be sterile.

A "pharmaceutically acceptable carrier" refers to a non-toxic solid, semisolid, or liquid filler, diluent, encapsulating material, formulation auxiliary, or carrier conventional in the art for use with a therapeutic agent that together comprise a "pharmaceutical composition" for administration to a subject. A pharmaceutically acceptable carrier is non-toxic to recipients at the dosages and concentrations employed and is compatible with other ingredients of the formulation. The pharmaceutically acceptable carrier is appropriate for the formulation employed.

Oncolytic viruses are viruses, which, when brought into contact with tumor cells, are capable of killing those cells. Viruses of interest are replication-selective or tropism modified for tumor cells, and often have been genetically modified to reduce replication capability in nontransformed cells. Viruses of interest include adenovirus; herpes simplex virus type-1; vaccinia virus; parvovirus; reovirus; Newcastle disease virus; and the like. Vaccinia virus is of particular interest.

As used herein, "virus" refers to the virus itself or derivatives thereof. The term covers all serotypes and subtypes and both naturally occurring and recombinant forms, except where otherwise indicated. A viral vector of the present disclosure can be in any of several forms, including, but not limited to, naked viral genomic DNA; a viral genome encapsulated in an virus coat; packaged in another viral or viral-like form (such as herpes simplex virus and AAV); encapsulated in a liposome; complexed with polylysine or other biocompatible polymer; complexed with synthetic polycationic molecules; conjugated with transferrin; complexed with compounds such as PEG to immunologically "mask" the molecule and/or increase half-life, or conjugated to a non-viral protein. For purposes of this disclosure, virus vectors are replicationcompetent in a target tumor cell.

**Oncolytic Viruses**

Mechanistic approaches to tumor-selective replication include the use of viruses with inherent tumor selectivity (for example, Newcastle disease virus (NDV), reovirus, vesicular stomatitis virus (VSV) and autonomous parvovirus); deletion of entire genes (herpes simplex virus (HSV), adenovirus and vaccinia virus) or functional gene regions (adenovirus and poliovirus) that are necessary for efficient replication and/or toxicity in normal cells but are expendable in tumor cells; engineering of tumor/tissue-specific promoters into viruses to limit expression of gene(s) necessary for replication to cancer cells (adenovirus and HSV); and modification of the viral coat to selectively target uptake to tumor cells (adenovirus and poliovirus).

Specific modifications known in the art include modifications to the virus for tumor selectivity include deletion of viral genes necessary for replication in normal cells but not tumor cells, *e.g.* adenovirus E1B 55 kD deletion; HSV-1 ribonucleotide reductase subunit disruption; adenovirus E1A CR1 or CR2 deletion; poliovirus PV1 deletion; *etc.* Tumor specific promoter element may be used to drive expression of early viral genes, *e.g.* adenovirus E1A under the control of alpha fetoprotein promoter/enhancer; adenovirus E1A or E1B under the control of PSA promoter/enhancer; adenovirus E1A under the control of alpha fetoprotein promoter/enhancer; HSV-1 ICP4 gene under the control of albumin promoter/enhancer; *etc.* The virus may be engineered to express a tumor-selective receptor in the virus coat, *e.g.* adenovirus having a replacement of CAR/integrin-binding with a tumor targeting ligand; and the like.

The oncolytic virus may further comprise a coding sequence or coding sequences for a protein or proteins to be administered to the patient. In some embodiments, such proteins are those that are cytotoxic or cytostatic for tumor cells, *e.g.* tumor necrosis factor (TNF), interleukin-2 (IL-2), drugs that can be utilized in an effective chemotherapeutic regime, such as multiple drug resistant protein. The DNA encoding such active protein ingredient may be obtained from any convenient source and, depending on the protein chosen, can be synthesized chemically, recovered from a cDNA library, isolated from genomic DNA, or otherwise obtained by means known in the art.

Proteins of interest include any protein(s) which has a desired effect on an infected cell in the subject to be treated. Advantages of the drug delivery system of the present disclosure, not forming part of the claimed invention, are experienced especially when the protein operates within the cytoplasm of a target cell or is released from an infected or lysed cell to induce a cytotoxic or cytostatic effect on neighboring cells (bystander effect). For example, tumor necrosis factor (TNF) is capable of selectively killing tumor cells, but needs to transit the cell membrane to exert its effect. Other proteins, such as ribotoxins and the various colonystimulating factors, also operate intracellularly.

It will be appreciated that viruses constructed to express two genes may include two proteins that have prophylactic or therapeutic value, or one gene could express a dominate selectable marker which would facilitate identifying cells transfected with the two gene construct. An example of a selectable marker would be resistance to G418 that is conferred on cells by the presence of the neomycin gene sequences. Alternatively, a second gene may be used for imaging purposes, for example the Sodium Iodide symporter (NIS) gene or somatostatin receptor gene may be expressed in order to locate and quantify viral gene expression by delivery of a radiolabeled tracer followed by PET or SPECT imaging.

DNAs encoding the foregoing proteins are available in the art and can be obtained bracketed with linker sequences for convenient manipulation, if desired. The nature of the delivery system is such that both genomic and cDNA sequences can be used, since introns can be processed in the environment transfected by the provirus. The protein drug can be encoded in the delivery virion to specify any form of the protein desired, for example, an active form, a mature form, a fused protein, a preprotein, or a preproprotein.

Vaccinia virus is of particular interest for the methods of the present disclosure. Wild-type vaccinia virus is well tolerated following both intra-tumoral and intravesical treatment, and vaccinia viruses expressing tumor-associated antigens or proinflammatory cytokines have been well tolerated in a number of Phase I trials using subcutaneous, intradermal or intra-tumoral inoculation. Vaccinia, unlike most viruses proposed for use in virotherapy, has the advantage of some demonstrated systemic delivery potential. However, since the virus is capable of infecting many different cell types, only a small fraction of the inoculum of any strain used for virotherapy may reach the tumor. Therefore, development of an effective means of delivering viruses to tumor targets is necessary for the development of effective therapy.

In one embodiment of the invention, the oncolytic virus is a vaccinia virus. All vaccinia strains tested, including the wild-type strain, show unusual replication kinetics in immune cells, where there is an initial extended eclipse period of slow replication followed by a rapid burst of replication. The eclipse phase is at least 1 day, usually at least 2 days, and not more than 4 days, usually after not more than 3 days post-infection.

A number of vaccinia virus strains have been shown to display an eclipse phase in CIK cells and may be used in the methods of the invention. The vaccinia strain Western Reserve (WR) alone or carrying deletions in the TK, VGF or both genes displayed an eclipse phase, which eclipse phase was 'enhanced' in the double deleted virus. Other vaccinia strains, including Wyeth and International Health Department (IHD) also displayed eclipse phases in CIK cells. In addition, a variety of deletion mutations in the WR backbone also displayed an eclipse phase, of note deletions that enhanced the production of the EEV (Extracellular Enveloped Virus) form of the virus and deletions that prevented the production of extracellular IFN binding proteins.

Replication of vaccinia containing a deletion of the thymidine kinase (TK) gene has been shown to be restricted to cells with elevated cellular levels of thymidine kinase, *e.g.* dividing cells and tumor cells. Vaccinia with TK deletions will replicate both in the effector cell cultures, where cell division has been stimulated by anti-CD3 antibody, and in tumor cells. Thus, in one embodiment, the oncolytic virus is a vaccinia virus comprising a genetic modification that substantially eliminates active TK expressed from the viral genome.

Viral genes whose deletion does not effect, or enhances eclipse phase include, without limitation, TK, VGF, B5R (EEV producing); B8R (IFNγ binding protein); B18R (Type I IFN binding protein). The oncolytic virus may comprise a genetic modification that substantially eliminates active viral growth factor (VGF); B5R (EEV producing); B8R (IFNγ binding protein); or B18R (Type I IFN binding protein) expressed from the viral genome. For example, the VGF gene product promotes cellular growth after secretion from infected cells, by interacting with growth factor receptors. VGF deletions have been shown to restrict viral replication to cells with mutations in the Ras/MAPK/ERK pathway, offering additional tumor selectivity. In one embodiment, the oncolytic virus is a vaccinia virus comprising a genetic modification that substantially eliminates active TK expressed from the viral genome and active VGF expressed from the viral genome.

"Genetic alteration", or "mutation", to decrease expression of genes of interest as described above, refers to any alteration to a gene wherein the expression of that gene is significantly decreased, or wherein the gene product is rendered nonfunctional, or its ability to function is significantly decreased. The term "gene" encompasses both the regions coding the gene product as well as regulatory regions for that gene, such as a promoter or enhancer. Such alterations render the product of the gene non-functional or reduce the expression of the gene such that the viral mutant has the properties of the instant invention. Moreover, the invention encompasses mutants with one or more mutation(s) in one or more gene(s) of interest. Thus, by "a" is intended one or more. For example, "a mutation in a TK gene" means that there can be one or more mutations in one or more TK genes.

Ways to achieve such alterations include any method to disrupt the expression of the product of the gene or any method to render the expressed protein nonfunctional. Numerous methods known to disrupt the expression of a gene are known, including the alterations of the coding region of the gene, or its promoter sequence in the by insertions, deletions and/or base changes. (See, Roizman and Jenkins, Science 229: 1208 (1985)).

A preferred mutation is the deletion of nucleic acids from a gene. Methods for the construction of engineered viruses and for the genetic manipulation of DNA sequences are known in the art. Generally, these include Ausubel et al., Chapter 16 in Current Protocols in Molecular Biology (John Wiley and Sons, Inc.); Paoletti et al., U.S. Pat. No. 4,603,112 (July 1986). Virological considerations also are reviewed in Coen, in Virology, 1990 (2.sup.nd ed.) Raven Press, pages 123-150.

## Immune Cells

Immune cells, for the purposes of the present disclosure, are autologous or allogeneic immune cells having cytolytic activity against tumor cells. The effector cells may have cytolytic activity that does not require recognition through the T cell antigen receptor. Cells of particular interest include cells of the T and/or NK lineage, *e.g.,* LAK cells, CIK cells, CTL, TIL cells, *etc.* The effector cells are typically obtained by culturing peripheral blood lymphocytes (PBL) in vitro with a cytokine and/or antigen combination that increases activation. In the methods of the present disclosure not forming part of the claimed invention, the activated effector cells are infected with virus, and administered to the patient. The cells are optionally separated from nondesired cells prior to culture, prior to administration, or both. Cell-mediated cytolysis of tumor cells by immunological effector cells is believed to be mediated by the local directed exocytosis of cytoplasmic granules that penetrate the cell membrane of the bound target cell.

Natural killer (NK) cells are cytotoxic cells belonging to a cell class responsible for cellular cytotoxicity without prior sensitization. IL-2-activated NK cells, the major effector population in lymphokine-activated killer (LAK) cells, are potent mediators of the lysis of autologous and allogeneic leukemic cells in vitro. LAK cells are non-B, non-T cells that are capable of recognizing cancer cells in a non-MHC-restricted fashion. LAK cells, which can be generated from either the normal or tumor-bearing host, appeared to represent a primitive immunosurveillance system capable of recognizing and destroying altered cells. NK cells often do not react with patient tumor cells unless they are activated by interferon, IL-2, or unless suppressor monocytes are removed from the effector cell population. IL-2 induces proliferation of T lymphocytes and NK cells and the production of IFN-gamma; it also results in the induction of LAK cells against previously NK-resistant cell preparations and cell lines. LAK activity can be generated from human and murine T cells following incubation with IL-2.

Most NK activity, as well as LAK activity, is mediated by the CD3- large granular lymphocyte (LGL) cell population. This lytic activity is observed against a variety of tumor cells and virally infected cells. Morphologically, NK cells are characterized as LGL cells containing a kidney-shaped nucleus and prominent azurophilic granules in their cytoplasm. Human LGL share both myelomonocytic *(e.g.,* CD11) and T cell *(e.g.,* CD2 and CD8)-related markers. However, the majority of human NK cell activity is mediated by CD3-, CD56+, and CD16+ lymphocytes, although CD16- NK cells have also been characterized. The CD16c cells also have high levels of antibody-dependent cellular cytotoxicity (ADCC). The CD16- NK cells express markers similar to CD16+ NK cells, including CD2, CD7, CD11b, CD38, CD45R, CD18, and p75 IL-2R. LAK cells have been utilized in vivo both in animals and in human beings for the treatment of melanoma, renal cell carcinoma, non-Hodgkin's lymphoma, and lung and colorectal cancers.

Cytotoxic T lymphocytes (CTL) reactive to autologous tumor cells are specific effector cells for adoptive immunotherapy. Induction and expansion of CTL is antigen-specific, and MHC restricted. Various types of cytokines other than IL-2 have also been reported to induce cytotoxic lymphocytes. A class of T lymphocytes with antitumor activity has been termed "tumor-infiltrating lymphocytes" (TIL). They possess more potent antitumor activity than LAK cells. They can be grown by culturing single-cell suspensions obtained from tumors in IL-2. Although lymphocytes comprise only a small subpopulation of the cells in a cancer nodule, some of these lymphocytes contain IL-2 receptors and grow under the influence of IL-2. Although tumor cells also grow in the culture, lymphocytes capable of eliminating the tumor cells have a selective growth advantage. After 2-3 weeks of culture, pure populations of lymphocytes without contaminating tumor cells are obtained. The major effectors of TIL cells are phenotypically CD3+CD56-CD8+ and are MHC restricted. Cancer patients have been treated with ex vivo anti-CD3-activated killer cells and IL-2.

Cytokine-induced killer (CIK) cells are highly efficient cytotoxic effector cells obtained by culturing peripheral blood lymphocytes (PBLs) in the presence of IFN-$\gamma$, IL-2 (or IL-12), and monoclonal antibody (mAb) against CD3, and optionally include IL-la. Cells may be cultured for at least about 1 week, at least about 2 weeks, at least about 3 weeks, or more, and usually not more than about 8 weeks in culture. The absolute number of CIK effector cells usually increases at least about 100-fold in such culture conditions, and may increase at least about 500-fold, at least about 1000-fold, or more.

CIK cells possess a higher level of cytotoxic activity and a higher proliferation rate than LAK cells. The phenotype of the cells with the greatest cytotoxicity expresses both the T-cell marker CD3 and the NK cell marker CD56. The expression of CD56 by the antigen is correlated with antitumor cytotoxicity. CD28, a major co-stimulatory signal of the TCR, is present on the cell surface only in a subset of CIK cells. CIK cells secrete IL-2, IL-6, and TNF-alpha. IL-4, IL-7, and IL-12 are not secreted. Most of these CD3+CD33+ cells co-expressed CD2, CD5, CD7, CD8 and HLA-DR but were negative for expression of CD4, CD13, CD14, CD15, and CD16. CD3cCD33c cells possessed no cytotoxic activity against tumor cells. CD3+CD56+ cells are expanded dramatically in CIK cell cultures. The percentage of CD3+CD56+ cells reaches a plateau after approximately 1-2 months of culture. The dominant cell phenotype in CIK cell cultures expressed the alpha-, beta-T-cell receptor (TCR-$\alpha$/$\beta$). In comparison to NK cells, the cytotoxicity mediated by CD3+CD56+ cells is also non-MHC restricted in the absence of activation, but it is non-ADCC dependent, since these doublepositive cells do not express CD16. Morphologically, these cells cannot be distinguished from NK cells.

CD314 is used herein interchangeably with NKG2D and is also known as KLRK1, KLR, and killer cell lectin like receptor K. CD314 is a transmembrane protein belonging to the CD94/NKG2 family of C-type lectin-like receptors. In humans, it is expressed by NK cells, $\gamma\delta$ T cells, CD8+ $\alpha\beta$ T cells and CIK cells.

A potent in vivo antitumor effect of CIK cells in an animal model can be achieved with as few as $1\times10^7$ cells. In humans, an effective dose is usually at least about $10^8$ cells, at least about $10^9$ cells, or at least about $10^{10}$ cells, or more. In some embodiments not forming part of the claimed invention, the effective dose is less than about $1\times10^7$, $2\times10^7$, $3\times10^7$, $4\times10^7$, $5\times10^7$, $6\times10^7$, $7\times10^7$, $8\times10^7$, $9\times10^7$, $1\times10^8$, $2\times10^8$, $3\times10^8$, $4\times10^8$, $5\times10^8$, $6\times10^8$, $7\times10^8$, $8\times10^8$, $9\times10^8$, $1\times10^9$, $2\times10^9$, $3\times10^9$, $4\times10^9$, $5\times10^9$, $6\times10^9$, $7\times10^9$, $8\times10^9$, $9\times10^9$, $1\times10^{10}$, $2\times10^{10}$, $3\times10^{10}$, $4\times10^{10}$ or $5\times10^{10}$. In some embodiments not forming part of the claimed invention, the effective dose is greater than about $5\times10^{10}$.

Immune cells useful in the methods of the present disclosure have an "eclipse" period following infection with the oncolytic virus. It has been found that cells possessing this property include, without limitation, T cell lines and CIK cells, where vaccinia displayed a clear eclipse phase in these cells in culture.

## The NKG2 Family of Proteins

NK, NKT and T cells demonstrate the ability to lyse cells that have reduced expression or have lost the ability to express the self-major histocompatibility complex (MHC) class I molecules. The cytolytic function is reported to be controlled by a balance of activating and inhibitory signals of specific NK-cell receptors. In humans, these genes were all type II transmembrane proteins with a C-type lectin domain. Two different superfamilies of these molecules were described: the p50/p58/p70 genes, which are immunoglobulin-like (Ig-like) and a second group of receptors comprises the NKG2 family. Glienke et al (1998) reported on the sequence of the four closely related transcripts of the NKG2 family: NKG2A and B which are alternative splice variants, and NKG2C, NKG2E and that the four genes are closely linked on chromosome 12 within one P1-derived artificial chromosome (PAC) clone. NKG2D (CD314) is related but with low sequence similarity (Glienke J. et al., 1998, Immunogenetics 48:163-173().

Zingoni et al (2018) review the cytolytic activity of the activating receptor NKG2D on NK, T and NKT cells and its' diverse ligands expressed on tumor cells and virally infected cells while only poorly expressed on normal cells. NKG2D ligands are upregulated in damaged, transformed or infected cells through specific cellular responses to different stimuli but all leading to the expression profile of a diverse set NKG2D ligands (from one to many types) on the cell surface. The prevailing theory is that these ligands of more than 100 different alleles for CICA, 40 for MICB and less than 20 for the ULBP's, cause the activation of NKG2D receptors uniquely shared by NK and T cells that medicate lytic activity against these stressed or infected cells. This redundancies and diversity of ligands were generated during the coevolution of the host and the pathogens (viruses being a major driver) resulting in both immune activation and immune evasion. Thus, the expression of diverse ligands drive balance between immune cell activation through receptor-ligand binding and the various tumor immune evasion strategies specifically of tumor immune evasion.

The interpretation of the preponderance of data is that NKG2D ligand diversity in the form of allelic variants of the same ligand that may differ in their avidity for NKG2D can also vary in their ability to tune the threshold of NKG2D signaling. In other words, the ligand tunes the receptor and promotes NK, NKT and T cell effector functions, especially MICA (Zingoni A. et al., 2018 March 12, Front. Immunol.).

However, in this report we have demonstrated that in the absence of NKG2D ligand on either tumor cells or virally infected cells, that immune populations under stress and infected with virus can specifically upregulate the expression of NKG2D, and increase the cytolytic activity when in the presence of tumor cells of virally infected cells.

**Methods of manufacture**

In some aspects, the present disclosure provides methods to manufacture stabilized populations of immune cells that maintain viability for up to or beyond 72 hours. These methods may be useful in production and transport of therapeutic immune cells used, for example, in the treatment of cancer. In some aspects, the populations of cells manufactured by the methods of the present disclosure have improved efficacy; for example, cells in the population have enhanced expression of NKG2D compared to cells produced by other methods.

The methods of the present disclosure not forming part of the claimed invention involve the culture of immune cells in vitro, the infection of the immune cells with an oncolytic virus, stabilization of the immune cells by holding the cells at room temperature, and the administration of the infected cells to patient suffering from cancer. The treatment is intended to reduce or eliminate cancer in the patient. Also provided but not forming part of the claimed invention are compositions of stabilized immune cells infected with an oncolytic virus, which cells may be provided in a culture medium, in aliquots suitable for delivery to a patient, and the like.

The immune cells are usually generated from a fresh or frozen hematopoietic cell population. The source of cells may be autologous or allogeneic relative to the patient, but will usually be of the same species, *e.g.* human cells for human patients, mouse cells for a mouse recipient, *etc.* Cell populations include, but are not limited to, cell populations obtained from peripheral blood, spleen, lymph node, bone marrow, mobilized peripheral blood, umbilical cord blood, *etc.*

Procedures for separation can include, but are not limited to, physical separation, magnetic separation, using antibody-coated magnetic beads, affinity chromatography, cytotoxic agents joined to a monoclonal antibody or used in conjunction with a monoclonal antibody, including, but not limited to, complement and cytotoxins, and "panning" with antibody attached to a solid matrix, *e.g.*, plate, elutriation or any other convenient technique. The use of physical separation techniques include, but are not limited to, those based on differences in physical (density gradient centrifugation and counter-flow centrifugal elutriation), cell surface (lectin and antibody affinity), and vital staining properties (mitochondria-binding dye rho123 and DNA-binding dye Hoechst 33342). These procedures are well known to those of skill in this art.

The cells are cultured as described herein followed by a hold at 2°C to 8°C to provide for a stabilized activated cell population. Such culture typically reduces the number of non-desired cells, *e.g.* alloreactive T cells, *etc.* Undesired cells may also be removed by selection. Separation of the desired cells for engraftment will generally use affinity separation. Techniques providing accurate separation include fluorescence activated cell sorters, which can have varying degrees of sophistication, such as multiple color channels, low angle and obtuse light scattering detecting channels, impedance channels, *etc.* The cells may be selected against dead cells by employing dyes associated with dead cells (propidium iodide, LDS). Any technique may be employed which is not unduly detrimental to the viability of the selected cells.

The affinity reagents may be specific receptors or ligands for cell surface molecules, *e.g.* CD8, CD4, *etc.* In addition to antibody reagents, peptide-MHC antigen and T cell receptor pairs may be used; peptide ligands and receptor; ligand and receptor molecules, and the like. Antibodies and T cell receptors may be monoclonal or polyclonal, and may be produced by transgenic animals, immunized animals, immortalized human or animal B-cells, cells transfected with DNA vectors encoding the antibody or T cell receptor, *etc.* The details of the preparation of antibodies and their suitability for use as specific binding agents are well-known to those skilled in the art.

The specific culture condition will be selected based on the type of effector cell that is desired. Where the effector cells are CIK cells, for example, the cells are washed and resuspended at in medium, *e.g.* RPMI, DME, *etc.* Interferon $\gamma$ is added to culture at an effective concentration, *e.g.* at from about 100 to about 10,000 U/ml, usually around about 1000 U/ml. A ligand specific for CD3, *e.g.* monoclonal anti-CD3 antibody is added to the culture at an effective concentration, for example OKT3 may be used at a concentration of from about 1 to about 500 ng/ml. The medium is changed at IL-2 added at regular intervals. In some embodiments, the effector cells are taken when the CIK population has expanded.

The population of effector cells is infected with the cytolytic virus. In some embodiments not forming part of the claimed invention, the virus does not cause cytolysis of the effector cells in the period of time between infection and patient administration. As described herein, an eclipse period of from about 1 day to not more than about 4 days provides a window of time where the virus does not cause significant cytolysis of the effector cells. In some embodiments, the eclipse period is any of about 1 day, about 2 days, about 3 days, about 4 days, about 5 days, about 6 days, about 7 days, about 8 days, about 9 days, or about 10 days. In some embodiments, the eclipse period is about 1 day to about 10 days, about 1 day to about 9 days, about 1 day to about 8 days, about 1 day to about 7 days, about 1 day to about 6 days, about 1 day to about 5 days, about 1 day to about 4 days, about 1 day to about 3 days, about 1 day to about 2 days, about 2 days to about 10 days, about 2 days to about 9 days, about 2 days to about 8 days, about 2 days to about 7 days, about 2 days to about 6 days, about 2 days to about 5 days, about 2 days to about 4 days, about 2 days to about 3 days, about 3 days to about 10 days, about 3 days to about 9 days, about 3 days to about 8 days, about 3 days to about 7 days, about 3 days to about 6 days, about 3 days to about 5 days, about 3 days to about 4 days, about 4 days to about 10 days, about 4 days to about 9 days, about 4 days to about 8 days, about 4 days to about 7 days, about 4 days to about 6 days, about 4 days to about 5 days, about 5 days to about 10 days, about 5 days to about 9 days, about 5 days to about 8 days, about 5 days to about 7 days, about 5 days to about 6 days, about 6 days to about 10 days, about 6 days to about 9 days, about 6 days to about 8 days, about 6 days to about 7 days, about 7 days to about 10 days, about 7 days to about 9 days, about 7 days to about 8 days, about 8 days to about 10 days, about 8 days to about 9 days, or about 9 days to about 10 days.

If used as a packaged virus, the virus may be administered in an appropriate physiologically acceptable carrier. The multiplicity of infection (MOI) will generally be in the range of about 0.001 to 100. In some embodiments not forming part of the claimed invention, the MOI is about any of 0.001, 0.005, 0.01. 0.05, 0.1, 0.5, 1.0, 5.0, 10, 50, or 100. In some embodiments not forming part of the claimed invention, the MOI is between about 0.001 and about 0.01, about 0.01 and about 0.1, about 0.1 and about 1.0, about 1.0 and about 10, or about 10 and about 100. The viruses may be administered one or more times. In some embodiments not forming part of the claimed invention, the virus is administered one, two, three, four, five or more than five times.

Alternatively, viral DNA may be used to transfect the effector cells, employing liposomes, general transfection methods that are well known in the art (such as calcium phosphate precipitation and electroporation), *etc.* Due to the high efficiency of transfection of viruses, one can achieve a high level of modified cells.

In some embodiments not forming part of the claimed invention, the present disclosure provides methods of producing a stabilized population of immune cells, the method comprising a) culturing immune cells under conditions to select and enhance natural killer T (NKT) cells, natural killer (NK) cells and/or T cells at about 37°C, b) removing the cells from the culture and maintaining the cells at about 2°C to about room temperature for up to about 72 hours, thereby generating the stabilized population of immune cells.

In some embodiments, the invention provides methods of producing a stabilized population of immune cells, wherein the immune cells comprise an oncolytic virus, the method comprising a) culturing immune cells under conditions to select and enhance NKT cells, NK cells and T cells at 37°C, b) contacting the cells with the oncolytic virus, and c) removing the cells from the culture and maintaining the cells at 2°C to 8°C for up to 72 hours.

In some embodiments, the invention provides methods of producing a stabilized population of immune cells, wherein the immune cells comprise an oncolytic virus, the method comprising a) culturing immune cells under conditions to select and enhance NKT cells, NK cells and T cells at 37°C, b) removing the cells from the culture and maintaining the cells at 2°C to room temperature for up to 12 to 36 hours, c) contacting the cells with the oncolytic virus, and d) maintaining the cells at 2°C to 8°C for up to 12 to 36 hours.

In some embodiments not forming part of the claimed invention, the present disclosure provides a manufacturing process for immune cells with enhanced efficacy. In some embodiments not forming part of the claimed invention, the present disclosure provides a method of enhancing the efficacy of therapeutic immune cells, the method comprising a) culturing immune cells under conditions to select and enhance NKT cells, NK cells and/or T cells at about 37°C, b) removing the cells from the culture and maintaining the cells at about 2°C to room temperature for up to about 72 hours. In some embodiments not forming part of the claimed invention, the present disclosure provides a method of enhancing the efficacy of therapeutic immune cells, wherein the immune cells comprise an oncolytic virus, the method comprising a) culturing peripheral blood mononuclear cells (PBMCs) under conditions to select and enhance NKT cells, NK cells and T cells at about 37°C, b) contacting the cells with the oncolytic virus, and c) removing the cells from the culture and maintaining the cells at room temperature for up to about 72 hours. In some embodiments not forming part of the claimed invention, the present disclosure provides a method of enhancing the efficacy of therapeutic immune cells, wherein the immune cells comprise an oncolytic virus, the method comprising a) culturing peripheral blood mononuclear cells (PBMCs) under conditions to select and enhance NKT cells, NK cells and T cells at about 37°C, b) removing the cells from the culture and maintaining the cells at about 2°C to about room temperature for up to about 12 to about 36 hours, c) contacting the cells with the oncolytic virus, and d) maintaining the cells at about 2°C to about room temperature for up to about 12 to about 36 hours.

In some embodiments, the invention provides methods of producing a stabilized population of immune cells as described above, the cells are removed from the culture and maintained at 2°C to room temperature for more than any of 6, 12, 18, 24, 30, 36, 42, 48, 54 60, 66, 72, 78, 84, 90, 96, 100, 120 hours or more than 120 hours. In some embodiments, the cells are removed from the culture and maintained at 2°C to room temperature for between any of 6 hours and 12 hours, 6 hours and 18 hours, 6 hours and 24 hours, 6 hours and 30 hours, 6 hours and 36 hours, 6 hours and 42 hours, 6 hours and 48 hours, 6 hours and 54 hours, 6 hours and 60 hours, 6 hours and 66 hours, 6 hours and 72 hours, 6 hours and 78 hours, 6 hours and 84 hours, or 6 hours and 90 hours. In some embodiments, the cells are removed from the culture and maintained at room temperature for between any of 12 hours and 18 hours, 12 hours and 24 hours, 12 hours and 30 hours, 12 hours and 36 hours, 12 hours and 42 hours, 12 hours and 48 hours, 12 hours and 54 hours, 12 hours and 60 hours, 12 hours and 66 hours, 12 hours and 72 hours, 12 hours and 78 hours, 12 hours and 84 hours, or 12 hours and 90 hours. In some embodiments, the cells are removed from the culture and maintained at 2°C to room temperature for between any of 18 hours and 24 hours, 18 hours and 30 hours, 18 hours and 36 hours, 18 hours and 42 hours, 18 hours and 48 hours, 18 hours and 54 hours, 18 hours and 60 hours, 18 hours and 66 hours, 18 hours and 72 hours, 18 hours and 78 hours, 18hours and 84 hours, or 18 hours and 90 hours. In some embodiments, the cells are removed from the culture and maintained at 2°C to room temperature for between any of 24 hours and 30 hours, 24 hours and 36 hours, 24 hours and 42 hours, 24 hours and 48 hours, 24 hours and 54 hours, 24 hours and 60 hours, 24 hours and 66 hours, 24 hours and 72 hours, 24 hours and 78 hours, 24 hours and 84 hours, or 24 hours and 90 hours. In some embodiments, the cells are removed from the culture and maintained 2°C to room temperature for between any of 30 hours and 36 hours, 30 hours and 42 hours, 30 hours and 48 hours, 30 hours and 54 hours, 30 hours and 60 hours, 30 hours and 66 hours, 30 hours and 72 hours, 30 hours and 78 hours, 30 hours and 84 hours, or 30 hours and 90 hours. In some embodiments, the cells are removed from the culture and maintained at 2°C to room temperature for between any of 36 hours and 42 hours, 36 hours and 48 hours, 36 hours and 54 hours, 36 hours and 66 hours, 36 hours and 66 hours, 36 hours and 72 hours, 36 hours and 78 hours, 36 hours and 84 hours, or 36 hours and 90 hours. In some embodiments, the cells are removed from the culture and maintained at 2°C to room temperature for between any of 42 hours and 48 hours, 42 hours and 54 hours, 42 hours and 60 hours, 42 hours and 66 hours, 42 hours and 72 hours, 42 hours and 78 hours, 42 hours and 84 hours, 42 hours and 90 hours, or 42 hours and 120 hours. In some embodiments, the cells are removed from the culture and maintained at 2°C to room temperature for between any of 48 hours and 54 hours, 48 hours and 60 hours, 48 hours and 66 hours, 48 hours and 72 hours, 48 hours and 78 hours, 48 hours and 84 hours, 48 hours and 90 hours, or 48 hours and 120 hours. In some embodiments, the cells are removed from the culture and maintained at 2°C to room temperature for between any of 54 hours and 60 hours, 54 hours and 66 hours, 54 hours and 72 hours, 54 hours and 78 hours, 54 hours and 84 hours, 54 hours and 90 hours, or 54 hours and 120 hours. In some embodiments, the cells are removed from the culture and maintained at 2°C to room temperature for between any of 60 hours and 66 hours, 60 hours and 72 hours, 60 hours and 78 hours, 60 hours and 84 hours, 60 hours and 90 hours, or 60 hours and 120 hours. In some embodiments, the cells are removed from the culture and maintained at 2°C to room temperature for between any of 66 hours and 72 hours, 66 hours and 78 hours, 66 hours and 84 hours, 66 hours and 90 hours, or 66 hours and 120 hours. In some embodiments, the cells are removed from the culture and maintained at 2°C to room temperature for between any of 72 hours and 78 hours, 72 hours and 84 hours, 72 hours and 90 hours, or 72 hours and 120 hours. In some embodiments, the cells are removed from the culture and maintained at 2°C to room temperature for between any of 78 hours and 84 hours, 78 hours and 90 hours, or 78 hours and 120 hours. In some embodiments, the cells are removed from the culture and maintained at 2°C to room temperature for between 84 hours and 90 hours. In some embodiments, the cells are removed from the culture and maintained at 2°C to room temperature for between 90 hours and 120 hours.

In some embodiments of the invention, the cells are removed from the culture and maintained at 2°C to room temperature for 12 hours to 36 hours prior to contacting the cells with virus. In some embodiments of the invention, the cells are removed from the culture and maintained at 2°C to room temperature for 12 hours to 36 hours prior to contacting the cells with virus and then maintained at 2°C to 8°C for 12 to 36 hours. In some embodiments, the cells are maintained at 2°C to room temperature for any one of 12, 14, 16, 18, 20, 22, 24, 26, 28, 30, 32, 34 or 36 hours prior to contacting the cells with virus. In some embodiments, the cells are maintained at 2°C to room temperature for between 12 and 36 hours, 12 and 34 hours, 12 and 32 hours, 12 and 30 hours, 12 and 28 hours, 12 and 26 hours, 12 and 24 hours, 12 and 22 hours, 12 and 20 hours, 12 and 18 hours, 12 and 16 hours, 12 and 14 hours, 14 hours and 36 hours, 14 hours and 34 hours, 14 hours and 32 hours, 14 hours and 30 hours, 14 hours and 28 hours, 14 hours and 26 hours, 14 hours and 24 hours, 14 hours and 22 hours, 14 hours and 20 hours, 14 hours and 18 hours, 14 hours and 16 hours, 16 and 36 hours, 16 and 34 hours, 16 and 32 hours, 16 and 30 hours, 16 and 28 hours, 16 and 26 hours, 16 and 24 hours, 16 and 22 hours, 16 and 20 hours, 16 and 18 hours, 18 and 36 hours, 18 and 34 hours, 18 and 32 hours, 18 and 30 hours, 18 and 28 hours, 18 and 26 hours, 18 and 24 hours, 18 and 22 hours, 18 and 20 hours, 20 hours and 36 hours, 20 hours and 34 hours, 20 hours and 32 hours, 20 hours and 30 hours, 20 hours and 28 hours, 20 hours and 26 hours, 20 hours and 24 hours, 20 hours and 22 hours, 22 hours and 36 hours, 22 hours and 34 hours, 22 hours and 32 hours, 22 hours and 30 hours, 22 hours and 28 hours, 22 hours and 26 hours, 22 hours and 24 hours, 24 hours and 36 hours, 24 hours and 34 hours, 24 hours and 32 hours, 24 hours and 30 hours, 24 hours and 28 hours, 24 hours and 26 hours, 26 hours and 36 hours, 26 hours and 34 hours, 26 hours and 32 hours, 26 hours and 30 hours, 26 hours and 28 hours, 28 hours and 36 hours, 28 hours and 34 hours, 28 hours and 32 hours, 28 hours and 30 hours, 30 hours and 36 hours, 30 hours and 34 hours, 30 hours and 32 hours, 32 hours and 36 hours, 32 hours and 34 hours, or 34 hours and 36 hours, hours prior to contacting the cells with virus. In some embodiments, the cells are maintained at 2°C to 8°C for any one of 12, 14, 16, 18, 20, 22, 24, 26, 28, 30, 32, 34 or 36 hours after contacting the cells with virus. In some embodiments, the cells are maintained 2°C to 8°C for between 12 and 36 hours, 12 and 34 hours, 12 and 32 hours, 12 and 30 hours, 12 and 28 hours, 12 and 26 hours, 12 and 24 hours, 12 and 22 hours, 12 and 20 hours, 12 and 18 hours, 12 and 16 hours, 12 and 14 hours, 14 hours and 36 hours, 14 hours and 34 hours, 14 hours and 32 hours, 14 hours and 30 hours, 14 hours and 28 hours, 14 hours and 26 hours, 14 hours and 24 hours, 14 hours and 22 hours, 14 hours and 20 hours, 14 hours and 18 hours, 14 hours and 16 hours, 16 and 36 hours, 16 and 34 hours, 16 and 32 hours, 16 and 30 hours, 16 and 28 hours, 16 and 26 hours, 16 and 24 hours, 16 and 22 hours, 16 and 20 hours, 16 and 18 hours, 18 and 36 hours, 18 and 34 hours, 18 and 32 hours, 18 and 30 hours, 18 and 28 hours, 18 and 26 hours, 18 and 24 hours, 18 and 22 hours, 18 and 20 hours, 20 hours and 36 hours, 20 hours and 34 hours, 20 hours and 32 hours, 20 hours and 30 hours, 20 hours and 28 hours, 20 hours and 26 hours, 20 hours and 24 hours, 20 hours and 22 hours, 22 hours and 36 hours, 22 hours and 34 hours, 22 hours and 32 hours, 22 hours and 30 hours, 22 hours and 28 hours, 22 hours and 26 hours, 22 hours and 24 hours, 24 hours and 36 hours, 24 hours and 34 hours, 24 hours and 32 hours, 24 hours and 30 hours, 24 hours and 28 hours, 24 hours and 26 hours, 26 hours and 36 hours, 26 hours and 34 hours, 26 hours and 32 hours, 26 hours and 30 hours, 26 hours and 28 hours, 28 hours and 36 hours, 28 hours and 34 hours, 28 hours and 32 hours, 28 hours and 30 hours, 30 hours and 36 hours, 30 hours and 34 hours, 30 hours and 32 hours, 32 hours and 36 hours, 32 hours and 34 hours, or 34 hours and 36 hours after contacting the cells with virus.

In some embodiments, the cells are maintained at between 2°C and room temperature following culture. In some embodiments, the cells are maintained at between 2°C and 8°C following culture. In some embodiments, the cells are maintained at between 2°C following culture. In some embodiments, the cells are maintained at between 4°C following culture. In some embodiments, the cells are maintained at between any of 2°C and 26°C, 2°C and 24°C, 2°C and 22°C, 2°C and 20°C, 2°C and 18°C, 2°C and 16°C, 2°C and 14°C, 2°C and 12°C, 2°C and 10°C, 2°C and 8°C, 2°C and 6°C, 2°C and 4°C, 4°C and 26°C, 4°C and 24°C, 4°C and 22°C, 4°C and 20°C, 4°C and 18°C, 4°C and 16°C, 4°C and 14°C, 4°C and 12°C, 4°C and 10°C, 4°C and 8°C, 4°C and 6°C, 6°C and 26°C, 6°C and 24°C, 6°C and 22°C, 6°C and 20°C, 6°C and 18°C, 6°C and 16°C, 6°C and 14°C, 6°C and 12°C, 6°C and 10°C, 6°C and 8°C, 8°C and 26°C, 8°C and 24°C, 8°C and 22°C, 8°C and 20°C, 8°C and 18°C, 8°C and 16°C, 8°C and 14°C, 8°C and 12°C, 8°C and 10°C, 10°C and 26°C, 10°C and 24°C, 10°C and 22°C, 10°C and 20°C, 10°C and 18°C, 10°C and 16°C, 10°C and 14°C, 10°C and 12°C, 12°C and 26°C, 12°C and 24°C, 12°C and 22°C, 12°C and 20°C, 12°C and 18°C, 12°C and 16°C, 12°C and 14°C, 14°C and 26°C, 14°C and 24°C, 14°C and 22°C, 14°C and 20°C, 14°C and 18°C, 14°C and 16°C, 16°C and 26°C, 16°C and 24°C, 16°C and 22°C, 16°C and 20°C, 16°C and 18°C, 18°C and 26°C, 18°C and 24°C, 18°C and 22°C, 18°C and 20°C, 20°C and 26°C, 20°C and 24°C, 20°C and 22°C, 22°C and 26°C, 22°C and 24°C, or 24°C and 26°C following culture

In some embodiments, the cells are maintained at between 2°C and room temperature following culture and prior to virus infection. In some embodiments, the cells are maintained at between 2°C and 8°C following culture and prior to virus infection. In some embodiments, the cells are maintained at 2°C following culture and prior to virus infection. In some embodiments, the cells are maintained at 4°C following culture and prior to virus infection. In some embodiments, the cells are maintained at between any of 2°C and 26°C, 2°C and 24°C, 2°C and 22°C, 2°C and 20°C, 2°C and 18°C, 2°C and 16°C, 2°C and 14°C, 2°C and 12°C, 2°C and 10°C, 2°C and 8°C, 2°C and 6°C, 2°C and 4°C, 4°C and 26°C, 4°C and 24°C, 4°C and 22°C, 4°C and 20°C, 4°C and 18°C, 4°C and 16°C, 4°C and 14°C, 4°C and 12°C, 4°C and 10°C, 4°C and 8°C, 4°C and 6°C, 6°C and 26°C, 6°C and 24°C, 6°C and 22°C, 6°C and 20°C, 6°C and 18°C, 6°C and 16°C, 6°C and 14°C, 6°C and 12°C, 6°C and 10°C, 6°C and 8°C, 8°C and 26°C, 8°C and 24°C, 8°C and 22°C, 8°C and 20°C, 8°C and 18°C, 8°C and 16°C, 8°C and 14°C, 8°C and 12°C, 8°C and 10°C, 10°C and 26°C, 10°C and 24°C, 10°C and 22°C, 10°C and 20°C, 10°C and 18°C, 10°C and 16°C, 10°C and 14°C, 10°C and 12°C, 12°C and 26°C, 12°C and 24°C, 12°C and 22°C, 12°C and 20°C, 12°C and 18°C, 12°C and 16°C, 12°C and 14°C, 14°C and 26°C, 14°C and 24°C, 14°C and 22°C, 14°C and 20°C, 14°C and 18°C, 14°C and 16°C, 16°C and 26°C, 16°C and 24°C, 16°C and 22°C, 16°C and 20°C, 16°C and 18°C, 18°C and 26°C, 18°C and 24°C, 18°C and 22°C, 18°C and 20°C, 20°C and 26°C, 20°C and 24°C, 20°C and 22°C, 22°C and 26°C, 22°C and 24°C, or 24°C and 26°C following culture and prior to virus infection.

In some embodiments, the cells are maintained at between 2°C and 8°C after virus infection. In some embodiments, the cells are maintained at 2°C after virus infection. In some embodiments, the cells are maintained at 2°C after virus infection. In some embodiments, the cells are maintained at between any of 2°C and 8°C, 2°C and 6°C, 2°C and 4°C, 4°C and 8°C, 4°C and 6°C, or 6°C and 8°C, after virus infection.

In some embodiments, the cells are maintained at the same temperature before virus infection. In some embodiments, the cells are maintained at different temperatures before virus infection and after virus infection. In some embodiments, the cells are maintained at 2°C to 8°C before virus infection and at 2°C to 8°C after virus infection. In some embodiments, the cells are maintained at 4°C before virus infection and at 2°C to 8°C after virus infection. In some embodiments, the cells are maintained at room temperature before virus infection and at 2°C to 8°C after virus infection. In some embodiments, the cells are maintained at room temperature before virus infection and at 4°C after virus infection.

The stabilized population of immune cells comprise cytokine-induced killer (CIK) cells. In some embodiments, the CIK cell are CD3+/CD56+ cells. In some embodiments, the percentage of CIK cells in the population is maintained or enhanced during the hold period at 2°C to 8°C. In some embodiments, the percentage of CIK cells in the population are enhanced by any of 5%, 10%, 15%, 20%, 25%, 30%, 35%, 40%, 45%, or 50% or any value therebetween. In some embodiments, the percentage of CIK cells in the population are enhanced by more than any of 5%, 10%, 15%, 20%, 25%, 30%, 35%, 40%, 45%, or 50% or any value therebetween.

In some embodiments, the stabilized population of immune cells comprise CD3+/CD56+ cells. In some embodiments, the percentage of CD3+CD56+ cells in the population is maintained or enhanced during the hold period at 2°C to 8°C. In some embodiments, the percentage of CD3+CD56+ cells in the population are enhanced by any of 5%, 10%, 15%, 20%, 25%, 30%, 35%, 40%, 45%, or 50% or any value therebetween. In some embodiments, the percentage of CD3+CD56+ cells in the population are enhanced by more than any of 5%, 10%, 15%, 20%, 25%, 30%, 35%, 40%, 45%, or 50% or any value therebetween.

In some embodiments, the stabilized population of immune cells comprise CD3+/CD56+ cells. In some embodiments, the percentage of CD14+ cells in the population is maintained or enhanced during the hold period at 2°C to 8°C. In some embodiments, the percentage of CD14+ cells in the population are enhanced by any of 5%, 10%, 15%, 20%, 25%, 30%, 35%, 40%, 45%, or 50% or any value therebetween. In some embodiments, the percentage of CD14+ cells in the population are enhanced by more than any of 5%, 10%, 15%, 20%, 25%, 30%, 35%, 40%, 45%, or 50% or any value therebetween.

In some embodiments, the stabilized population of immune cells comprise CD3+/CD56+ cells. In some embodiments, the percentage of CD3+/CD56+/CD14+ cells in the population is maintained or enhanced during the hold period at 2°C to 8°C. In some embodiments, the percentage of CD3+/CD56+/CD14+ cells in the population are enhanced by any of 5%, 10%, 15%, 20%, 25%, 30%, 35%, 40%, 45%, or 50% or any value therebetween. In some embodiments, the percentage of CD3+/CD56+/CD14+ cells in the population are enhanced by more than any of 5%, 10%, 15%, 20%, 25%, 30%, 35%, 40%, 45%, or 50% or any value therebetween.

In some embodiments, the viability of cells in the stable population of immune cells is greater than 70%. In some embodiments, the viability of cells of the stable population of immune cells is greater than and of 70%, 75%, 80%, 85%, 90%, 95% or 100%. In some embodiments, the viability of cells in the stable population of cells is between and of 70% and 75%, 70% and 80%, 70% and 85%, 70% and 90%, 70% and 95%, 70% and 100%, 75% and 80%, 75% and 85%, 75% and 90%, 75% and 95%, 75% and 100%, 80% and 85%, 80% and 90%, 80% and 95%, 80% and 100%, 85% and 90%, 85% and 95%, 85% and 100%, 90% and 95%, 95% and 100%. In some embodiments, the viability of cells in the stable population of cells is between and of 70% and 75%, 70% and 80%, 70% and 85%, 70% and 90%, 70% and 95%, 70% and 100%, 75% and 80%, 75% and 85%, 75% and 90%, 75% and 95%, 75% and 100%, 80% and 85%, 80% and 90%, 80% and 95%, 80% and 100%, 85% and 90%, 85% and 95%, 85% and 100%, 90% and 95%, 95% and 100% for more than any of 48, 54, 60, 66, 72, 78, 84 or 90 hours of hold at 2°C to 8°C. In some embodiments, viability is measured by trypan blue exclusion. In some embodiments, viability is measured by flow cytometry. In some embodiments, viability is measured by flow cytometry using Sytox™ blue dead cell stain. In some embodiments, viability in Flow cytometry may also be assessed by Zombie-NIR™ staining or Zombie-Violet™ Dye.

In some embodiments of the invention, the immune cells are derived from peripheral blood mononuclear cells (PBMCs). As used herein, a "peripheral blood mononuclear cells" or "PBMCs" refers to a heterogeneous population of blood cells having a round nucleus. In some embodiments, PBMCs include lymphocytes such as T cells, B cells, NK cells (including NKT cells and CIK cells) and monocytes such as macrophages and dendritic cells. PBMCs can be isolated by means known in the art. For example, PBMCs can be derived from peripheral blood of an individual based on density of PBMCs compared to other blood cells. In some embodiments, PBMCs are derived from peripheral blood of an individual using Ficoll *(e.g.,* a ficoll gradient). In some embodiments, PBMCs are derived from peripheral blood of an individual using ELUTRA® cell separation system. In some embodiments, PBMCs are isolated from an individual. In some embodiments not forming part of the claimed invention, the PBMCs are autologous to an individual to be treated with the cells of the present disclosure. In some embodiments not forming part of the claimed invention, the PBMCs are allogeneic to an individual to be treated.

In some embodiments not forming part of the claimed invention, the present disclosure provides methods of enhancing the eclipse period of an oncolytic virus, wherein the oncolytic virus in therapeutic immune cells, the method comprising a) culturing peripheral blood mononuclear cells (PBMCs) under conditions to select and enhance natural killer T (NKT) cells, natural killer (NK) cells and T cells at about 37°C, b) contacting the cells with the oncolytic virus, and c) removing the cells from the culture and maintaining the cells at about 2°C to about 8°C for up to about 120 hours. In some embodiments not forming part of the claimed invention, the present disclosure provides methods of enhancing the eclipse period of an oncolytic virus, wherein the oncolytic virus in therapeutic immune cells, the method comprising a) culturing peripheral blood mononuclear cells (PBMCs) under conditions to select and enhance natural killer T (NKT) cells, natural killer (NK) cells and T cells at about 37°C, b) removing the cells from culture and maintaining the cells at about 2°C to room temperature, c) contacting the cells with the oncolytic virus, and d) removing the cells from the culture and maintaining the cells at about 2°C to about 8°C for up to about 120 hours. In some embodiments, the cells are maintained at about 2°C, about 3°C, about 4°C, about 5°C, about 6°C, about 7°C, or about 8°C. In some embodiments, the cells are maintained for between any of about 12 hours and about 24 hours, about 24 hours and about 36 hours, about 36 hours and about 48 hours, about 48 hours and about 60 hours, about 60 hours and about 72 hours, about 72 hours and about 84 hours, about 84 hours and about 96 hours, about 96 hours and about 108 hours, or about 108 hours and about 120 hours. In some embodiments, the oncolytic virus is a vaccinia virus. In some embodiments the vaccinia virus comprises a mutation in the viral thymidine kinase (TK) gene and/or the viral growth factor (VGF) gene. In some embodiments, the vaccinia virus comprises a mutation in the viral thymidine kinase gene. In some embodiments, the vaccinia virus comprises a mutation in the viral growth factor (VGF) gene. In some embodiments, the oncolytic virus is replication competent. In some embodiments, the vaccinia virus is an EEV virus. In some embodiments, the enhanced eclipse period is an extended eclipse period. In some embodiments, the enhanced eclipse period is extended by more than about any of 12 hours, 24 hours, 36 hours, 48 hours, 60 hours, 72 hours, 84 hours, 96 hours, 108 hours, or 120 hours.

In some embodiments of the invention, the immune cells cultured under conditions to select and enhance NKT cells, NK cells and/or T cells are cultured in more than any of 1 liter, 2 liters, 3 liters, 4 liters, 5 liters, 6 liters, 7 liters, 8 liters, 9 liters or 10 liters of culture media. In some embodiments, the immune cells cultured under conditions to select and enhance NKT cells, NK cells and/or T cells are cultured in between 1 liter and 10 liters, 2 liters and 10 liters, 3 liters and 10 liters, 4 liters and 10 liters, 5 liters and 10 liters, 6 liters and 10 liters, 7 liters and 10 liters, 8 liters and 10 liters, 9 liter and 10 liters, 1 liter and 9 liters, 2 liters and 9 liters, 3 liters and 9 liters, 4 liters and 9 liters, 5 liters and 9 liters, 6 liters and 9 liters, 7 liters and 9 liters, 8 liters and 9 liters, 1 liter and 8 liters, 2 liters and 8 liters, 3 liters and 8 liters, 4 liters and 8 liters, 5 liters and 8 liters, 6 liters and 8 liters, 7 liters and 8 liters, 1 liter and 7 liters, 2 liters and 7 liters, 3 liters and 7 liters, 4 liters and 7 liters, 5 liters and 7 liters, 6 liters and 7 liters, 1 liter and 6 liters, 2 liters and 6 liters, 3 liters and 6 liters, 4 liters and 6 liters, 5 liters and 6 liters, 1 liter and 5 liters, 2 liters and 5 liters, 3 liters and 5 liters, 4 liters and 5 liters, 1 liter and 4 liters, 2 liters and 4 liters, 3 liters and 4 liters, 1 liter and 3 liters, 2 liters and 3 liters, or 1 liter and 2 liters. In some embodiments, the immune cells are cultured in a bag. In some embodiments, the immune cells are cultured in a cell factory. In some embodiments, the immune cells are cultured in a bioreactor.

In some embodiments of the invention, the immune cells cultured under conditions to select and enhance NKT cells, NK cells and/or T cells are cultured for any of 10 days to 50 days prior to holding the cells at 2°C to 8°C. In some embodiments of the invention, the immune cells cultured under conditions to select and enhance NKT cells, NK cells and/or T cells are cultured for any of 10 days, 15 days, 20 days, 25 days, 28 days, 30 days, 32 days, 34 days, 36 days, 40 days, 45 days or 50 days prior to holding the cells at 2°C to 8°C. In some embodiments, the cells are cultured for between 10 days and 15 days, 10 days and 20 days, 10 days and 25 days, 10 days and 30 days, 10 days and 35 days, 10 days and 40 days, 10 days and 45 days, 10 days and 50 days, 15 days and 20 days, 15 days and 25 days, 15 days and 30 days, 15 days and 35 days, 15 days and 40 days, 15 days and 45 days, 15 days and 50 days, 20 days and 25 days, 20 days and 30 days, 20 days and 35 days, 20 days and 40 days, 20 days and 45 days, 20 days and 50 days, 25 days and 30 days, 25 days and 35 days, 25 days and 40 days, 25 days and 45 days, 25 days and 50 days, 30 days and 35 days, 30 days and 40 days, 30 days and 45 days, 30 days and 50 days, 35 days and 40 days, 35 days and 45 days, 35 days and 50 days, 40 days and 45 days, 40 days and 50 days, or 45 days and 50 days.

In some embodiments of the invention, room temperature is 22°C. In some embodiments, room temperature is 22°C ± 0.5°C, 22°C ± 0.6°C, 22°C ± 0.7°C, 22°C ± 0.8°C, 22°C ± 0.9°C, 22°C ± 1.0°C, or 22°C ± 2.0°C.

In some embodiments of the invention, the cells are maintained at 2°C to 8°C in a bag. In some embodiments, the bag is a plastic bag. In some embodiments, the bag is not gas-permeable. In some embodiments, the bag is not $CO_2$-permeable. In some embodiments, the cells are maintained at 2°C to 8°C in less than any of 5% $CO_2$, 4% $CO_2$, 3% $CO_2$, 2% $CO_2$, 1% $CO_2$, or 0.1% $CO_2$.

In some embodiments of the invention, the cells are maintained at 2°C to 8°C in a salt solution. In some embodiments, the cells are maintained in a Plasma-Lyte©. In some embodiments, the salt solution is essentially free of serum and/or growth factors. In some embodiments, the salt solution comprises less than any of 1% serum, 0.9% serum, 0.8% serum, 0.7% serum, 0.6% serum, 0.5% serum, 0.4% serum, 0.3% serum, 0.2% serum, 0.1% serum, or 0.01% serum. Plasma-Lyte© is a crystalloid solution for intravenous infusion, with varying electrolyte formulation. Generally the solution has a composition that mimics human physiological plasma electrolyte concentrations, osmolality and pH. In some embodiments, the cells are maintained at 2°C to 8°C in a salt solution comprising 140 mM sodium, 5 mM potassium, 98 mM chloride, 1.5 mM magnesium, 27 mM acetate, and 23 mM gluconate. In some embodiments, the Plasma-Lyte© is Plasma-Lyte© A or Plasma-Lyte© 148.

In some embodiments of the invention, the cells are maintained at 2°C to 8°C in the dark.

The cells of the stabilized population of immune cells comprise an oncolytic virus. In some embodiments, the oncolytic virus is a vaccinia virus. In some embodiments, the vaccinia virus comprises a mutation in the viral thymidine kinase (TK) gene and/or the viral growth factor (VGF) gene. In some embodiments, the vaccinia virus comprises a mutation in the viral thymidine kinase gene. In some embodiments, the vaccinia virus comprises a mutation in the viral growth factor (VGF) gene. In some embodiments, the oncolytic virus is replication competent. In some embodiments, the vaccinia virus is an EEV virus.

In some embodiments of the invention, the immune cells are infected with the oncolytic virus at a multiplicity of infection (MOI) of 1.0 to a MOI of 10. In some embodiments of the invention, the immune cells are infected with the oncolytic virus at a multiplicity of infection (MOI) of any of 0.001, 0.005, 0.01, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3. 1.4, 1.5, 1.6, 1.7, 1.8, 1.9, 2.0, 3.0, 4.0. 5.0. 6.0 7.0, 8.0, 9.0 or 10.0, or any value therebetween.

In some embodiments, the immune cells are infected with an oncolytic virus at 37°C. In some embodiments, the immune cells are infected with an oncolytic virus at any of 22°C, 25°C, 30°C, 35°C, 37°C, 39°C, or 42°C or any temperature therebetween. In some embodiments, the immune cells are infected with an oncolytic virus for 15 min to 6 hours. In some embodiments, the immune cells are infected with an oncolytic virus for any of 15 min, 30 min, 45 min, 1 hour, 2 hours, 3 hours, 4 hours, 5 hours, 6 hours or more than 6 hours. In some embodiments, the immune cells are infected with an oncolytic virus for 2 hours. In some embodiments, the immune cells are infected with an oncolytic virus at 37°C for 2 hours. In some embodiments, the oncolytic virus is a vaccinia virus. In some embodiments, the immune cells are infected with a vaccinia virus at 37°C for 2 hours.

## Methods of Treatment

The following references to methods of treatment are to be interpreted as references to the compounds, pharmaceutical compositions and medicaments of the present disclosure for use in a method for treatment of the human (or animal) body by therapy (or for diagnosis), and do not form part of the claimed invention.

The population of infected effector cells are injected into the recipient. Determination of suitability of administering cells of the present disclosure will depend, inter alia, on assessable clinical parameters such as serological indications and histological examination of tissue biopsies. Generally, a pharmaceutical composition is administered.

In some embodiments, the present disclosure provides methods of treating cancer in an individual, the method comprising administering to the individual an effective amount of a stabilized population of immune cells comprising an oncolytic virus prepared as described herein.

In some embodiments, the present disclosure provides methods of treating cancer in an individual, the method comprising administering to the individual an effective amount of a stabilized population of immune cells comprising an oncolytic virus, wherein the stabilized population of immune cells is prepared by a method comprising a) culturing immune cells under conditions to select and enhance natural killer T (NKT) cells, natural killer (NK) cells and T cells at about 37°C, b) contacting the cells with the oncolytic virus, and c) removing the cells from the culture and maintaining the cells at about 2°C to room temperature for up to about 72 hours.

In some embodiments, the present disclosure provides methods of treating cancer in an individual, the method comprising administering to the individual an effective amount of a stabilized population of immune cells comprising an oncolytic virus, wherein the stabilized population of immune cells is prepared by a method comprising a) culturing immune cells under conditions to select and enhance natural killer T (NKT) cells, natural killer (NK) cells and T cells at about 37°C, b) removing the cells from the culture and maintaining the cells at about 2°C to room temperature for up to about 12 to about 36 hours, c) contacting the cells with the oncolytic virus, and c) maintaining the cells at about 2°C to room temperature for up to about 12 to about 36 hours.

In some embodiments, the present disclosure provides methods of treating cancer in an individual, the method comprising a) isolating immune cells from the individual, b) culturing the immune cells from the individual under conditions to select and enhance NKT cells, NK cells and T cells at about 37°C, c) contacting the cells with the oncolytic virus, and d) removing the cells from the culture and maintaining the cells at about 2°C to room temperature for up to about 72 hours, and e) administering to the individual an effective amount of a stabilized population of immune cells comprising an oncolytic virus.

In some embodiments, the present disclosure provides methods of treating cancer in an individual, the method comprising a) isolating immune cells from the individual, b) culturing the immune cells from the individual under conditions to select and enhance NKT cells, NK cells and T cells at about 37°C, c) removing the cells from the culture and maintaining the cells at about 2°C to room temperature for up to about 12 to about 36 hours, d) contacting the cells with the oncolytic virus, and e) maintaining the cells at about 2°C to room temperature for up to about 12 to about 36 hours, and e) administering to the individual an effective amount of a stabilized population of immune cells comprising an oncolytic virus.

In some embodiments of the methods of treatment of the present disclosure, wherein the oncolytic virus remains in the immune cell until the immune cell interacts with a tumor in the individual. In some embodiments, the stabilized population of immune cells is administered to the individual during the eclipse period of the oncolytic virus.

In some embodiments of the methods of treatment of the present disclosure, the immune cells are autologous cells. In other embodiments, the immune cells are allogeneic cells.

Routes of administration include systemic injection, *e.g.* intravascular, subcutaneous, or intraperitoneal injection, intratumor injection, *etc.* Where the recipient animal is a human, the number of cells injected will usually be at least

about $0.5\times10^8$ and not more than about $5\times10^{10}$, more usually at least about $1\times10^8$ or at least about $1\times10^9$. In some embodiments, not more than about any of $0.5\times10^8$, $0.75\times10^8$, $1.0\times10^8$, $2.0\times10^8$, $3.0\times10^8$, $4.0\times10^8$, $5.0\times10^8$, $6.0\times10^8$, $7.0\times10^8$, $8.0\times10^8$, $9.0\times10^8$, $1.0\times10^9$, $10\times10^9$, $3.0\times10^9$, $4.0\times10^9$, $5.0\times10^9$, $6.0\times10^9$, $10\times10^9$, $8.0\times10^9$, $9.0\times10^9$, $1.0\times10^{10}$, $10\times10^{10}$, $3.0\times10^{10}$, $4.0\times10^{10}$, or $5\times10^{10}$ is administered to the patient. In some embodiments, between about any of $0.5\times10^8$ and $1.0\times10^8$, $1.0\times10^8$ and $5.0\times10^8$, $5.0\times10^8$ and $1.0\times10^9$, $1.0\times10^9$ and $5.0\times10^9$, $5.0\times10$ and $1.0\times10^{10}$, or $1.0\times10^{10}$ and $5.0\times10^{10}$ is administered to the patient.

The present disclosure provides methods of suppressing tumor cell growth, comprising contacting a tumor cell with an infected effector cell of the present disclosure such that the oncolytic virus enters the tumor cell, and there is selective cytotoxicity for the tumor cell. The composition may be administered once, or a series of times, *e.g.* daily, weekly, semimonthly, *etc.* The efficacy may be monitored by standard methods as appropriate to the specific cancer, *e.g.* tumor size, biopsy, presence of tumor cells in the blood, *etc.* Tumor cell growth can be assessed by determining whether tumor cells are proliferating using a $^3$H-thymidine incorporation assay, or counting tumor cells. "Suppressing" tumor cell growth means any or all of the following states: slowing, delaying, and stopping tumor growth, as well as tumor shrinkage. "Suppressing" tumor growth indicates a growth state that is curtailed when compared to growth without contact with the cells of the present disclosure.

Cancer, as used herein, refers to hyperproliferative conditions. The term usually denotes malignant cell populations. Such disorders have an excess cell proliferation of one or more subsets of cells, which often appear to differ from the surrounding tissue both morphologically and genotypically. The excess cell proliferation can be determined by reference to the general population and/or by reference to a particular patient, *e.g.* at an earlier point in the patient's life. Hyperproliferative cell disorders can occur in different types of animals and in humans, and produce different physical manifestations depending upon the affected cells.

In some embodiments of the present disclosure, the stabilized population of immune cells is used to treat cancer. In some embodiments, the cancer is any one, but not limited to, leukemias, lymphomas (Hodgkins and non-Hodgkins), sarcomas, melanomas, adenomas, carcinomas of solid tissue including breast cancer and pancreatic cancer, hypoxic tumors, squamous cell carcinomas of the mouth, throat, larynx, and lung, genitourinary cancers such as cervical, ovarian, and bladder cancer, hematopoietic cancers, head and neck cancers, and nervous system cancers, such as gliomas, astrocytomas, meningiomas, *etc.,* benign lesions such as papillomas, and the like. In some embodiments, the cancer is a colorectal cancer, an epithelial ovarian cancer, a gastric cancer, a hepatocellular cancer, an osteosarcoma, or triple negative breast cancer.

In some embodiments, the cancers for treatment include, but are not limited to, ovarian and breast cancer and lymphomas. Ovarian cancer is the second most commonly diagnosed gynecologic malignancy, the deadliest gynecologic malignancy, and the fourth leading cause of cancer-related deaths in women in the USA. About 1 in 70 women eventually develops ovarian cancer, and 1 in 100 women dies of it. Ovarian cancer affects predominantly perimenopausal and postmenopausal women.

Ovarian tumors are the most histologically diverse group of tumors. At least 80% of malignant ovarian tumors arise from the coelomic epithelium. The most common type is serous cystadenocarcinoma, which accounts for 75% of cases of epithelial ovarian cancer. Others include mucinous, endometroid, transitional cell, Brenner, clear cell, and unclassified carcinomas. The remaining 20% of malignant ovarian tumors are germ cell and sex cord-stromal cell tumors, which are nonepithelial in origin, and metastatic carcinomas to the ovary (most commonly, breast and GI carcinomas). Germ cell tumors, which arise from the primary germ cells of the ovary, occur in young women and are uncommon in women >30 yr. Malignant germ cell tumors include dysgerminomas, immature teratomas, endodermal sinus tumors, embryonal carcinomas, choriocarcinoma, and polyembryomas. Stromal malignancies include granulosatheca cell tumors and Sertoli-Leydig cell tumors.

Ovarian cancer spreads by direct extension, by intraperitoneal implantation via exfoliation of cells into the peritoneal cavity, by lymphatic dissemination in the pelvis and paraaortic region, and, less commonly, hematogenously to the liver or lungs.

CA 125 is a cell surface glycoprotein detectable in 80% of cases of epithelial ovarian cancer. However, it is not specific to patients with ovarian cancer and, among premenopausal patients, can be mildly elevated in several benign disorders, including endometriosis, pelvic inflammatory disease, pregnancy, and leiomyomata uteri. For patients with advanced-stage epithelial ovarian cancer, cytoreductive (tumor-debulking) surgery is advised to improve the efficacy of adjunctive therapies. The goal is to reduce the tumor burden so that the maximum diameter of the remaining implants is <1 cm. Cytoreductive surgery usually includes total hysterectomy, bilateral salpingo-oophorectomy, omentectomy, and excision of the tumor from any other sites. Rectosigmoid resection (usually with primary reanastomosis), radical peritoneal stripping, resection of diaphragmatic peritoneum, or splenectomy may be required. Prognosis for patients with advanced disease is directly related to the success of cytoreductive surgery.

The methods of the combination may be combined with conventional chemotherapeutic, radiologic and/or surgical methods of treatment. Cytotoxic agents that act to reduce cellular proliferation are known in the art and widely used. Such agents include alkylating agents, such as nitrogen mustards, *e.g.* mechlorethamine, cyclophosphamide, melphalan (L-sarcolysin), *etc.;* and nitrosoureas, *e.g.* carmustine (BCNU), lomustine (CCNU), semustine (methyl-CCNU), streptozocin, chlorozotocin, *etc.* Antimetabolite agents include pyrimidines, *e.g.* cytarabine (CYTOSAR-U), cytosine arabinoside, fluorouracil (5-FU), floxuridine (FUdR), *etc.;* purines, *e.g.* thioguanine (6-thioguanine), mercaptopurine (6-MP), pentostatin, fluorouracil (5-FU) *etc.;* and folic acid analogs, *e.g.* methotrexate, 10-propargyl-5,8-dideazafolate (PDDF, CB3717), 5,8-dideazatetrahydrofolic acid (DDATHF), leucovorin, *etc.* Other natural products include azathioprine; brequinar; alkaloids and synthetic or semi-synthetic derivatives thereof, *e.g.* vincristine, vinblastine, vinorelbine, *etc.;* podophyllotoxins, *e.g.* etoposide, teniposide, *etc.;* antibiotics, *e.g.* anthracycline, daunorubicin hydrochloride (daunomycin, rubidomycin, cerubidine), idarubicin, doxorubicin, epirubicin and morpholino derivatives, *etc.;* phenoxizone biscyclopeptides, *e.g.* dactinomycin; basic glycopeptides, *e.g.* bleomycin; anthraquinone glycosides, *e.g.* plicamycin (mithromycin); anthracenediones, *e.g.* mitoxantrone; azirinopyrrolo indolediones, *e.g.* mitomycin; and the like. Other chemotherapeutic agents include metal complexes, *e.g.* cisplatin (cis-DDP), carboplatin, *etc.;* ureas, *e.g.* hydroxyurea; and hydrazines, *e.g.* N-methylhydrazine. Other anti-proliferative agents of interest include immunosuppressants, *e.g.* mycophenolic acid, thalidomide, desoxyspergualin, azasporine, leflunomide, mizoribine, azaspirane (SKF 105685), *etc.* The antineoplastic agents taxols (or taxanes) hyperstabilize polymerized microtubules, leading to mitotic arrest and cytotoxicity in proliferating cells. Taxanes (or taxols), such as paclitaxel, docetaxel, *etc.* are of interest. Also of interest are the microtubule stabilizing epothilones (*see* Bollag et al. (1995) Cancer Research, Vol 55, Issue 11 2325-2333), *e.g.* epothilone A and epothilone B. Retinoids, *e.g.* vitamin A, 13-cis-retinoic acid, trans-retinoic acid, isotretinoin, *etc.;* carotenoids, *e.g.* betacarotene, vitamin D, *etc.* Retinoids regulate epithelial cell differentiation and proliferation and are used in both treatment and prophylaxis of epithelial hyperproliferative disorders. The present disclosure relates to the selective killing of neoplastic cells by combined viral-mediated oncolysis and immune therapy, which combination provides a synergistic benefit when compared to either of the single therapies. The present disclosure provides for oncolytic virus-infected immune cells, a method of killing neoplastic cells using oncolytic virus-infected immune cells, and a pharmaceutical composition containing oncolytic virus-infected immune cells. In the combined therapeutic, the effector cells were shown to retain their ability to traffic to tumors. At the tumor site the oncolytic virus was released deep in the tumor rather than merely at the surface; thus, the cell-mediated delivery of the virus led to enhanced biodistribution within the tumor. In addition, the cytotoxic effects of the effector cells may be increased by viral replication in the tumor target. This combined therapeutic has been demonstrated to be safe, with minimal viral infection of normal tissues, and highly effective.

It has been shown that the effector cells of the present disclosure can be used to deliver oncolytic virus to a recipient capable of mounting an anamnestic response against the oncolytic virus, *e.g.* a vaccinia virus can be successfully delivered to a patient previously immunized with vaccinia. This property overcomes a limitation of previously described viral therapy, where a single agent, *e.g.* an oncolytic virus, is ineffective in repeat doses or in vaccinated individuals due to the recipient immune response. It has been found that a polyclonal, anti-vaccinia antibody failed to recognize CIK cells infected with vaccinia, although the antibody did recognize tumor cells infected with vaccinia. VIG (vaccinia immunoglobulin) is an FDA approved treatment for adverse events following smallpox vaccination. Doses of VIG that completely neutralized vaccinia (prevented vaccinia from infecting a cell layer following 2 h of exposure to VIG) had no effect on the ability of infected CIK cells to transfer virus to a cell layer. Animals treated with vaccinia (or infected CIK cells) produced CTLs (cytotoxic T lymphocytes) that could recognize infected tumor cells, but not infected CIK cells. Tumor bearing and immunized mice were treated (IV) with vaccinia or infected CIK cells. There was very little evidence of vaccinia in the tumor when it was delivered alone. There were multiple areas of positively stained cells (for vaccinia infection) within the tumors when infected CIK cells were used. Tumor bearing animals treated with high doses ($10\times$ therapeutic levels) of VIG, and then treated with infected CIK cells still produced signal (bioluminescence imaging from virally encoded luciferase) from within the tumor.

In some embodiments, the present disclosure provides for uses of a composition of a stable population of immune cells in the manufacture of a medicament to treat a disorder (*e.g.*, cancer) in an individual, wherein the stabilized population of immune cells is prepared by a method comprising a) culturing immune cells under conditions to select and enhance natural killer T (NKT) cells, natural killer (NK) cells and T cells at about 37°C, b) contacting the cells with the oncolytic virus, and c) removing the cells from the culture and maintaining the cells at about 2°C to room temperature for up to about 72 hours.

In some embodiments, the present disclosure provides for uses of a composition of a stable population of immune cells in the manufacture of a medicament to treat a disorder (*e.g.*, cancer) in an individual, wherein the stabilized population of immune cells is prepared by a method comprising a) culturing immune cells under conditions to select and enhance natural killer T (NKT) cells, natural killer (NK) cells and T cells at about 37°C, b) removing the cells from the culture and maintaining the cells at about 2°C to room temperature for up to about 12 to about 36 hours, c) contacting the cells with the oncolytic virus, and c) maintaining the cells at about 2°C to room temperature for up to about 12 to about 36 hours.

In some embodiments, the present disclosure provides compositions of a stable population of immune cells for use in treating a disorder (*e.g.*, cancer) in an individual, wherein the stabilized population of immune cells is prepared by a method comprising a) culturing immune cells under conditions to select and enhance natural killer T (NKT) cells, natural killer (NK) cells and T cells at about 37°C, b) contacting the cells with the oncolytic virus, and c) removing the cells from the culture and maintaining the cells at about 2°C to room temperature for up to about 72 hours.

In some embodiments, the present disclosure provides compositions of a stable population of immune cells for use in treating a disorder (*e.g.*, cancer) in an individual, wherein the stabilized population of immune cells is prepared by a method comprising a) culturing immune cells under conditions to select and enhance natural killer T (NKT) cells, natural killer (NK) cells and T cells at about 37°C, b) removing the cells from the culture and maintaining the cells at about 2°C to room temperature for up to about 12 to about 36 hours, c) contacting the cells with the oncolytic virus, and c) maintaining the cells at about 2°C to room temperature for up to about 12 to about 36 hours.

**Pharmaceutical Compositions**

The following references to pharmaceutical compositions are provided for reference, and do not form part of the claimed invention.

The present disclosure also includes compositions, including pharmaceutical compositions, containing the immune cells described herein. Such compositions are useful for administration, for example, when measuring the effectiveness of cell killing in an individual. In some embodiments, these compositions further comprise a pharmaceutically acceptable excipient. These compositions, which can comprise an effective amount of an the immune cells of this disclosure in a pharmaceutically acceptable excipient, are suitable for systemic administration to individuals in unit dosage forms, sterile parenteral solutions or suspensions, sterile non-parenteral solutions or oral solutions or suspensions, oil in water or water in oil emulsions and the like. Formulations for parenteral and nonparenteral drug delivery are known in the art and are set forth in Remington's Pharmaceutical Sciences, 18.sup.th Edition, Mack Publishing (1990). Compositions also include lyophilized and/or reconstituted forms of the vectors of the present disclosure.

In some embodiments, the present disclosure provides pharmaceutical compositions of immune cells prepared by the methods described herein. In some embodiments, the present disclosure provides pharmaceutical compositions of immune cells infected with an oncolytic virus prepared by the methods described herein. In some embodiments, the present disclosure provides pharmaceutical compositions of immune cells infected with a vaccinia virus prepared by the methods described herein. In some embodiments, the immune cells are natural killer T (NKT) cells, natural killer (NK) cells and/or T cells. In some embodiments, the immune cells comprise cytokine-induced killer (CIK) cells. In some embodiments, the present disclosure provides pharmaceutical compositions of NKT cells, NK cells and/or T cells infected with a vaccinia virus prepared by the methods described herein. In some embodiments, the present disclosure provides pharmaceutical compositions of CIK cells infected with a vaccinia virus prepared by the methods described herein. In some embodiments, the vaccinia virus comprises a mutation in the viral thymidine kinase (TK) gene and/or the viral growth factor (VGF) gene. In some embodiments, the pharmaceutical composition is in a bag.

**Kits**

The following references to kits are provided for reference, and do not form part of the claimed invention.

Also provided are kits or articles of manufacture for use in producing a stabilized population of immune cells; *e.g.*, a stabilized population of immune cells comprising an oncolytic virus. In some embodiments, the kits comprise stabilized immune cells and/or an oncolytic virus. In some embodiments, the kits comprise components described herein (*e.g., cells, bags, reagents for analyzing a population of immune cells) in suitable packaging. Suitable packaging materials are known in the art, and include, for example, vials (such as sealed vials), vessels, ampules, bottles, jars, flexible packaging (*e.g., sealed Mylar or plastic bags), and the like. These articles of manufacture may further be sterilized and/or sealed.

In some embodiments, the present disclosure provides kits for treating cancer in an individual, wherein the kit comprises pharmaceutical compositions of stabilized immune cells prepared by the methods described herein. In some embodiments, the kits comprise an oncolytic virus. In some embodiments, the kits comprise pharmaceutical compositions of stabilized immune cells infected with an oncolytic virus prepared by the methods described herein.

In some embodiments, the immune cells are natural killer T (NKT) cells, natural killer (NK) cells and/or T cells. In some embodiments, the immune cells comprise cytokine-induced killer (CIK) cells. In some embodiments, the kit comprises pharmaceutical compositions of NKT cells, NK cells and/or T cells infected with a vaccinia virus prepared by the methods described herein. In some embodiments, the kits comprise pharmaceutical compositions of CIK cells infected with a vaccinia virus prepared by the methods described herein. In some embodiments, the vaccinia virus comprises a mutation in the viral thymidine kinase (TK) gene and/or the viral growth factor (VGF) gene.

The present disclosure also provides kits comprising components of the methods described herein and may further comprise instructions for performing said methods to manufacture stabilize immune cells and/or instructions for producing and introducing such cells to an individual. The kits described herein may further include other materials, including buffers, diluents, filters, needles, syringes, vessels, bags and package inserts with instructions for performing any of the methods described herein; *e.g.*, instructions for producing stabilized populations of immune cells and for introducing such cells to an individual.

EXAMPLES

**Example 1**

The effects of hold-times and viral infection on immune cell stability and activation was investigated at both a one-liter culture scale and a manufacturing 5 liter scale.

**Materials and Methods**

*Summary of CIK cell expansion*

*Description of CIK Cells Expansion Process*

Following screening of the donor, sufficient quantities of cells (>$10^{10}$ cells) will be collected from the patient by unstimulated apheresis and cryopreserved in aliquots. At the appropriate time, the cells will be thawed and the CIK culture will be inoculated with the appropriate level of cells in growth medium) in gas permeable bags or flasks. Media will be replaced as required.

On day 18 of the culture, samples will be obtained for QC testing. According to cell count and viability on day 14-21, the cells will be collected. Samples will be removed for final release tests including phenotype and cytotoxicity assessments.

*Preparation of the Final Suspension of CRX100*

Once the CIK cells have completed expansion and fulfilled release requirements, the CIK cells will be harvested and washed and placed in 'blood bags' for subsequent delivery into patients. Samples are removed and sent to the Clinical Laboratories for sterility testing, phenotypic analysis, and cytotoxicity assays.

Bags containing the harvested CIK cells will then be taken into the Biosafety/Isolation room where the material is thawed and mixed with virus, as required for the designated dose.

The cells and virus will be gently rocked and incubated in the closed blood bag. All work with the virus will be done within the Biosafety hood in the isolation room. Bags will be incubated for a period of at least 2 hours (or until results of sterility tests are returned, typically 2 hours) within the isolation room to allow infection of CIK cells to occur. This solution will be used within 24 hours of product release.

*Container Closure System*

Vaccinia virus is stored in 2 mL, single-use glass vials that will be stored at -80°C. The expansion process for CIK cells is carried out in gas-permeable cell culture bags. These sourced bags are made from a single-web polyolefin gas permeable film. It has an integrated tubing to allow filling, feeding, and sampling while staying in closed system. The bags are used to harvest, expand cell cultures of CIK cells. For Transfer and infusion of the CRX100 suspension Charter 500 ml transfer pack that are non-permeable are used.

*Isolation and Culture of CIK Cells at 1 liter scale.*

Human peripheral blood mononuclear cells (PBMCs) were obtained from a fresh LeukoPak (AllCells, Alameda, CA) and cultured in suspension in 40 mL of 5% Human Ab-serum AIM-V media (ThermoFisher, Waltham, MA) in 1L gas permeable bags (Charter Medical, Winston-Salem, NC). On day 0, cell cultures were treated with 400 $\mu$L of 5$\mu$g/mL IFN-$\gamma$ (R&D Systems, Minneapolis, MN). On day 1, 1mg/mL anti-CD3 mAb OKT3 (Takara Bio, Mountain View, CA) and 100 $\mu$g/mL human recombinant IL-2 (R&D Systems, Minneapolis, MN) was added to each culture. Cultures of cytokine-induced killer (CIK) cells were incubated in a humidified 5% $CO_2$ environment at 37°C. Samples of the culture were taken every 3-4 days. Cell counts were obtained using a Trypan blue exclusion assay. Cell surface markers were assayed via flow cytometry. If cell density exceeded $3 \times 10^6$ cells/mL, cultures were diluted with media to return the culture to $2 \times 10^6$ cells/mL. Cultures were re-treated with 300 IU/mL IL-2 during each culture access. If the total volume of the expansion bag exceeded 500 mL, cultures were split into two 1L expansion bags before additional dilution.

*Harvest and Washing of CIK Cells*

On day 21 to day 32 of culture expansion, immune cells were harvested and washed using a COBE 2991 Cell Processor (Terumo, BCT, Lakewood, CO) and Plasma-Lyte© (Baxter Int., Deerfield, IL). Harvested cells were collected in a 400 mL fluid transfer bag (FTB) and diluted with additional Plasma-Lyte$^©$ until a density of $8.57 \times 10^6$ cells/mL was reached. The total cell count, percent yield, and initial volume of the harvested cells was determined. The harvested immune cell Plasma-Lyte$^©$ suspension was split evenly between two FTBs using a tube sealer. Bag spikes with a ChemoLock system (ICU Medical, San Clemente, CA) were inserted into each FTB. Gas exchange specifications of the culture expansion bags and FTBs may found in Table 1.

**Table 1. Gas exchange specifications**

| Bag | $O_2$ | $CO_2$ | Water Vapor |
|---|---|---|---|
| FTB-400S PVC | 750 | 5000 | 8 |
| Charter EXP-PAK EVA | 2200 | 9000 | 3.9 |
| Metrix EVA | 1100 | 6000 | 4.7 |

*Hold Process of CIK Cells*

Cell suspensions were held at either ambient room temperature (22°C $\pm$ 5 °C) or 37°C. Both FTBs were maintained in the dark without agitation while lying flat within a secondary container. Samples will be obtained from each FTB at the time points shown below in Table 2 and the following assays will be performed: % viability, flow cytometry, and appearance.

**Table 2. Sampling Schedule**

| Elapsed Time [Hours] | Assays Performed |
|---|---|
| 0 | V+F+A |
| 2 | V+A |
| 4 | V+F+A |
| 6 | V+A |
| 8 | V+F+A |
| 12 | V+A |
| 20 | V+A |
| 24 | V+F+A |
| 36 | V+A |
| 48 | V+F+A |
| 72 | V+F+A |
| V= % Viability of Cells<br>F = Flow Cytometry<br>A = Appearance | |

*Sampling of Culture for cell counting and flow cytometry.*

Immediately following preparation of both FTBs, 0.5mL samples were taken from each bag and an initial cell count was performed. During each culture access, a 3 mL syringe was connected to a ChemoLock port with a male ChemoLock ending. Prior to access, the ChemoLock surfaces on the bag spike and 3mL syringe were cleaned with an IPA wipe. Cultures were removed from their secondary containers and brought to a homogenous suspension through a series of 360° nutating movements immediately preceding sampling. The ChemoLock ports on the bag and syringe were connected while the bag was held vertically. The bag was inverted such that syringe was now at the bottom and the bag spike inside the bag is submerged in product. 0.5mL of product sample was then collected and transferred into a 1.5mL screw cap tube.

*Analytical Tests*

*Percent Viability.* Viability of cells within each FTB was assessed via Trypan Blue exclusion assay. 25 $\mu$L of

sample was mixed with 25 µL of 0.4% Trypan Blue (VWR, Radnor, PA) stain (forming a 1:2 dilution) and incubated for 1 minute before being transferred into a standard Neubauer counting chamber. Live and dead cells were manually counted at 40x magnification using phase contrast. Percent viability was determined as followed:

$$\% \, Viability = \frac{\# \, Live \, Cells}{\# \, Live \, Cells + \# \, Dead \, Cells} \times 100$$

*Flow Cytometry.* Flow Cytometry was used to identify cell surface markers and characterize cell subpopulations. The following cell surface markers were assessed: CD3, CD56, and CD314 (BioLegend, San Diego, CA). Sytox Blue (BioLegend, San Diego, CA) was also used as a viability stain. Samples were diluted $2 \times 10^6$ cells/mL. 150 µL of each sample was added per well in a 96 well plate to be processed. Each well was then incubated with 1 µL of each antibody stock (CD3, CD56, and CD314) in 50µL Ab-binding buffer (BioLegend, San Diego, CA) at 4°C, washed 2x, and finally resuspended in 100 µL PBS (Corning, Corning, NY) with 2 µL of $100 \times$ Sytox Blue working stock solution.

*Appearance.* A description of the appearance of each FTB was made during each culture access. Turbidity, color, and cell clumping were qualitatively assessed. If the cell suspensions remained clear, the bag was described as "OK." If the color or turbidity of the sample changed significantly during the course of the stability study, a gram stain would be performed on the sample to determine if bacterial contamination had occurred.

**Study Criteria**

Samples were processed immediately after taken from each transfer bag. Flow cytometry samples were processed (antibody staining and wash) immediately, and stored for up to 2 hours, if necessary, at 4°C prior to analysis in a flow cytometer. This hold study was considered successful if all data obtained at each tested timepoint met the relevant stability specification described in Table 3.

**Table 3. Acceptance Criteria**

| Parameter | Acceptance Criteria |
|---|---|
| **Cell Viability** | ≥ 70% |
| **Flow Cytometry** | ≥ 4% CD3+/CD56+ |
| **Appearance** | Colorless to Slightly Yellow |
| **Gram Stain** | No Detectable Organisms |

**Methods and Methods for 5L scale and above.**

CRX100, consisting of cytokine-induced killer (CIK) cells cultured from either freshly collected or thawed peripheral blood cells and combined with Vaccinia Virus (vvDD), is an experimental autologous cell therapy that will be manufactured in GMP conditions. In order to demonstrate the feasibility of the proposed manufacturing process and to challenge and confirm the effectiveness of the procedures written during technology transfer, at least two engineering process runs will be performed, resulting in two unique lots of CRX100 product. The protocol is divided into two sections (1) to address the manufacture of the CIK cells and (2) to address the manufacture of the final drug product CRX100. Part 1 can be completed without completion of Part 2. This protocol describes the production and testing activities as well as the acceptance criteria that will be used to demonstrate the successful manufacture of the final CRX100 product. The data collected from these production lots and characterization assays may support regulatory submission.

*Study design*

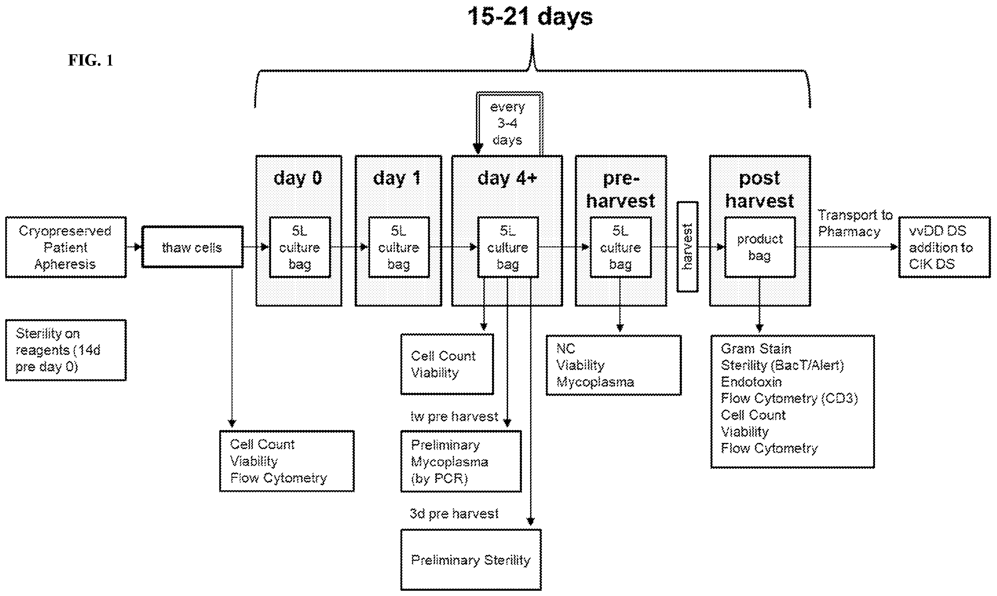

FIG. 1 is a high-level process flow diagram which describes the engineering process sequence of events and the testing performed in this study.

The CRX100 cell product manufacturing process includes two main stages 1) the bulk cell product (CIK) production and (2) the final drug product (CRX100) production.

The production of the bulk cell substance (CIK) stage of the manufacturing process includes the inoculation of $2 \times$ 5L EXP culture bags with fresh peripheral blood cells, the maintenance of the culture over a 21-day period, and the harvest, formulation and fill of CIK cells into the final infusion bag.

The production of the CRX100 final product includes the thaw of the vvDD bulk drug substance and combination of the virus with the CIK cells in the final infusion bag as well as the completion of the rapid release testing and generation of the interim COA, will be addressed in this protocol.

CIK cells represent a small fraction of peripheral blood T cells and their ex vivo expansion is necessary to obtain sufficient cells for clinical applications. CIK Cells are incubated in gas-permeable culture bags for at least 21 days before being harvested for infusion. CIK cells lose activity when cryopreserved so current trials are based on fresh infusion of the cells. Therefore, completion of release testing within a few hours of harvesting the cells is critical. Cultures may be started from freshly collected or cryopreserved peripheral blood cells as specified by protocol. Typically, CIK cell cultures show a decline in all peripheral blood phenotypes except CD3+ cells during the first four days after which CD3+ cells show rapid expansion with cytotoxic cells expressing the NKG2D receptor (CD314) dominating the harvested cell product. A subset of cells in this population co-expresses CD3 and CD56. For historical reasons, the CD3+CD56+ subset is tracked as an indicator of the overall expansion of the cytotoxic cells present in the culture.

### *Preparation of Reagents*

*Preparation of AIM-V Medium with 5% heat-inactivated Human AB Serum.*

AIM-V medium is supplied by its manufacturer in 10 liter bags. A fresh bag of medium should be used for each culture. A completed CIK Cell Medium label will be affixed to the bag when medium preparation begins. Each bag prepared with Human AB serum is considered a unique lot of culture medium. It is designated for use with the intended recipient's CIK cell culture only. Then, aseptically, heat-inactivated Human AB Serum will be added to the AIM-V medium at a concentration of 5%. The prepared CIK cell Medium will be stored at 2-8°C, shielded from light.

*Preparation of Interferon-$\gamma$*

This working dilution will be prepared on the day of culture and must be used within 24 hours of preparation. Vials of Interferon $\gamma$ containing 100 mcg or 2 million IU in 0.5 ml and stored at 20°C will be used for its preparation. The content of the Interferon vial (0.5 ml) will be added to 19.5 ml sterile water to obtain a working dilution will a final concentration of 100,000 IU/ml or 5 mcg/ml.

*Preparation of Proleukin IL-2*

This working solution stock should be prepared at least 14 days prior to use to allow for completion of sterility testing. Vial with concentration of 1.0 mg or, 21 million IU/vial and stored at -20°C will be used for its preparation. Using sterile technique, 1 ml of 100 mM Acetic Acid (R&D Systems) will be used to reconstitute the vial of IL-2. The contents of the reconstituted vial will be added back to the remaining 9 ml of 100 mM acetic acid to have a working dilution at $2.1 \times 10^6$ IU/ml.

20 aliquots of 1 ml will be prepared and frozen in 2 ml cryovials. Approximately 1 ml of the IL-2 will be reserved for sterility testing.

*Preparation of Orthoclone OKT-3 Sterile Solution*

Stock solution and working dilution are prepared at least 14 days prior to use in order to allow completion of sterility testing. 1 ml OKT3 will be taken from the manufacturer's vial to prepare frozen aliquots of Working Dilution. The remainder of the stock solution will be frozen at -80°C as 1 ml aliquots in cryovials after being labeled adequately.

OKT-3 working dilution at 1 mg/ml will be prepared by adding 1 ml of stock solution (from fresh vial or thawed aliquot above) to 19 ml of Normosol (1:20 dilution). Then 3 ml of that solution will be added to 27 ml Normosol (1:200 dilution total) to obtain a final working dilution 5 $\mu$g/ml. The working dilution will be frozen in eight 5 ml cryovials with 3.5 ml/ vial that will be labelled adequately. Samples of the residual working dilution will be taken for sterility testing.

### CIK Expansion

*Culture Inoculation - Day 0*

If culture medium has been refrigerated, allow it to warm to room temperature before adding cells to avoid cell stress.

For Culture Preparation using fresh cells, samples will be taken first for NC count, Hct, differential, viability, ABO/Rh, if flow cytometry if not done previously. Then the product will be weighted, and the volume will be calculated based on 1.06 g/ml, if not done previously.

CIK cell cultures will be inoculated at $2 \times 10^6$ viable TNC per ml with 200 ml of culture volume in each cell culture bag (*i.e.,* $400 \times 10^6$ cells/bag). In order to assure that sufficient culture volume is prepared for the protocol, the total volume to prepare should be determined as 200 ml x (number of bags specified + 0.5 bag).

Required volumes of cell suspension and interferon-γ will be added to the medium filled transfer pack and mix gently but thoroughly after each. The top 1/3 of the 5L cell culture bags will be clamped off in order to create a surface area for gas exchange appropriate for a 200 ml culture Using a sterile docking device and tared balance 212 g (200 ml) of culture suspension will be transferred to each culture bag. All CIK Cell Culture bags will be labeled adequately and placed in 37°C incubator.

*Initiating CIK Cell Expansion - Day + 1*

IL-2 and OKT3 will be added to the culture 18-24 hours after inoculation to provide the necessary signals for T cell expansion in culture. IL-2 will be added to a final concentration of 300 IU/ml and OKT3 will be added to a final concentration of 50 ng/ml in the culture bags.

*Maintaining Cell Expansion - Days +4 through Culture Harvest*

CIK cell cultures are formulated to promote the growth and differentiation of T cell subpopulation present in the inoculum. Consequently, other cell types do not survive and the cultures initially show a dramatic decrease in both cell number and viability with the nadir occurring on Day +3 or Day +4 after which both the cell number and viability increase. The surviving cells will be predominantly CD3+ (T cells).

Cultures will be removed from the incubator and assessed for cell count, viability, and subset representation every 3-4 days to determine culture growth and activity. To avoid unnecessary entries into all the culture bags, one bag will be used as the reference bag for the duration of the culture.

When the cell concentration reaches $3 \times 10^6$ cells/ml, the need for additional medium based on anticipated cell growth rate before the next scheduled count should be evaluated. For optimal growth, the cell concentration should not exceed $4 \times 10^6$ cells/ml. Regardless of the cell concentration at the time of counting, IL-2 must be added every 3-4 days to sustain expansion.

*Pre-Harvest Preparation*

Three days prior to culture harvest (*e.g.*, Day +18 of a 21-day culture), is the final entry into the culture bags until harvest. Cell count and viability should be performed. Culture medium and IL- 2 should be added as needed and samples from each bag for pre-harvest sterility testing and mycoplasma testing should be prepared.

**CIK Cell Culture Harvest Preparation**

Prior to harvest. Samples are taken for sterility testing, mycoplasma, endotoxin, NC & viability post wash Cytotoxicity assay, flow cytometry.

*Preparation of vvDD/CIK-CRX100 Suspension*

Once the CIK cells have completed expansion and fulfilled release requirements, they are harvested, washed twice and re-suspended in Normosol (Hospira) and 1% human serum albumin and placed in 'blood bags'. Samples are removed and sent to the Clinical Laboratories for sterility testing, phenotypic analysis and cytotoxicity assays.

The Bags containing the harvested CIK cells will then be taken into the Biosafety/Isolation room, and a vaccinated technician will prepare the material by thawing and diluting the virus, as required for the designated dose. Each dose will be carefully injected through the septum of the bags containing the CIK cells.

The production of the CRX100 final product includes the thaw of the vvDD bulk drug substance and combination of the virus with the CIK cells in the final infusion bag as well as the completion of the rapid release testing and generation of the interim COA.

*Thaw of vvDD Virus*

The calculated volume of vvDD to achieve a MOI of XX will be thawed in a 37°C water bath with continuous swirling of vial during the thawing process. The vial is removed promptly when the last ice crystals have thawed.

**Combination of vvDD with bulk cell substance**

*CRX100 Assessment and Release*

The CRX100 will remain in quarantine until all release testing has been completed and all release criteria have been met.

*Identity/Purity assay:*

A representative sample at each monitoring point will be assessed by this flow cytometry-based assay. Through surface staining (CD3, CD56, NKG2D), this assay can detect the levels of expression of the specified markers from the cell population. The protocol for this assay has been provided by BioEclipse and will be qualified at the ACTL. Frequency of X should be NMT X%.

*Appearance Assay:*

The final CIK bulk cell substance will be examined visually to assess appearance. Acceptance criterion for this assay is confirmation of a colorless to slightly yellow appearance. The bag integrity will be verified to be intact and the label information will be verified to be accurate.

*Potency Assay:*

The potency assay is the number of NKG2D+ cells in the CD3+CD56+ CIK cell preparation. The CD3+CD56+ CIK cells should represent at least 4% of the total cell population. The potency of the CIK cell drug substance is an independent combination effect of 1) CIK cells binding to tumor cells via the NKG2D receptor on their own surface and the NKG2D ligands on the surface of the tumor cells, which initiates a perforin (DAP 10 pathway) cytotoxic response, and 2) the ability of CD3+CD56+ CIK cells to chemotactically enter the tumor microenvironment along a tumor cell initiated chemokine gradient to release the oncolytic virus into the tumor. Since infection of the tumor cells with the vvDD replicating oncolytic virus results in expression of NKG2D ligand on the surface of the tumor cell, the core potency is centered around the virus infecting a tumor cell. Thus, CD3+CD56+ cells with NKG2D receptor are recognized as containing all of the elements to reach the tumor cell and amplify the signal to recruit more of the therapeutic. The vvDD virus has the ability to enter and replicate in all tumor cells with an active thymidine kinase pool and RAS pathway. This is represented in > 70% of tumors. We have performed animal models with CRX100 at 1:1 MOI and 10:1 MOI of virus to CIK cells with no difference in potency. We have also performed in vitro cytotoxicity assays with killing of UCI 101 and a lymphoma cell line that demonstrated the synergy of the cell/virus combination at very low levels of effector to target ratios.

**RESULTS**

1 Liter culture studies

*Hold study 1.* PBMCs were used to expand and select NKT, NK, and T cells in a 1liter gas permeable bag. Cells were harvested at day 32 of culture, washed, split placed into one of two non-gas-permeable bags. Samples were stored in the dark with no agitation at either 22°C or 37°C. Samples were withdrawn and tested at time points from 0 to 72 hours. Viability was assessed at RT and 37°C by two methods: Trypan blue exclusion and Sytox blue reagent in flow cytometry. The percentage of CIK in the population, defined as CD3+/CD56+, cells were also measured. The percentage of CIK cells that express CD314 was also measured. This stability study demonstrated that expanded immune cells remain above 86% viability with no significant change in % CIK at RT for up to 48 hours. An increase in expression of CD314 by CIK cells was seen. Cells stored at 37°C dropped below 70% Trypan blue viability by 12 hours. Results are shown in Table 4.

**Table 4. Hold study 1 - CIK cells at ambient room temperature (22°C) or 37°C**

| | % Viability (Counting Chamber) | | % CIK Cells (CD3+/CD56+) | | % CD314+ All Cells | | % CD314+ CIK Cells | | Appearance | |
|---|---|---|---|---|---|---|---|---|---|---|
| Time [hours] | Bag 1 (RT) | Bag 2 (37°C) | Bag 1 (RT) | Bag 2 (37°C) | Bag 1 (RT) | Bag 2 (37°C) | Bag 1 (RT) | Bag 2 (37°C) | Bag 1 (RT) | Bag 2 (37°C) |
| 0 | 98.5 | 98.5 | 14.0 | 14.0 | 20.9 | 20.9 | 50.9 | 50.9 | OK | OK |
| 2 | 96.5 | 91.2 | | | | | | | OK | OK |
| 4 | 87.1 | 80.1 | 12.5 | 12.0 | 21.1 | 14.5 | 25.9 | 40.5 | OK | OK |

(continued)

| | % Viability (Counting Chamber) | | % CIK Cells (CD3+/CD56+) | | % CD314+ All Cells | | % CD314+ CIK Cells | | Appearance | |
|---|---|---|---|---|---|---|---|---|---|---|
| Time [hours] | Bag 1 (RT) | Bag 2 (37°C) | Bag 1 (RT) | Bag 2 (37°C) | Bag 1 (RT) | Bag 2 (37°C) | Bag 1 (RT) | Bag 2 (37°C) | Bag 1 (RT) | Bag 2 (37°C) |
| 6 | 81.9 | 76.7 | | | | | | | OK | OK |
| 8 | 83.7 | 76.6 | 13.0 | 13.2 | 22.0 | 21.7 | 51.4 | 59.7 | OK | OK |
| 12 | 85.6 | 69.5 | | | | | | | OK | OK |
| 20 | 76.8 | 5.2 | 14.0 | | 30.8 | | 72.6 | | OK | OK |
| 24 | 82.4 | 15.8 | 11.0 | | 26.1 | | 66.0 | | OK | OK |
| 36 | 86.5 | 16.5 | | | | | | | OK | OK |
| 48 | 86.5 | 16.5 | 11.2 | | 24.2 | | 64.3 | | OK | OK |
| 72 | 50.8 | 6.1 | 7.0 | | 18.8 | | 66.7 | | OK | OK |

*Hold study 2.* The same culture of PBMCs used to expand and select NKT, NK, and T cells from hold study 1 was used. Cells were harvested at day 39 of culture, washed, split placed into one of two non-gas-permeable bags. Samples were stored in the dark with no agitation at either 22°C or 37°C. Samples were withdrawn and tested at time points from 0 to 72 hours. Viability was assessed at RT and 37°C by two methods: Trypan blue exclusion and Sytox blue reagent in flow cytometry. The % CIK cells were also measured. This stability study demonstrated that expanded immune cells remain above 86% viability with no significant change in % CIK at RT for up to 48 hours. Cells stored at 37°C dropped below 70% Trypan blue viability by 12 hours. An increase in expression of CD314 by CIK cells was seen for cells held at ambient temperature and at 37°C. Results are presented in Table 5.

Table 5. Hold study 2 - CIK cells at ambient room temperature (22°C) or 37°C

| | % Viability (Counting Chamber) | | % CIK Cells (CD3+/CD56+) | | % CD314+ | | % CD314+ CIK Cells | | Appearance | |
|---|---|---|---|---|---|---|---|---|---|---|
| Time [hours] | Bag 1 (RT) | Bag 2 (37°C) | Bag 1 (RT) | Bag 2 (37°C) | Bag 1 (RT) | Bag 2 (37°C) | Bag 1 (RT) | Bag 2 (37°C) | Bag 1 (RT) | Bag 2 (37°C) |
| 0 | 94.4 | 94.4 | 8.6 | 8.6 | 14.6 | 14.6 | 16.9 | 16.9 | OK | OK |
| 2 | 89.1 | 86.2 | | | | | | | OK | OK |
| 4 | 88.5 | 72.8 | 7.3 | 5.0 | 12.8 | 12.2 | 56.1 | 56.1 | OK | OK |
| 6 | 79.1 | 55.8 | | | | | | | OK | OK |
| 8 | 83.7 | 49.5 | 6.8 | 4.4 | 11.8 | 12.1 | 58.6 | 62.0 | OK | OK |
| 12 | 93.8 | 52.7 | 7.7 | 4.2 | 13.2 | 11.7 | 54.4 | 66.0 | OK | OK |
| 24 | 84.4 | 17.8 | 6.2 | | 14.7 | | 63.9 | | OK | OK |
| 48 | 87.3 | 8.2 | 6.2 | | 12.9 | | 59.0 | | OK | OK |
| 72 | 73.2 | 10.0 | 5.8 | | 8.0 | | 51.2 | | OK | OK |

### 5L culture studies

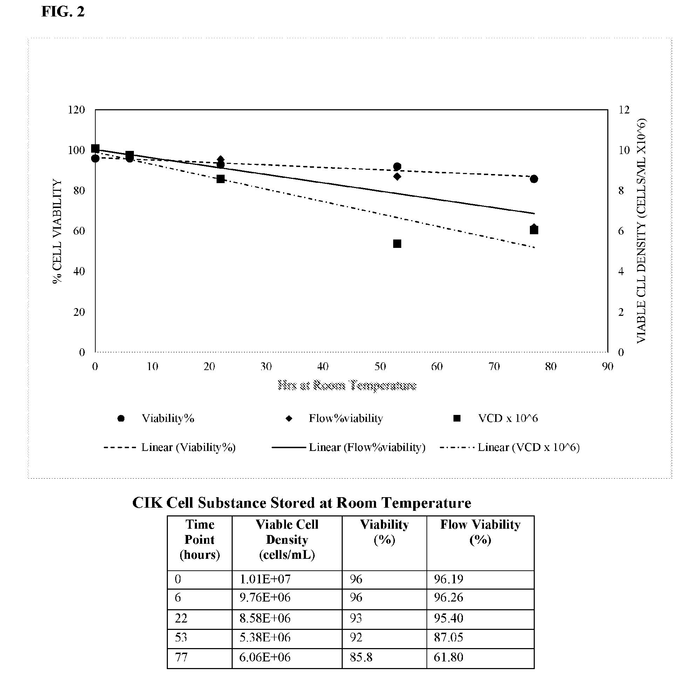

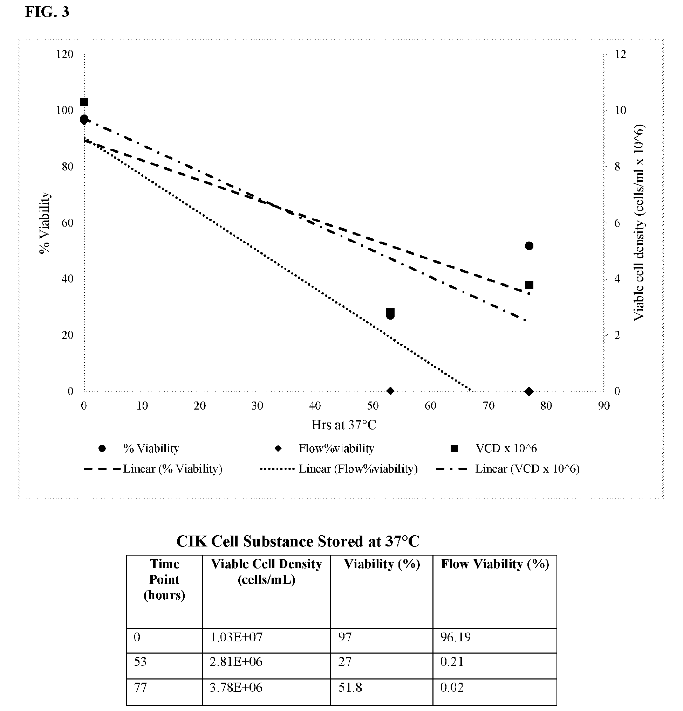

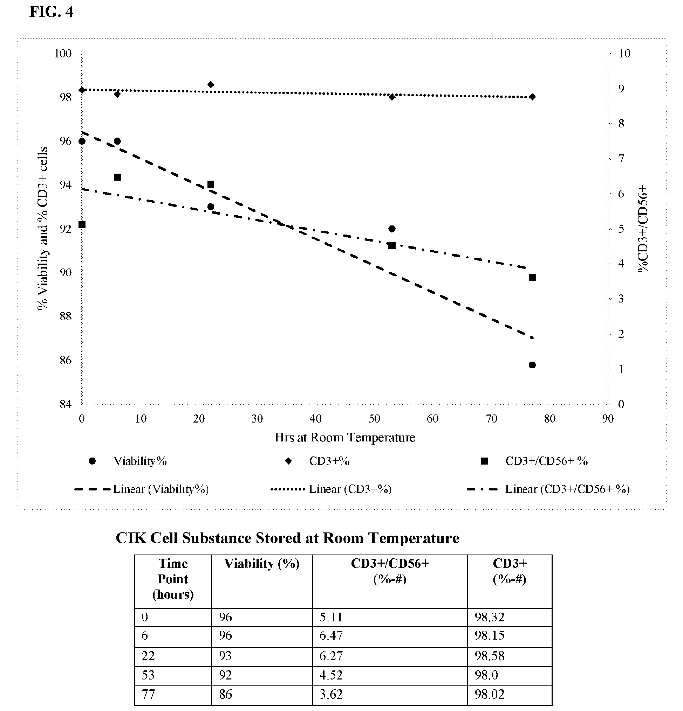

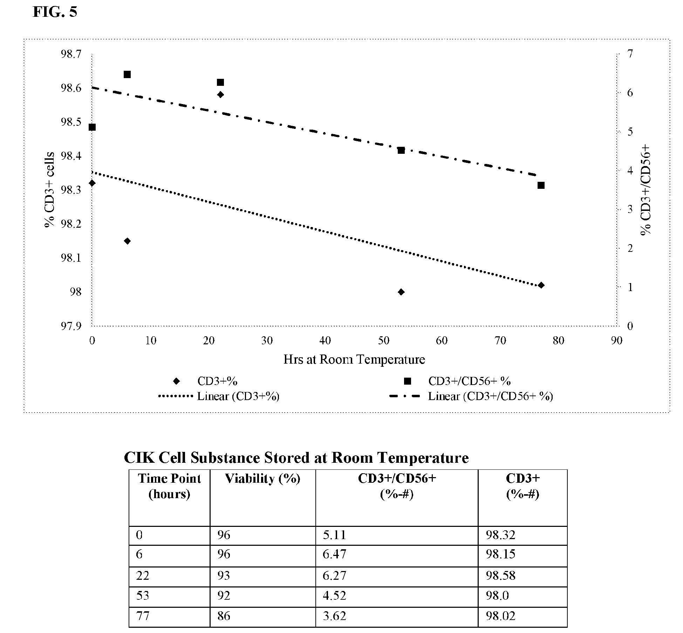

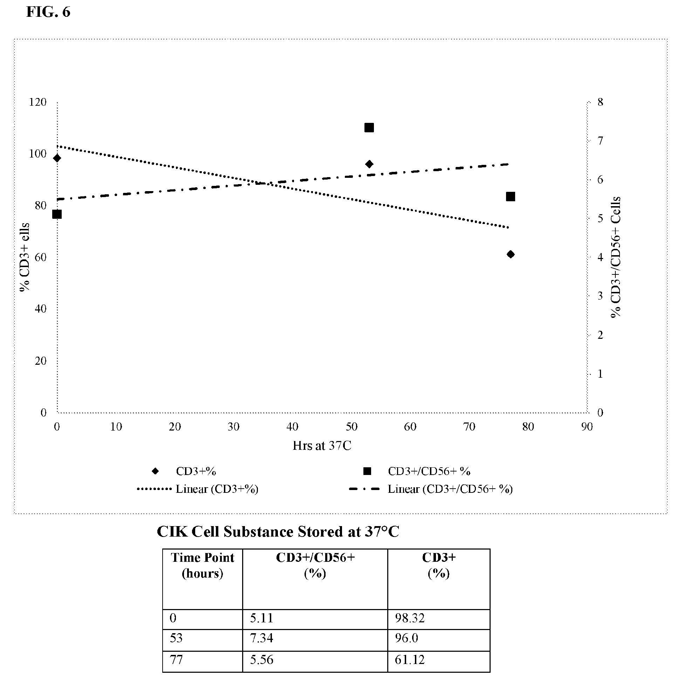

PBMCs were used to expand and select NKT, NK, and T cells in a 5 liter culture. On day 21, after initial sampling was performed, two culture bags were connected to the COBE 2991 cell processor. Cells from the culture were concentrated and washed three times with harvest buffer Plasma-Lyte© A with 0.5% HSA). Washed cells were then placed into one of two non-gas-permeable bags. Samples were stored in the dark with no agitation at either room temperature (22°C) or 37°C. 0.5mL samples were withdrawn and tested at time points shown in Table 6 from 0 to 77 hours. Viability was assessed at RT and 37°C by two methods: Trypan blue exclusion and Sytox blue in flow cytometry. The percent CIK (defined as CD3+/CD56+) were measured as were the percent CD3+ cells and percent CD314+ cells. This hold study indicated that expanded immune cells remained above 85.8% viable at 77 hours with no significant drop in % CIK cells for the duration of the study. Cells stored at 37°C, alternatively, exhibited a Trypan blue viability of 27% at 53 hours. The percent CD314+ cells in the population increased over time. Results are presented in Table 6 and FIGs. 2-6.

**Table 6. Stability of cells from 5L culture - CIK cells at ambient room temperature (22°C) or 37°C**

| Condition | Time Point (hrs) | Viable Cell Density (cells/mL) | Viability (%) | Flow Viability (%-#) | CD3+/CD56+ (%-#) | CD3+ (%-#) | CD314+ (%-#) | Appearance |
|---|---|---|---|---|---|---|---|---|
| **Room Temp** | 0 | 1.01e7 | 96 | 96.19 | 5.11 | 98.32 | 40.71 | OK |
| | 6 | 9.76e6 | 96 | 96.26 | 6.47 | 98.15 | 33.33 | OK |
| | 22 | 8.58e6 | 93 | 95.40 | 6.27 | 98.58 | 41.89 | OK |
| | 53 | 5.38e6 | 92 | 87.05 | 4.52 | 98.0 | 50.12 | OK |
| | 77 | 6.06e6 | 85.8 | 61.80 | 3.62 | 98.02 | 51.53 | OK |
| **37°C** | 0 | 1.03e7 | 97 | 96.19 | 5.11 | 98.32 | 40.71 | OK |
| | 53 | 2.81e6 | 27 | 0.21 | 7.34 | 96.0 | 53.35 | OK |
| | 77 | 3.78e6 | 51.8 | 0.02 | 5.56 | 61.12 | 50.00 | OK |

**Viability studies of cells from 5L virus infected culture held at ambient temperature or 37°C**

PBMCs were used to expand NK, NKT and T cells in a 5 liter culture as described above. After 21 days of culture, cells were infected with an oncolytic vaccinia virus with deletions of the TK and VGF genes (vvDD) at an MOI of 0.01 (low dose) or 1.0 (high dose) at 37°C for two hours. Cells were then placed in gas impermeable bags and held at ambient temperature or 37°C as described above. At various times, samples were removed and analyzed for cell viability and for percent CIK cells (CD3+/CD56+), CD3+ and CD314+ cells.

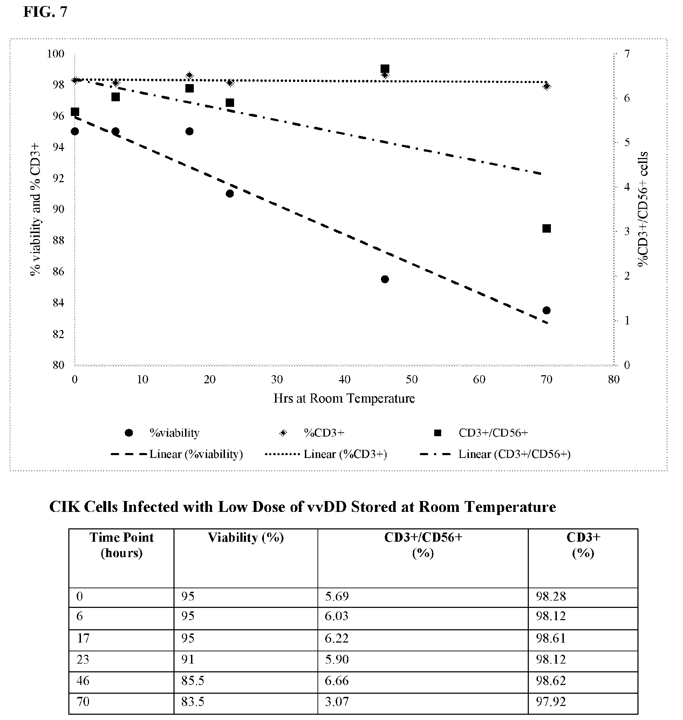

Results of the low dose infected culture is presented in Table 7 and FIG. 7. Cell viability remained high over 70 hours when held at room temperature. There was a drop in CIK cells and an increase in CD314+ cells over the course of the study. The clinically acceptable percent CIK cells is 4%.

**Table 7. Low Dose vvDD at Ambient Room Temperature (22°C) and 37°C**

| Condition | Time Point (hrs) | Viable Cell Density (cells/mL) | Viability (%) | Flow Viability (%-#) | CD3+/CD56+ (%-#) | CD3+ (%-#) | CD314+ (%-#) | Appearance |
|---|---|---|---|---|---|---|---|---|
| **Low Dose/ Room Temp** | 0 | 8.77e6 | 95 | 96.19 | 5.69 | 98.28 | 36.94 | OK |
| | 6 | 9.85e6 | 95 | 96.60 | 6.03 | 98.12 | 20.56 | OK |
| | 17 | 9.85e6 | 95 | 94.76 | 6.22 | 98.61 | 45.25 | OK |
| | 23 | 8.19e6 | 91 | 84.08 | 5.90 | 98.12 | 31.60 | OK |
| | 46 | 8.17e6 | 85.5 | 89.06 | 6.66 | 98.62 | 49.70 | OK |
| | 70 | 6.17e6 | 83.5 | 59.53 | 3.07 | 97.92 | 48.19 | OK |

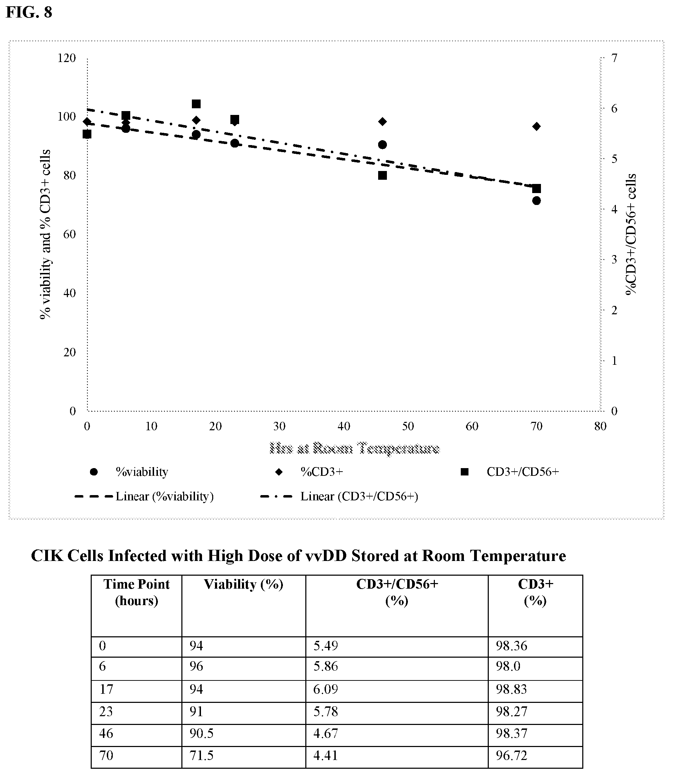

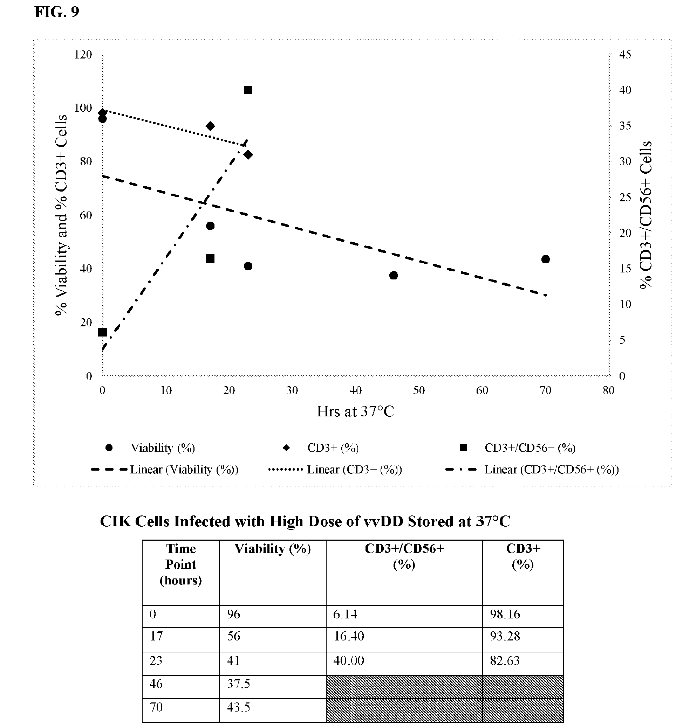

Results of the high dose infected culture is presented in Table 8 and FIG. 8 and 9. Cell viability remained high over 70 hours when held at room temperature. Viability of 70% was acceptable for clinical applications. As with the low dose, there was a drop in CIK cells and an increase in CD314+ cells over the course of the study.

**Table 8. CIK Cells + high dose vvDD at ambient Room Temperature (22°C) and 37°C**

| Condition | Time Point (hrs) | Viable Cell Density (cells/mL) | Viability (%) | Flow Viability (%-#) | CD3+/CD56+ (%-#) | CD3+ (%-#) | CD314+ (%-#) | Appearance |
|---|---|---|---|---|---|---|---|---|
| High Dose/ Room Temp | 0 | 1.00e7 | 94 | 98.48 | 5.49 | 98.36 | 35.04 | OK |
| | 6 | 9.43e6 | 96 | 95.71 | 5.86 | 98.0 | 25.98 | OK |
| | 17 | 9.80e6 | 94 | 91.32 | 6.09 | 98.83 | 42.29 | OK |
| | 23 | 8.60e6 | 91 | 81.56 | 5.78 | 98.27 | 42.08 | OK |
| | 46 | 5.91e6 | 90.5 | 78.93 | 4.67 | 98.37 | 49.34 | OK |
| | 70 | 4.75e6 | 71.5 | 38.67 | 4.41 | 96.72 | 59.01 | OK |
| High Dose/ 37°C | 0 | 1.01e7 | 96 | 93.21 | 6.14 | 98.16 | 39.23 | OK |
| | 17 | 5.46e6 | 56 | 2.38 | 16.40 | 93.28 | 66.59 | OK |
| | 23 | 3.13e6 | 41 | 0.14 | 40.00 | 82.63 | 73.68 | OK |
| | 46 | 2.75e6 | 37.5 | | | | | OK |
| | 70 | 3.16e6 | 43.5 | | | | | OK |

NKT, NK and T cells were prepared from a second five-liter culture. Cells were mixed with a high dose of virus at 37°C and stored in the dark without agitation at either room temperature or 37°C. Samples were obtained and assayed for viability and cell surface markers as described above. Results are shown in Table 9. Viability remained high and the present CIK cells remained constant for at least 48 hours.

**Table 9. CIK Cells + high dose vvDD at ambient Room Temperature (22°C) and 37°C**

| Condition | Time Point (hrs) | Viable Cell Density (cells/mL) | Viability (%) | Flow Viability (%-#) | CD3+/CD56+ (%-#) | CD3+ (%-#) | CD314+ (%-#) | Appearance |
|---|---|---|---|---|---|---|---|---|
| High Dose/ Room Temperature | 0 | 5.78e6 | 83.8 | 95.8 | 10.77 | 98.97 | 72.03 | OK |
| | 48 | 5.01e6 | 80.0 | 89.40 | 10.50 | 99.33 | 76.55 | OK |
| | 108 | 3.42e6 | 48.0 | 2.47 | 6.34 | 88.78 | 83.41 | OK |
| High Dose/ 37°C | 0 | 5.78e6 | 83.8 | 95.8 | 10.77 | 98.97 | 72.03 | OK |
| | 48 | 2.86e6 | 52.0 | 0.16 | 10.78 | 64.70 | 58.82 | OK |
| | 108 | 2.98e6 | 40.0 | 0.40 | 0.00 | 6.67 | 3.33 | OK |

**Example 2**

The stability and activation of immune cells was further investigated. Five-liter cultures were grown and processed as described in Example 1 with the further modifications as described below.

PBMCs were used to expand NK, NKT and T cells in a 5 liter culture as described in Example 1. After 21 days of culture, cells were infected with an oncolytic vaccinia virus with deletions of the TK and VGF genes (vvDD) at an MOI of 1.0 (high dose) at 37°C for two hours. Cells were then placed in gas impermeable bags and held at ambient temperature or 37°C as described above. At various times samples were removed and analyzed for cell viability and for percent CIK cells (CD3+CD56+), CD3+ and CD314+ cells.

Results are presented in Table 10. Cell viability remained high over 48 hours when held at room temperature. There was a drop in CIK cells and an increase in CD314+ cells over the course of the study.

**Table 10. CIK Cells + high dose vvDD at ambient Room Temperature (22°C) and 37°C**

| Condition | Time Point (hrs) | Viable Cell Density (cells/mL) | Viability (%) | Flow Viability (%-#) | CD3+/CD56+ (%-#) | CD3+ (%-#) | CD314+ (%-#) | Appearance | Gram Stain |
|---|---|---|---|---|---|---|---|---|---|
| Harvest Complete | | 3.37E+06 | 89 | 99.13 | 22.96 | 96.13 | 73.26 | OK | No Organisms Seen |
| High Dose/ Room Temp | 0 | 7.80E+06 | 88 | 98.63 | 23.93 | 96.39 | 77.32 | OK | No Organisms Seen |
| | 2 | 6.98E+06 | 91 | | | | | OK | |
| | 5 | 7.33E+06 | 91 | | | | | OK | |
| | 8 | 7.09E+06 | 91 | 96.55 | 21.63 | 95.57 | 73.28 | OK | |
| | 16 | 6.60E+06 | 89 | 95.87 | 20.85 | 96.25 | 80.95 | OK | |
| | 22 | 5.95E+06 | 87 | 96.34 | 21.4 | 97.76 | 78.11 | OK | No Organisms Seen |
| | 32 | 6.60E+06 | 84 | 92.96 | 17.08 | 95.72 | 76.96 | OK | |
| | 40 | 6.54E+06 | 87 | 75.29 | 19.75 | 96.79 | 79.34 | OK | |
| | 48 | 6.37E+06 | 83 | 86.35 | 17.23 | 97.47 | 78.39 | OK | No Organisms Seen |
| High Dose/ 37°C | 0 | 7.80E+06 | 88 | 98.63 | 23.93 | 96.39 | 77.32 | OK | |
| | 2 | 1.07E+07 | 93 | | | | | OK | |
| | 5 | 8.33E+06 | 90 | | | | | OK | |
| | 8 | 6.36E+06 | 87 | 90.67 | 23.34 | 96.11 | 73.38 | OK | |
| | 16 | 3.73E+06 | 54 | 38.27 | 30.98 | 93.2 | 59.86 | OK | |
| | 22 | 1.30E+06 | 21 | 6.01 | 55.93 | 99.12 | 87.66 | OK | No Organisms Seen |
| | 32 | | | | | | | | |
| | 40 | | | | | | | | |
| | 48 | | | | | | | | |

In a second arm of the study, cells were stored in Plasma-Lyte© at room temperature for 24 hours prior to infection with vaccinia virus. As seen in Table 11, cells remained viable at room temperature for the 24-hour hold period before viral infection and remained viable for at least 24 hours post infection. There was a drop in viability of cells maintained at 37°C following infection. Notably, the percentage of CIK cells in the population was maintained and the percentage of NKG2D+ (CD314+) cells increased.

**Table 11. Ambient Room Temperature (22°C) hold before infection.**

| Condition | Time Point (hrs) | Viable Cell Density (cells/mL) | Viability (%) | Flow Viability (%-#) | CD3+/CD56+ (%-#) | CD3+ (%-#) | CD314+ (%-#) | Appearance | Gram Stain |
|---|---|---|---|---|---|---|---|---|---|
| Harvest Complete | | 3.37E+06 | 89 | 99.13 | 22.96 | 96.13 | 73.26 | OK | No Organisms Seen |

(continued)

| Condition | Time Point (hrs) | Viable Cell Density (cells/mL) | Viability (%) | Flow Viability (%-#) | CD3+/CD56+ (%-#) | CD3+ (%-#) | CD314+ (%-#) | Appearance | Gram Stain |
|---|---|---|---|---|---|---|---|---|---|
| Pre-vvDD addition - CIK stored at RT | 2 | 7.48E+06 | 93 | 99.09 | 18.04 | 96.37 | 73.05 | OK | |
| | 7 | 8.03E+06 | 93 | 97.44 | 20.39 | 95.17 | 71.71 | OK | |
| | 10 | 7.82E+06 | 90 | 97.13 | 19.74 | 95.41 | 78.64 | OK | |
| | 18 | 7.66E+06 | 90 | 97.43 | 21.34 | 95.72 | 83.72 | OK | |
| | 24 | 8.94E+06 | 90 | 95.54 | 22.55 | 97.21 | 80.83 | OK | No Organisms Seen |
| vvDD addition | -2 | 6.74E+06 | 90 | 95.85 | 21.88 | 97.16 | 80.55 | OK | No Organisms Seen |
| High Dose/ Room Temp | 0 | 6.11E+06 | 91 | 95.53 | 18.27 | 95.15 | 77.07 | OK | No Organisms Seen |
| | 3 | 6.59E+06 | 87 | 94.9 | 20.63 | 97.18 | 67.47 | OK | |
| | 8 | 7.21E+06 | 89 | 91.83 | 18.04 | 94.89 | 75.33 | OK | |
| | 16 | 7.34E+06 | 90 | 82.01 | 20.39 | 97.31 | 80.91 | OK | |
| | 24 | 7.45E+06 | 90 | 84.87 | 18.3 | 96.2 | 79.41 | OK | |
| High Dose/ 37°C | 0 | 6.11E+06 | 91 | 95.53 | 18.27 | 95.15 | 77.07 | OK | No Organisms Seen |
| | 3 | 5.41E+06 | 84 | 88.68 | 18.78 | 97.16 | 57.12 | OK | |
| | 8 | 3.64E+06 | 69 | 63.3 | 16.47 | 95.35 | 75.15 | Clumps | |
| | 16 | 1.74E+06 | 26 | 4.49 | 51.38 | 96.18 | 89.92 | OK | |
| | 24 | 2.46E+06 | 36 | 0.24 | 35.16 | 71.42 | 65.93 | OK | No Organisms Seen |

In a third arm of the study, cells were stored in Plasma-Lyte© at 2-8°C for 24 hours prior to infection with vaccinia virus. As seen in Table 12, cells remained viable at 2-8°C for the 24-hour period before viral infection and remained viable for at least 24 hours at room temperature post infection. There was a drop in viability of cells maintained at 37°C following infection. There was a drop in viability of cells maintained at 37°C following infection. Notably, the percentage of CIK cells in the population was maintained at 2-8°C hold and increased post-infection. The percentage of NKG2D+ (CD314+) cells increased during the 2-8°C hold.

**Table 12. Ambient Room Temperature (22°C) hold before infection.**

| Condition | Time Point (hrs) | Viable Cell Density (cells/mL) | Viability (%) | Flow Viability (%-#) | CD3+/CD56+ (%-#) | CD3+ (%-#) | CD314+ (%-#) | Appearance | Gram Stain |
|---|---|---|---|---|---|---|---|---|---|
| Harvest Complete | | 3.37E+06 | 89 | 99.13 | 22.96 | 96.13 | 73.26 | OK | No Organisms Seen |
| Pre-vvDD addition - CIK stored at 2-8C | 2 | 6.74E+06 | 91 | 97.03 | 21.69 | 95.71 | 80.67 | OK | |
| | 7 | 7.43E+06 | 93 | 96.22 | 21.24 | 95.42 | 61.44 | OK | |
| | 10 | 6.79E+06 | 91 | 94.59 | 19.98 | 95.93 | 80.32 | OK | |
| | 18 | 5.69E+06 | 92 | 97.74 | 20.75 | 96.03 | 83.6 | OK | |
| | 24 | 6.89E+06 | 92 | 97.11 | 21.88 | 97 | 80 | OK | No Organisms Seen |

(continued)

| Condition | Time Point (hrs) | Viable Cell Density (cells/mL) | Viability (%) | Flow Viability (%-#) | CD3+/CD56+ (%-#) | CD3+ (%-#) | CD314+ (%-#) | Appearance | Gram Stain |
|---|---|---|---|---|---|---|---|---|---|
| vvDD addition | -2 | 7.85E+06 | 92 | 97.18 | 20.32 | 96.64 | 79.42 | OK | No Organisms Seen |
| High Dose/ Room Temp | 0 | 7.02E+06 | 90 | 97 | 19.61 | 95.16 | 68.99 | OK | No Organisms Seen |
| | 3 | 5.96E+06 | 90 | 94.28 | 23.64 | 96.36 | 78.47 | OK | |
| | 8 | 6.46E+06 | 87 | 96.4 | 19.69 | 95.92 | 64.27 | OK | |
| | 16 | 7.96E+06 | 92 | 83.92 | 20.98 | 98.32 | 83.15 | OK | |
| | 24 | 6.86E+06 | 89 | 78.67 | 25.5 | 99.02 | 80.51 | OK | No Organisms Seen |
| High Dose/ 37°C | 0 | 7.02E+06 | 90 | 97 | 19.61 | 95.16 | 68.99 | OK | No Organisms Seen |
| | 3 | 5.86E+06 | 87 | 92.91 | 20.28 | 97.24 | 63.32 | OK | |
| | 8 | 5.40E+06 | 83 | 82.52 | 18.34 | 98.4 | 34.4 | OK | |
| | 16 | 3.10E+06 | 34 | 14.96 | 40.33 | 95.01 | 84.66 | OK | |
| | 24 | 3.20E+06 | 43 | 0.63 | 43.91 | 87.82 | 76.28 | OK | No Organisms Seen |

## Example 3

Degranulation of immune cells and tumor cells after incubation in a gas impermeable bag was evaluated.

**MATERIALS & METHODS**

*Expansion and Shipment of CIK Cells*

Cytokine-induced Killer (CIK) cells were cultured and expanded from human peripheral blood mononuclear cells (PBMCs) at the Advanced Cell Therapy Laboratory (ACTL) in San Diego, CA. PBMCs were obtained from a LeukoPak (AllCells, Alameda, CA) and cultured in suspension in 40 mL of AIM-V media + 5% Human Ab-serum in 1L gas permeable bags. On day 0, cell cultures were treated with 400$\mu$L of 5$\mu$g/mL IFN-$\gamma$. On day 1, 1mg/mL anti-CD3 mAb OKT3 and 100 $\mu$g/mL human recombinant IL-2 was added to the culture bag. Cultures of CIK cells were incubated in a humidified 5% $CO_2$ environment at 37°C. If cell density exceeded $3 \times 10^6$ cells/mL, cultures were diluted with media to return the culture to $2 \times 10^6$ cells/mL. Cultures were re-treated with 300 IU/mL IL-2 every 3-4 days. If the total volume of the expansion bag exceeded 500mL, cultures were split into two 1L expansion bags before additional dilution. On day 21 of culture, cells were washed using a COBE 2991 Cell Processor (Terumo BCT, Lakewood, CO) with Plasma-Lyte$^©$ (Baxter Int., Deerfield, IL). Harvested cells were collected in a 400mL fluid transfer bag (FTB) and diluted with Plasma-Lyte$^©$ + 5% Human Ab-serum until a density of ~$8.57 \times 10^6$ cells/mL was reached. The FTB of harvested immune cells was placed in a container and shipped overnight to the Open Medicine Institute (Mountain View, CA). The FTB was received the following morning.

*Preparation of Effector Cell Line*

A ChemoLock bag spike was inserted into the FTB containing expanded immune cells. A 3mL syringe with LuerLock system was connected to a female ChemoLock port and attached to the bag spike inserted in the FTB. The FTB was then placed flat into a secondary container in a drawer at ambient temperature (22°C) where it remained without agitation. A 2.0 mL sample was withdrawn using the syringe and transferred into a 5.0mL snap-cap tube labeled "Effector Cells." 25 $\mu$L was withdrawn for an initial assessment of viability and cell density was performed using a hemocytometer and Trypan Blue stain (See Analytical Tests - Percent Viability). The percentage of viable cells and initial cell density of immune cells was recorded. The remaining "Effector Cells" were centrifuged at $400 \times g$ for 5 minutes and washed with PBS twice. Cells were then resuspended in AIM-V media (ThermoFisher, Waltham, MA) until cell density reached $8.57 \times 10^6$ cells/mL.

*Preparation & Staining of Target Cell Line*

A 10 mL suspension culture of chronic myelogenous leukemia cells (K-562) was prepared in a T75 flask with AIM-V media. One day prior to reception of the immune cells from ACTL, K-562 cells were split such that cell density remained at $4 \times 10^6$ cells/mL. A 1.2mL sample of K-562 cells was obtained from the T75 flask using a 5 mL glass pipette and transferred into a 1.5 mL tube labeled "Target Cells." Viability and cell density were obtained using a hemocytometer and Trypan Blue stain (*See* Analytical Tests - Percent Viability).

Cell Tracker Orange (CTO) (ThermoFisher, Waltham, MA) was used as a marker for the K-562 cell line. 9 μL of cell culture grade dimethyl sulfoxide (DMSO) (VWR, Radnor, PA) was added to 50 μg of CTO dye. 2 μL of CTO/DMSO solution was added to 2 mL of serum-free AIM-V media to create a 10 μM CTO solution. The remaining "Target Cells" were centrifuged at $400 \times g$ for 5 minutes and resuspended in 1 mL of AIM-V serum free media to reach a concentration of $5 \times 10^6$ cells/mL. 2 mL of CTO solution was added to the cell suspension and incubated for 30 minutes at 37°C in a 5% $CO_2$. K-562 cells were then washed twice with AIM-V media and resuspended in 2 mL media. An additional cell count was performed to confirm a concentration of $5 \times 10^6$ cells/mL was obtained.

*Hour 00 Incubation of Target and Effector Cells*

Harvested immune (effector) cells and K-562 (target) cells were co-incubated at relative concentrations described in Table 13.

5.0 mL snap-cap tubes were labeled 1-6 in accordance with Table 13. Based on the viable cell density determined during counting in the hemocytometer, the requisite volume of target and effector cells were added to their corresponding tube. AIM-V media was then added to each tube if necessary, ensuring that each co-incubation was in exactly 1.0 mL of solution. Target and effector cells were co-incubated in the dark at ambient room temperature for 30 minutes. After 30 minutes, the following analytical tests were performed.

**TABLE 13. Incubation Ratios of Target and Effector cells**

| Sample | E:T Ratio | Effector Cell Population | Target Cell Population |
|---|---|---|---|
| 1 | 1:0 | $0.5 \times 10^6$ | - |
| 2 | 0:1 | - | $0.5 \times 10^6$ |
| 3 | 1:1 | $0.5 \times 10^6$ | $0.5 \times 10^6$ |
| 4 | 10:1 | $5.0 \times 10^6$ | $0.5 \times 10^6$ |
| 5 | 40:1 | $20.0 \times 10^6$ | $0.5 \times 10^6$ |
| 6 | 40:0 | $20.0 \times 10^6$ | - |

*Analytical Tests*

*Percent Viability.* Viability of cells within each FTB was assessed via Trypan Blue exclusion assay. 25 μL of sample was mixed with 25μL of 0.4% Trypan Blue (VWR, Radnor, PA) stain (forming a 1:2 dilution) and incubated for 1 minute before being transferred into a standard Neubauer counting chamber. Live and dead cells were manually counted at 40x magnification using phase contrast. Percent viability was determined as followed:

$$\% \text{ Viability} = (\# \text{ Live Cells})/(\# \text{ Live Cells} + \# \text{ Dead Cells}) \times 100$$

*Flow Cytometry.* Flow Cytometry was used to identify cell surface markers and characterize degranulation of effector cells as they bind to target (K-562) cells and secrete cytotoxic molecules. In brief, degranulation serves as an indicator of the cytotoxicity of effector cells. CD107a (BioLegend, San Diego, CA) was used as a marker of degranulation. Moreover, the following cell surface markers were also assessed: CD3, CD56, and CD314 (BioLegend, San Diego, CA). CTO was used to discriminate between effector and target cells while Sytox Blue (BioLegend, San Diego, CA) indicated viability of each subpopulation. Samples were diluted $2 \times 10^6$ cells/mL. 150 μL of each sample was added per well in a 96-well plate to be processed. Each well was then incubated with 1 μL of each antibody stock (CD3, CD56, CD314, and CD107a) in 50μL Ab-binding buffer (BioLegend, San Diego, CA) at 4°C, washed 2x, and finally resuspended in 100μL

PBS (Corning, Corning, NY) with 2μL of 100x Sytox Blue working stock solution.

*Hour 24 Incubation of Target and Effector Cells*

Co-incubation of K-562 and immune cells was performed again 24 hours later for 30 minutes. Viability and flow cytometry analysis were also performed.

**RESULTS**

Flow Cytometry was used to identify cell surface markers and characterize degranulation of effector cells as they bind to target cells and secrete cytotoxic molecules. Results are presented in Table 14 at T=0 hours (four hours co-incubation in Plasma-Lyte© and Table 15 at T=24 hours (30 minutes co-incubation in media).

**Table 14**

| Group | E:T ratio | % Total Cell Viability | % CD3+/CD56+ (CIK) | % CD107a+ (CIK) | % CD107a+ (K-562) |
|---|---|---|---|---|---|
| 1 | 1:0 | 95.19 | 8.55 | 0.55 | n/a |
| 2 | 0:1 | 20.24 | 0.73 | n/a | 3.37 |
| 3 | 1:1 | 51.60 | 9.31 | 3.54 | 1.65 |
| 4 | 10:1 | 89.59 | 9.65 | 0.84 | 0.49 |
| 5 | 40:1 | 71.52 | 10.06 | 0.49 | 0.00 |
| 6 | 40:0 | 57.06 | 9.79 | 0.00 | n/a |

**Table 15**

| Group | E:T ratio | % Total Cell Viability | % CD107a+ (lymphocytes) | % CD107a+ (K-562) |
|---|---|---|---|---|
| 1 | 1:0 | 94.83 | 0.47 | n/a |
| 2 | 0:1 | 56.16 | n/a | 1.79 |
| 3 | 1:1 | 61.52 | 2.58 | 1.55 |
| 4 | 10:1 | 79.60 | 1.92 | 1.16 |
| 5 | 40:1 | 56.79 | 7.93 | 7.21 |
| 6 | 40:0 | 26.35 | 4.1 | n/a |

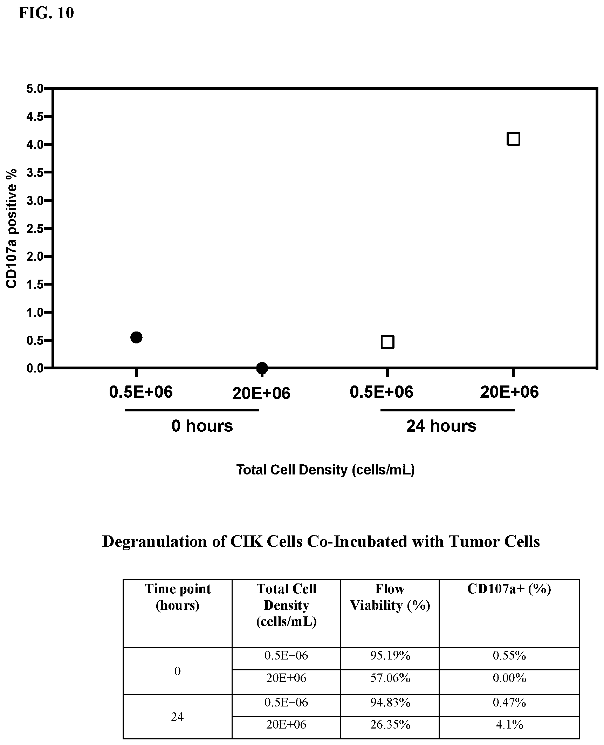

Percentage of CIK cells positive for CD107a at 1:0 and 40:0 ratios (in the absence of K-562 tumor cells) are shown in FIG. 10.

**Example 4.**

The effects of hold-times and viral infection on immune cell stability and activation with a 4°C or room temperature hold was investigated at both a one-liter culture scale and a manufacturing 5 liter scale.

**Materials and Methods**

Summary of CIK cell expansion

*Isolation and Culture of CIK Cells*

Human peripheral blood mononuclear cells (PBMCs) were obtained from a LeukoPak (StemExpress, Folsom, CA) and cultured in suspension in AIM-V media + 5% Human AB serum (ThermoFisher, Waltham, MA). Cells were seeded in 5 L gas permeable bags (Bag#1 and Bag#2) (Charter Medical, Winston-Salem, NC). 200 mL ($2.76 \times 10^6$ cells) were seeded into Bag#1, 200 mL ($2.25 \times 10^6$ cells) were seeded into Bag#2, and 30-40 mL were seeded into Bag#3. On day 0, cell cultures were treated with 2 mL of 5 μg/mL IFN-γ (R&D Systems, Minneapolis, MN). On day 1, 1 mg/mL anti-CD3 mAb OKT3 (Takara Bio, Mountain View, CA) and 100 μg/mL human recombinant IL-2 (R&D Systems, Minneapolis, MN) was added to each culture. Cultures of cytokine-induced killer (CIK) cells were incubated in a humidified 5% $CO_2$ environment at 37°C. Samples of the culture were taken every 3-4 days. Cell counts were obtained using a Trypan blue exclusion assay. Cell surface markers were assayed via flow cytometry. If cell density exceeded $3 \times 10^6$ cells/mL, cultures were diluted with media to return the culture to $2.3 \times 10^6$ cells/mL. Cultures were re-treated with 300 IU/mL IL-2 during each culture access. If the total volume of the expansion bag exceeded 2.5 L, cultures were split into two 5L expansion bags before additional dilution.

*Harvest and Washing of CIK Cells*

On day 20 of culture expansion, immune cells were harvested and washed using a COBE 2991 Cell Processor (Terumo, BCT, Lakewood, CO) and Plasma-Lyte© + 0.5% Human AB serum. Harvested cells were collected in a 400 mL fluid transfer bag (FTB) and diluted with additional Plasma-Lyte© until a density of $6.0 \times 10^6$ cells/mL was reached. The total cell count, percent yield, and initial volume of the harvested cells was determined. The harvested immune cell Plasma-Lyte© suspension was split evenly between two FTBs using a tube sealer. Bag spikes with a ChemoLock system (ICU Medical, San Clemente, CA) were inserted into each FTB. Gas exchange specifications of the culture expansion bags and FTBs are as described in Example 1.

*Hold Process of CIK Cells*

Cell suspensions were held at 4°C, ambient room temperature (22°C $\pm$ 5 °C) or 37°C. FTBs were maintained in the dark without agitation while lying flat within a secondary container. Samples were obtained from each FTB at various time points and the following assays will be performed: % viability, flow cytometry, and appearance.

*Sampling of Culture for cell counting and flow cytometry*

Immediately following preparation of both FTBs, 0.5 mL samples were taken from each bag and an initial cell count was performed. During each culture access, a 3 mL syringe was connected to a ChemoLock port with a male ChemoLock ending. Prior to access, the ChemoLock surfaces on the bag spike and 3 mL syringe were cleaned with an IPA wipe. Cultures were removed from their secondary containers and brought to a homogenous suspension through a series of 360° nutating movements immediately preceding sampling. The ChemoLock ports on the bag and syringe were connected while the bag was held vertically. The bag was inverted such that syringe was now at the bottom and the bag spike inside the bag is submerged in product. 0.5 mL of product sample was then collected and transferred into a 1.5 mL screw cap tube.

*Analytical Tests*

*Percent Viability.* Cell counts were determined via Trypan Blue exclusion using a TC20™ Automated Cell Counter (Bio-Rad).

*Flow Cytometry.* Flow Cytometry was used to identify cell surface markers and characterize cell subpopulations. The following cell surface markers were assessed: CD3, CD56, and CD314 (BioLegend, San Diego, CA). Zombie Violet™ (BioLegend, San Diego, CA) was used as an additional viability dye. Samples were diluted to $2 \times 10^6$ cells/mL. 250 µL of each sample was added to an individual FACS tube (VWR, Radnor, PA) to be processed. Each tube was first incubated with 85 µL of Zombie Violet™ (1:1000 dilution of dye in 1× PBS) for 15 minutes incubation at room temperature. 5 µL of each antibody stock (CD3, CD56, and CD314) were then added to each stained sample and allowed to incubate for 30 minutes at 4°C. Samples were then washed 2×, and incubated in 250 µL of fixation buffer for 30 mins at 4°C. Samples were finally washed and resuspended in 100 µL cell staining buffer (Corning, Corning, NY).

*Appearance.* A description of the appearance of each FTB was made during each culture access. Turbidity, color, and cell clumping were qualitatively assessed. If the cell suspensions remained clear, the bag was described as "OK." If the color or turbidity of the sample changed significantly during the course of the stability study, a Gram stain would be performed on the sample to determine if bacterial contamination had occurred.

*Study Criteria*

Samples were processed immediately after taken from each transfer bag. Flow cytometry samples were processed (antibody staining and wash) immediately, and stored for up to 2 hours, if necessary, at 4°C prior to analysis in a flow cytometer. This hold study was considered successful if all data obtained at each tested timepoint met the relevant stability specification as described in Example 1.

**RESULTS**

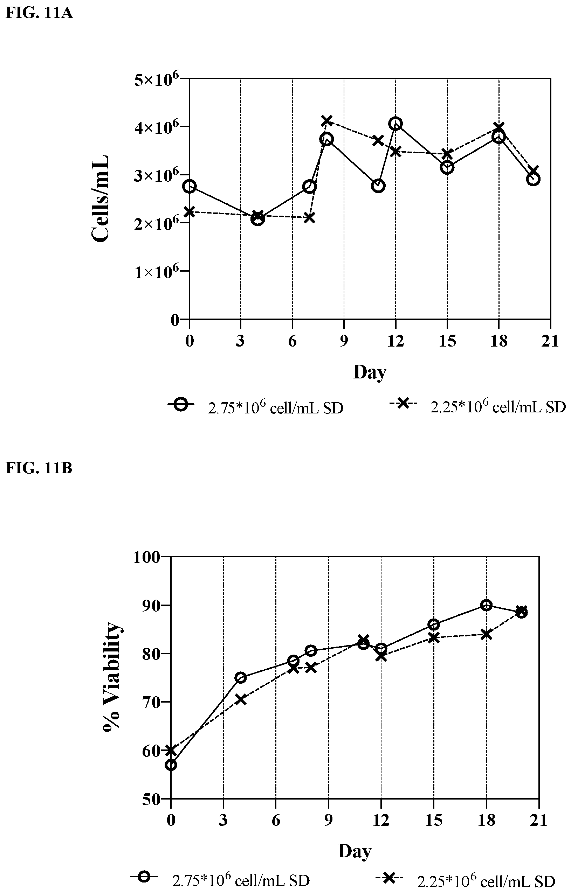

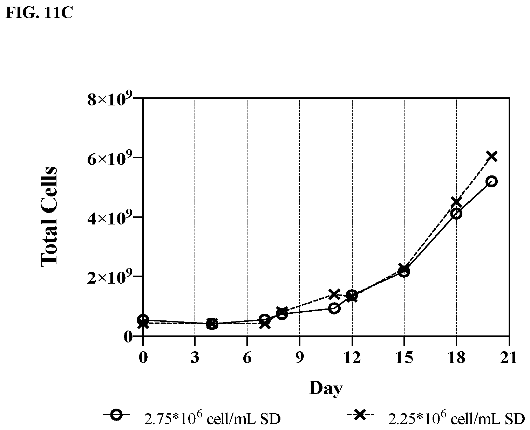

The concentration of cells and percent viability was quantified across different samples, including Bag #1 and Bag #2. The total cellular density (cells/mL) were quantified using the TC20™ automated cell counter (Bio-Rad) over a period of 20 days. Overall, the total amount of cells, cell concentration, and viability and was comparable across bags (FIGs. 11A-11C and Table 16), suggesting that initial seeding conditions do not influence the final compositions of the culture. Additionally, the percentage viability increases as a function of time and the total amount of cells are stable throughout the duration of the experiments.

**TABLE 16. Cell expansion overview**

| Day | Total vol. (mL) | # Cells | Viability | Total cells |
|---|---|---|---|---|
| **Bag#1** | | | | |
| 0 | 200 | 2.76E+06 | 57% | 5.52E+08 |
| 4 | 200 | 2.08E+06 | 75% | 4.16E+08 |
| 7 | 205 | 2.75E+06 | 79% | 5.64E+08 |
| 11 | 340 | 2.77E+06 | 82% | 9.42E+08 |
| 12 | 340 | 4.06E+06 | 81% | 1.38E+09 |
| 15 | 690 | 3.15E+06 | 86% | 2.17E+09 |
| 18 | 1087 | 3.79E+06 | 90% | 4.12E+09 |
| 20 | 1790 | 2.91E+06 | 89% | 5.21E+09 |
| **Bag#2** | | | | |
| 0 | 200 | 2.23E+06 | 60% | 4.46E+08 |
| 4 | 200 | 2.15E+06 | 71% | 4.30E+08 |
| 7 | 205 | 2.11E+06 | 77% | 4.33E+08 |
| 11 | 382 | 3.71E+06 | 83% | 1.42E+09 |
| 12 | 380 | 3.48E+06 | 80% | 1.32E+09 |
| 15 | 663 | 3.43E+06 | 83% | 2.27E+09 |
| 18 | 1135 | 3.98E+06 | 84% | 4.52E+09 |
| 20 | 1963 | 3.08E+06 | 89% | 6.05E+09 |

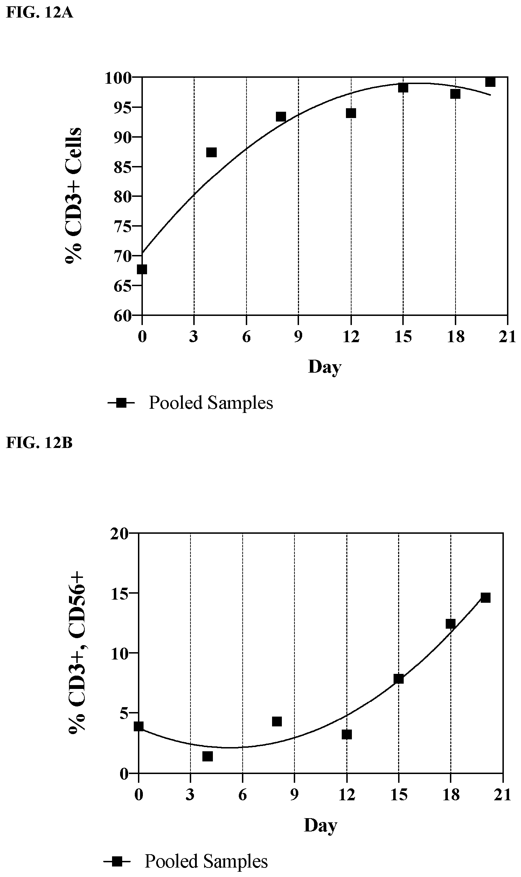

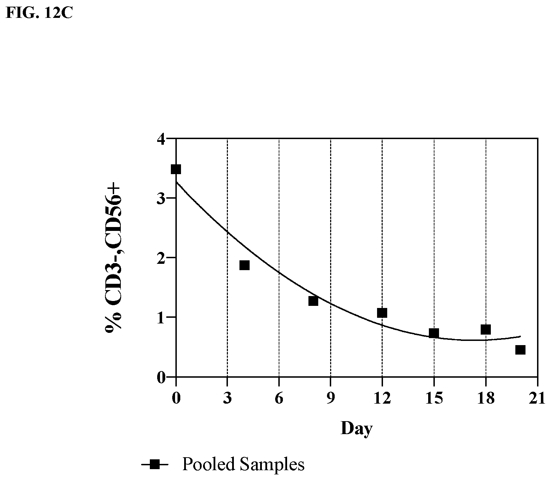

Flow cytometry was used to quantify the percentage of CD3+ cells within samples pooled from Bag #1 and Bag #2 (Table 17). The percentage of CD3+ cells increases as a function of time. By day 20, the last day of measurements, CD3+ cells represented 99.20% of all cells within the samples (FIG. 12A). The ratio of CD3+ to CD56+ cells increased as a function of time, suggesting that there is an overall expansion of cytotoxic cells within the culture (FIG. 12B). By day 20, the percentage of CD3+/CD56+ cells reached 14.6% (FIG. 12B). Interestingly, the percentage of CD3-/CD56+ cells decreased as a function of time (FIG. 12C), indication that the NK cell population was reducing within the culture sample. On day 21, the percentage of CD3-/CD56+ cells was found to be 0.45%, while the initial percentage on day 0 was 3.48% (FIG. 12C).

**TABLE 17. Quantification of cells by subpopulations**

| Day # | % CD3+/CD56+ | % CD3+/CD56- | % CD3-/CD56+ | % CD314+ [Viable Cells] | % CD314+ [CD3+/CD56+] | %CD314+ [CD3+/CD56-] | %CD314+ [CD3-/CD56+] |
|---|---|---|---|---|---|---|---|
| 0 | 3.87 | 64.00 | 3.48 | 32.10 | 78.50 | 33.90 | 94.40 |
| 4 | 1.40 | 88.20 | 1.87 | 16.90 | 81.80 | | |
| 8 | 4.27 | 88.20 | 1.27 | 30.60 | 76.60 | 30.60 | 87.23 |

(continued)

| Day # | % CD3+/CD56+ | % CD3+/CD56- | % CD3-/CD56+ | % CD314+ [Viable Cells] | % CD314+ [CD3+/CD56+] | %CD314+ [CD3+/CD56-] | %CD314+ [CD3-/CD56+] |
|---|---|---|---|---|---|---|---|
| 12 | 3.21 | 91.50 | 1.07 | 47.50 | 71.50 | 46.50 | 90.90 |
| 15 | 7.85 | 90.56 | 0.73 | 57.36 | 73.20 | 55.93 | 91.73 |
| 18 | 12.46 | 84.75 | 0.79 | 61.46 | 71.83 | 60.00 | 95.97 |
| 20 | 14.60 | 84.90 | 0.45 | 62.70 | 75.60 | 60.30 | 99.20 |

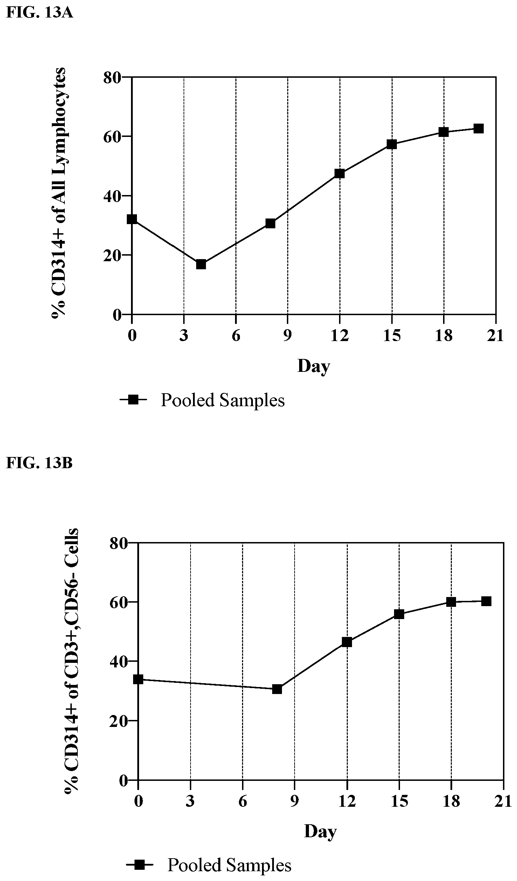

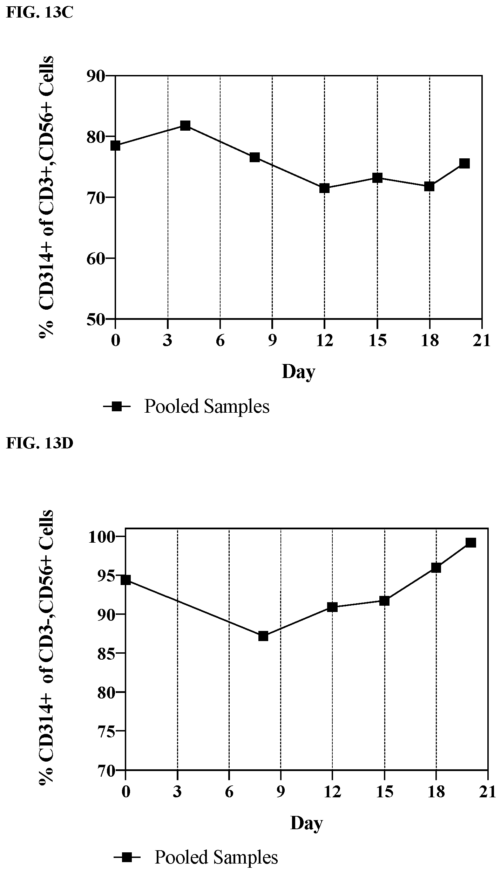

Additional measurements were taken to further quantify cell subpopulations within samples. The total percentage of CD314+ cells that are lymphocytes within the cell culture sample was determined. The percentage of CD314 cells increased as a function of time, reaching a final value of 62.70% on day 20 of experiments (FIG. 13A). Next, to determine the proportion of percentage of CD314+ cells that are T cells was determined by recording the ratio of CD314+ cells to CD3+/CD56- cells. As observed for all lymphocytes, percentage of CD314+ T cells increased over time, reaching a final recorded percentage of 60.30% by day 20 of experimentation (FIG. 13B). Interestingly, while T cell-specific cell markers indicated an expanding percentage of cells within the culture samples over time, CD314+ CIK cell populations remained stable over time. On day 20 of experimentation, the percentage of CD314+ CIK cells was 75.60, while the initial value on day 0 was around 78% (FIG. 13C). Next, the total percentage of CD314+ NK cells was analyzed by quantifying the percentage of CD314+ cells relative to CD3-/CD56+ cells. As observed for CIK cells, the NK cell population was largely unaffected by the total culture incubation time, with an initial measurement of ~99% on day 0 and a final measurement of 99.20% on day 21 (FIG. 13D).

*Hold*

Three bags were seeded at densities of $6 \times 10^6$ cells/mL and stored at three different temperature conditions: Bag #1 was stored at room temperature, Bag #2 was stored at 37°C, and Bag #3 was stored at 4°C. Cell concentration and cell viability measurements were taken for all three bags ranging from 0 - 115 hours post incubation. A summary of the measurements is provided in Table 18.

**TABLE 18. Effects of temperature on cell concentration and percentage viability upon hold**

| Hour | Bag #1 [RT] | | Bag #2 [37°C] | | Bag #3 [4°C] | |
|---|---|---|---|---|---|---|
| | [Cell] | % Viability | [Cell] | % Viability | [Cell] | % Viability |
| 0 | 5.25E+06 | 90.50% | 6.52E+06 | 91.50% | 5.06E+06 | 90.80% |
| 2 | 5.88E+06 | 88.30% | 5.50E+06 | 88.25% | 3.84E+06 | 89.25% |
| 4 | 6.75E+06 | 90.00% | 5.37E+06 | 91.80% | 3.46E+06 | 92.50% |
| 6 | 5.47E+06 | 90.25% | 5.79E+06 | 92.25% | 5.05E+06 | 92.50% |
| 8 | 5.97E+06 | 88.75% | 5.49E+06 | 90.75% | 6.81E+06 | 92.25% |
| 20 | 5.05E+06 | 91.25% | 4.57E+06 | 86.25% | 4.55E+06 | 92.00% |
| 24 | 4.86E+06 | 90.25% | 3.90E+06 | 74.75% | 6.62E+06 | 92.50% |
| 30 | 5.02E+06 | 92.00% | 3.45E+06 | 58.25% | 5.60E+06 | 93.25% |
| 32 | 3.56E+06 | 91.25% | 3.33E+06 | 60.50% | 5.21E+06 | 91.75% |
| 34 | 7.15E+06 | 92.75% | 2.56E+06 | 48.50% | 6.28E+06 | 93.00% |
| 42 | 5.64E+06 | 90.75% | 4.00E+06 | 62.75% | 5.36E+06 | 92.25% |
| 115 | 3.97E+06 | 72.50% | 3.59E+06 | 57.00% | 4.67E+06 | 86.25% |

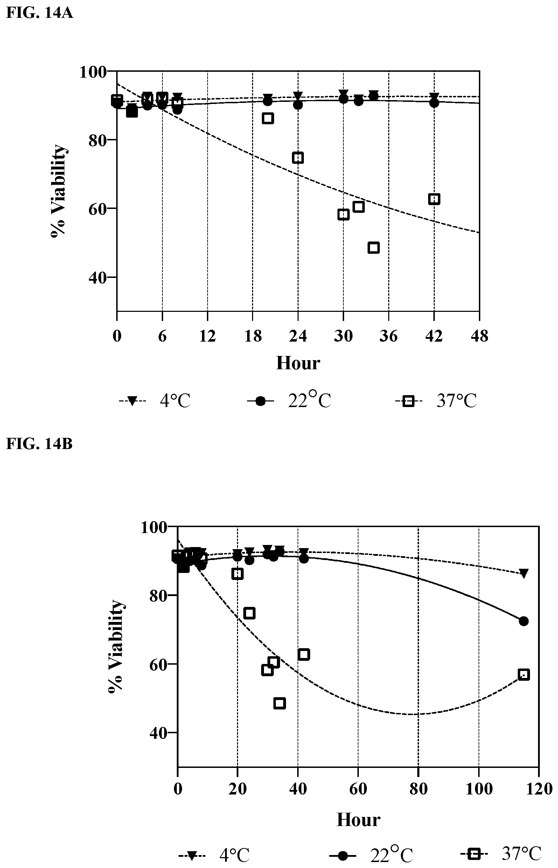

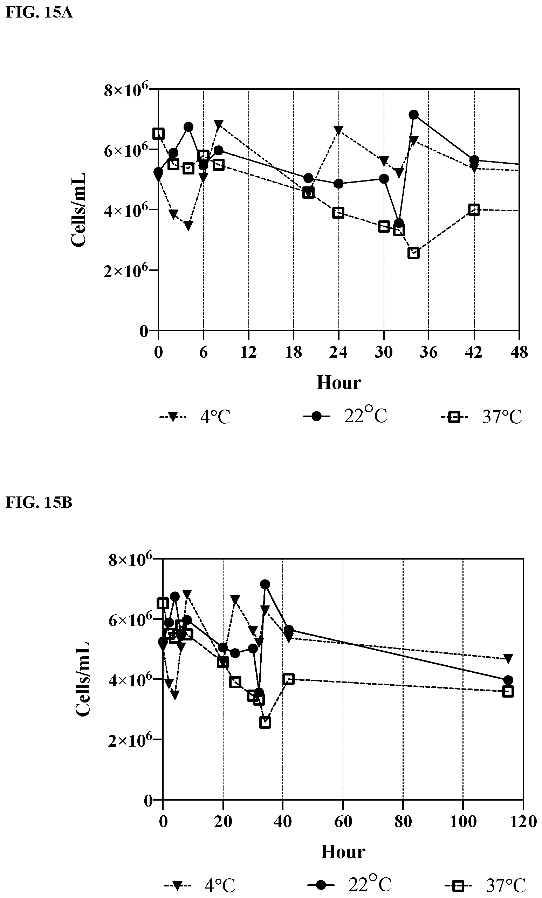

The time-series measurements of cell concentration across cultures in different storage temperatures is shown for a maximum of 48 hours and 115 hours of incubation time. Overall, cell cultures held at room temperature and 4°C showed an increased cell concentration relative to cultures held at 37°C (FIG. 14A). The differences in storage temperature becomes apparent at longer hold times. The rate of change in cell concentration of cultures held at 37°C appears greater than that of room temperature or 4°C stored cells (FIG. 14B), with the 37°C cells decreasing more quickly over time. Cell viability was measured across different temperature conditions as well. In general, cell viability greatly decreased over 48 hours in cell cultures incubated at 37°C (FIG. 15A). In contrast, cultures incubated at room temperature or 4°C showed high cell viability percentages that remained unaffected over a 48 hour time frame. By 115 hours, cell viability in room temperature and 4°C stored cultures decreased while 37°C incubated cultures appeared to stabilize, yet at a lower percentage cell viability than the lower temperature cultures (FIG. 15B).

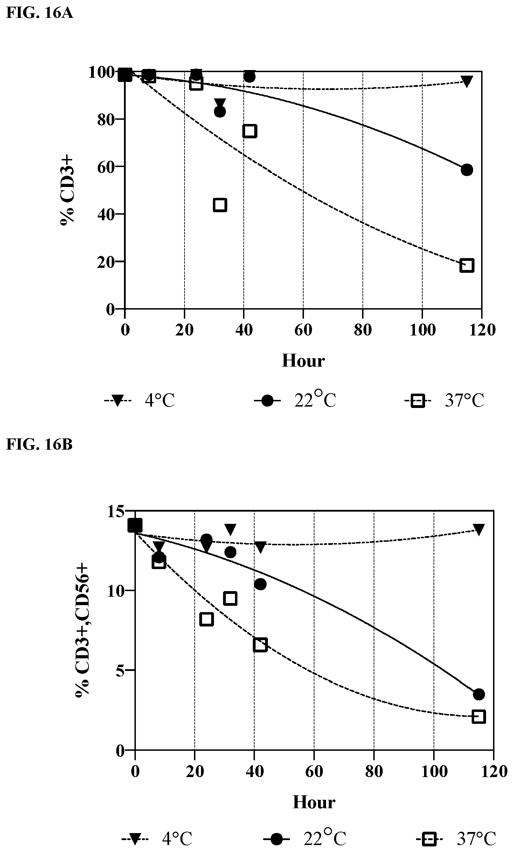

Next, cell subpopulations were compared across cell cultures stored at different temperatures. Flow cytometry was used to characterize cells with a focus on quantifying CD3+ expression and CD56+ expression (FIG. 16A, Table 19). As cultures were stored up 115 hours within 37°C conditions, the percentage of CD3+ greatly decreased relative to the initial measurement taken at time 0. Cultures stored in room temperature conditions had a somewhat reduced CD3+ percentage at 115 hours of incubation time. In contrast, 4°C storage conditions maintained the CD3+ percentages across all time points measured, suggesting that the culture remains more stable at 4°C than at the other temperatures measured (FIG. 16A). A striking difference between 4°C storage conditions and room temperature and 37°C was seen for the percentage of CD3+/CD56+ cells. Room temperature and 4°C storage resulted in similar percentages from 0-24 hours of incubation. A robust decrease in CD3+/CD56+ was seen in room temperature conditions beginning at hour 32, and the percentage continued to decrease throughout the remainder of the experiment for 115 hours. While the percentage of CD3+/CD56+ at room temperature fell from 10.4% to 3.5% between the hours of 42 and 115, cells stored at 4°C saw an increase of % CD3+/CD56+ from 12.7% to 13.8%. Percentages of CD3+/CD56+ cells stored at 37°C began to decrease earlier in time, at 24 hours post-incubation, and the percentages continued to decrease throughout the duration of the experiment as well. The final percentages of room temperature and 37°C storage conditions were 3.50% and 2.10%, respectively, which are both strikingly lower than 4°C storage conditions, which contained 13.8% (FIG. 16B, Table 20).

**TABLE 19. % of CD3+ cells**

| Hour | Room Temp | 37°C | 4°C |
| --- | --- | --- | --- |
| 0 | 98.7 | 98.7 | 98.7 |
| 8 | 98.6 | 98.0 | 98.6 |
| 24 | 98.7 | 95.0 | 98.6 |
| 32 | 83.1 | 43.9 | 86.2 |
| 42 | 97.9 | 74.9 | 98.1 |
| 115 | 58.6 | 18.4 | 95.7 |

**TABLE 20. % of CD3+/CD56+ cells**

| Hour | Room Temp | 37°C | 4°C |
| --- | --- | --- | --- |
| 0 | 14.1 | 14.1 | 14.1 |
| 8 | 12.1 | 11.8 | 12.7 |
| 24 | 13.2 | 8.2 | 12.7 |
| 32 | 12.4 | 9.5 | 13.8 |
| 42 | 10.4 | 6.6 | 12.7 |
| 115 | 3.5 | 2.1 | 13.8 |

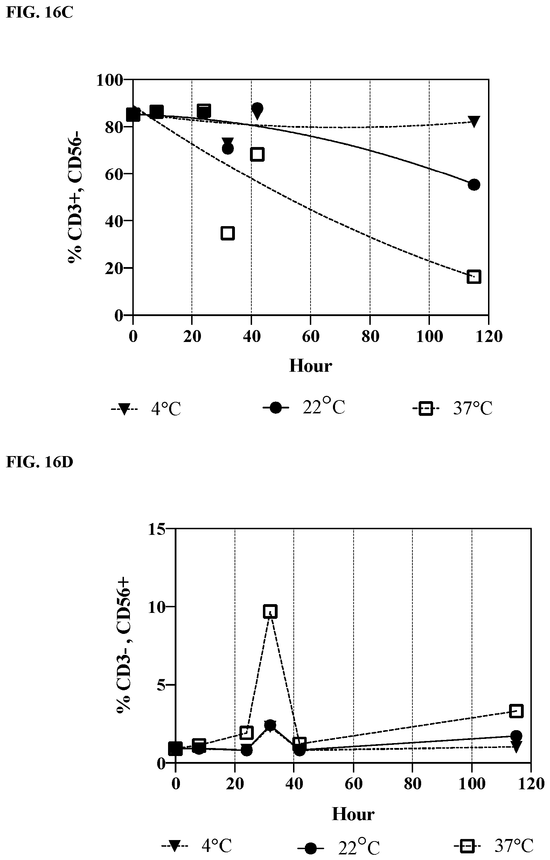

To further characterize the immune cells present within the cell culture, the percentage of CD3+/CD56- T cells were quantified across temperature incubation conditions. Cultures maintained at 4°C maintained a stable percentage throughout the duration of the measurements from 0 to 120 115 hours, with a final value of 82%. Similarly, the room temperature incubated cells maintained stable percentages, but began to decrease to a value of 55.30% by hour 115. Lastly, 37°C incubated cells showed a strong decrease in percentage of CD3+/CD56- T cells, reaching a value of 16.3% by hour 115 (FIG. 16C, Table 21). In contrast, the percentage of CD3-/CD56+ cells NK cells were comparable across incubation temperature conditions. Generally, the percentages remained low and had little effects across time (FIG. 16D, Table 22).

**TABLE 21. % CD3+/CD56-**

| Hour | Room Temp | 37°C | 4°C |
|------|-----------|------|-----|
| 0 | 85.1 | 85.1 | 85.1 |
| 8 | 86.5 | 86.3 | 85.8 |
| 24 | 85.6 | 86.8 | 85.9 |
| 32 | 70.8 | 34.8 | 72.8 |
| 42 | 87.8 | 68.3 | 85.3 |
| 115 | 55.3 | 16.3 | 82.0 |

**TABLE 22. % CD3-/CD56+**

| Hour | Room Temp | 37°C | 4°C |
|------|-----------|------|-----|
| 0 | 0.9 | 0.9 | 0.9 |
| 8 | 0.9 | 1.1 | 0.9 |
| 24 | 0.8 | 2.0 | 0.8 |
| 32 | 2.8 | 10.4 | 2.3 |
| 42 | 0.8 | 1.2 | 0.8 |
| 115 | 2.0 | 3.3 | 1.0 |

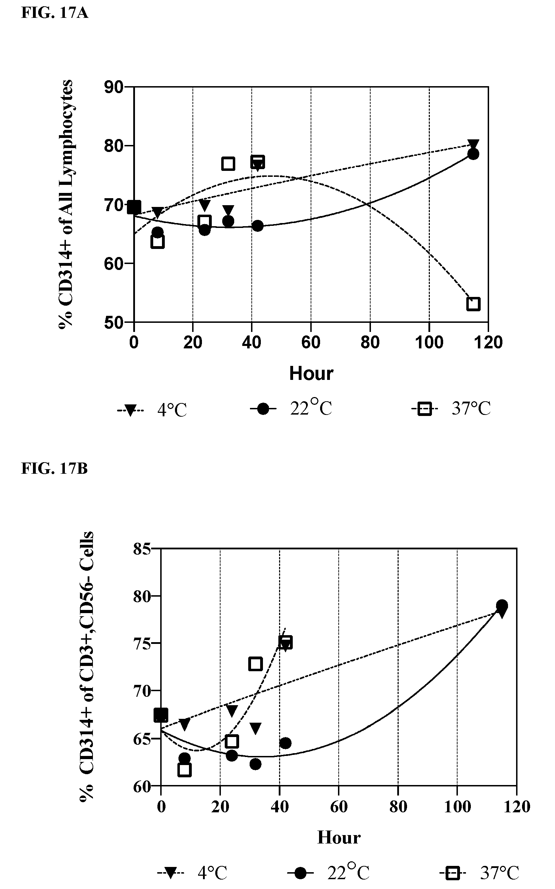

Populations of all lymphocytes were quantified across the temperature conditions. The percentage of CD314+ cells between room temperature and 4°C incubation temperatures were comparable across time, and had a weak increase in CD314 populations by hour 115 (FIG. 17A, Table 23). In contrast, cells incubated in 37°C had a sharp decrease by hour 115 (FIG. 17A). When percentages of CD314+ cells relative to CD3+/CD56- T cells were quantified, 4°C or room temperature incubation appeared to strongly enhance the percentages by the end of the experiment. Cell percentages of cultures incubated at 37°C increased as well, but at a higher rate (FIG. 17B, Table 24).

**TABLE 23. %CD314+ [VCs] All lymphocytes**

| Hour | Room Temp | 37°C | 4°C |
|------|-----------|------|-----|
| 0 | 69.6 | 69.6 | 69.6 |
| 8 | 65.3 | 63.7 | 68.6 |
| 24 | 65.7 | 67.1 | 69.8 |
| 32 | 67.2 | 76.9 | 68.6 |
| 42 | 66.4 | 77.2 | 76.6 |
| 115 | 78.6 | 53.1 | 80.0 |

**TABLE 24. %CD314+ [CD3+/CD56-]**

| Hour | Room Temp | 37°C | 4°C |
|------|-----------|------|-----|
| 0 | 67.4 | 67.4 | 67.4 |
| 8 | 62.9 | 61.7 | 66.4 |
| 24 | 63.2 | 64.7 | 67.8 |
| 32 | 62.3 | 72.8 | 66.0 |
| 42 | 64.5 | 75.1 | 74.7 |
| 115 | 79.0 | | 78.2 |

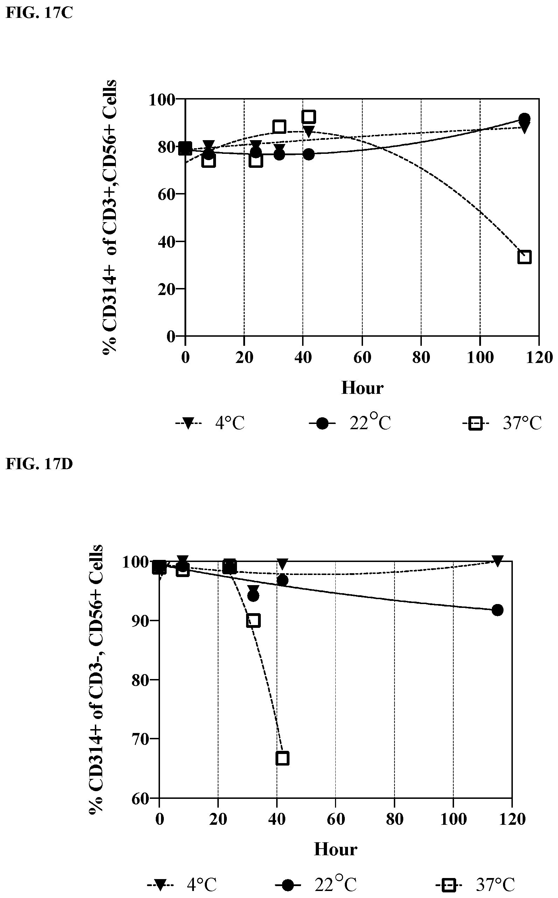

Interestingly, when the percentage of CIK cells and NK expressing CD314+ and was quantified, it was observed that lower temperatures maintained a stable amount of the populations across all time points tested. In contrast, cultures incubated at 37°C had a large reduction in percentages of CIK and NK cells expressing CD314 by the final time points tested (FIG. 17C and 17D, Tables 25 and 26). These results point to the potential to enhance culture stability by storage at 4°C or room temperature when cells are maintained for extended periods of time.

**TABLE 25. %CD314+ [CD3+/CD56+] CIK cells**

| Hour | Room Temp | 37°C | 4°C |
|------|-----------|------|------|
| 0 | 79.1 | 79.1 | 79.1 |
| 8 | 76.8 | 74.0 | 80.0 |
| 24 | 77.5 | 74.0 | 80.1 |
| 32 | 76.6 | 88.4 | 78.5 |
| 42 | 76.7 | 92.5 | 86.1 |
| 115 | 91.7 | | 87.8 |

**TABLE 26. %CD314+ [CD3+/CD56+] NK cells**

| Hour | Room Temp | 37°C | 4°C |
|------|-----------|------|------|
| 0 | 99.0 | 99.0 | 99.0 |
| 8 | 99.2 | 98.6 | 100.0 |
| 24 | 98.9 | 99.1 | 99.5 |
| 32 | 94.2 | 90.0 | 95.0 |
| 42 | 96.8 | 66.7 | 99.5 |
| 115 | 91.8 | | 100.0 |

## Example 5

The purpose of the study was to investigate the stability and potency of CIK-DS and CRX100-DP by phenotypic assessment after prolonged storage at 4°C and ambient room temperature (22°C) immediately after COBE washing. CIK cells were harvested on day 26 of culture and washed using a COBE 2991 cell processor. Manufacturing specifications dictate approximately $3 \times 10^9$ cells in 350mL of Plasma-Lyte$^{©}$ (Baxter International, Deerfield, IL) supplemented with 0.5% Human AB Serum, a concentration of $8.57 \times 10^6$ cells/mL. Consequently, CIK cells were suspended to a similar concentration ($1 \times 10^7$ cells/mL).

Resuspended CIK cells were divided equally into 2 seed bags, each containing approximately 160 mL of cell product ($3.5 \times 10^9$ cells per bag). Next, double-deleted vaccinia virus (vvDD-CDSR) drug substance was added to one bag at a multiplicity of infection (MOI) of 0.1. The two seed bags were gently palpitated for 60 seconds and kept at 37°C, 5.0% $CO_2$ for 2 hours. The two seed bags (CIK cells alone and CRX100) were each evenly split into two news bags, yielding four bags total. The other CIK cell and CRX100 bags (each containing 80 mL cell product) were used for subsequent hold studies or potency assays. 30 mL of the CRX100 and CIK cell bags were obtained and transferred into a 300 mL transfer pack to be stored in a 4°C refrigerator in the dark, while another 30 mL aliquot of the CRX100 and CIK cells were stored in a 300 mL transfer bag at ambient temperature (22°C) in the dark.

Viability and cell surface marker expression by flow cytometry were assessed at various time points (0, 4, 12, 22, 42, 48, 69, 94, and 120 hours). Plaque assays on Vero E6 cell line were performed with samples from each bag every 24 hours to assess cell-associated and cell-free viral titer. Plaque assays revealed an increase in cell-associated and cell-free virus after 48 hours in CRX100 bag stored at 4.0°C, while no increase in cell-associated or cell-free virus was observed at ambient temperature.

Storage at 4.0°C resulted in prolonged viability above 80% for at least 120 hours across both CRX100 and CIK cell bags. Meanwhile, bags stored at 22°C remained above 70% viable for 120 hours. Introduction of vvDD-CDSR at MOI 0.1 did not noticeably affect viability, cell concentration, or percentage of cells expressing NKG2D receptor. However, a higher percentage of CD3-/CD56+ NK cells were observed in CIK bags infected with vvDD-CDSR as compared to CIK cell controls by hour 94. Storage at 4°C resulted in an increase in the percentage of CD3+ cells positive for NKG2D (CD314) expression compared to cells stored at 22°C. Storage at 4°C also yielded a higher percentage of CIK cells by hour 120, while the percentage of cells expressing CD314 remained relatively similar across all bags. Importantly, a potency assay post 120-hour revealed CRX100 stored at 4.0°C resulted in significantly greater killing of target OVCAR-3 tumor cells compared to CRX100 held at ambient temperature. Indeed, CRX100 held at 4.0°C for 120 hours produced similar cytotoxic effects compared to CRX100 used immediately after washing.

Data from previous hold studies conducted on CIK cells indicated that prolonged storage at $22 \pm 3°C$ maintains cell viability for at least 96 hours and surface marker phenotype for 72 hours. Moreover, a previous study demonstrated that an immediate 2-hour hold at 37°C after harvest resulted in no apparent effect on total cell count, viability, or cell marker phenotype. A recent hold study demonstrated that CRX100 stored at 30.0°C, 5% $CO_2$ remained above 70% for 48 hours. However, no previous study has yet indicated the viability, cell concentration, viral shedding profile, surface marker expression profile, or potency of CRX100 stored at 4.0°C or ambient temperature. Characterization of these effects due to prolonged storage at 4.0°C or ambient temperature are critical to understanding the storage conditions for an optimal drug product.

In this study, CRX100 and CIK cells alone were harvested, resuspended in Plasma-Lyte© (supplemented with 0.5% human AB serum), and stored at either 4°C or ambient temperature (22°C). Samples were collected at various time points between 0 and 120 hours to assess viability, cell concentration, cell surface phenotype, and viral shedding. After the conclusion of the hold study, the remaining samples were used for a potency assay on the ovarian carcinoma cell line, OVCAR-3 (ATCC). OVCAR-3 carcinoma cell line was chosen since the cells were positive for the expression of NKG2D ligand (MicA/B) and also susceptible to vaccinia virus infection.

CIK cells were harvested on day 26 of culture, washed using a COBE 2991 processor, and resuspended to approximately $1 \times 10^7$ cells/mL. Resuspended CIK cells were split into 2 seed bags, each containing approximately 160 mL of cell product. Double-deleted vaccinia virus (vvDD-CDSR) was added at an MOI of 0.1 to one of the bags. Both seed bags were then placed into a 37°C, 5.0% $CO_2$ incubator for 2 hours. After incubation, each seed bag was again split, yielding 4 total bags containing approximately 80 ml of cell product. The other CIK cell and CRX100 bags (each containing 80 mL cell product) were used for subsequent hold studies or potency assays. 30 mL of the CRX100 and CIK cell bags were obtained and transferred into a 300 mL transfer pack to be stored in a 4°C refrigerator in the dark, while another 30 mL aliquot of the CRX100 and CIK cells were stored in a 300 mL transfer bag at ambient temperature (22°C) in the dark. The remaining cells from CRX100 and CIK bags were used immediately for performing a potency assay on OVCAR-3 tumor cell line.

Using a Chemolock system, 1.0 mL samples were obtained from each bag at the time points as shown in Table 27. Samples were then assessed for appearance (A), cell concentration, and viability (V) using the Bio-Rad TC-20™ automated cell counter. Cell surface markers (CD3, CD56, and CD314) were characterized via flow cytometry (F). At the appropriate time points below, a 1.5 mL aliquot was used to compare viral shedding at different temperatures. Samples were spun down at 1000 g for 10 minutes from each bag and supernatant (cell-free virus) was separated from the cell pellet (cell-associated virus). Supernatant was serially diluted in media and 1 mL of each dilution was added to confluent Vero E6 cells in a six well plate. Cell pellets were resuspended in 1 mL of media and freeze-thawed for 60 secs each in dry ice/ethanol and 37°C water bath five times to release membrane-bound and intracellular virus. Cell lysate was vortexed gently at the end of each freeze-thaw and dilutions were similarly plaqued on Vero E6 cells. Plaques were counted after 3-4 days.

**Table 27**

| Time [Hours from Initiation] | Assays |
| --- | --- |
| 0 | V+F+A+P |
| 4 | V+A |
| 12 | V+A |
| 22 | V+F+A+P |
| 42 | V+A |
| 48 | V+F+A+P |
| 69 | V+F+A+P |
| 94 | V+F+A+P |
| 120 | V+F+A |
| V: viability; F: flow cytometry; A: appearance; P: plaque assay | |

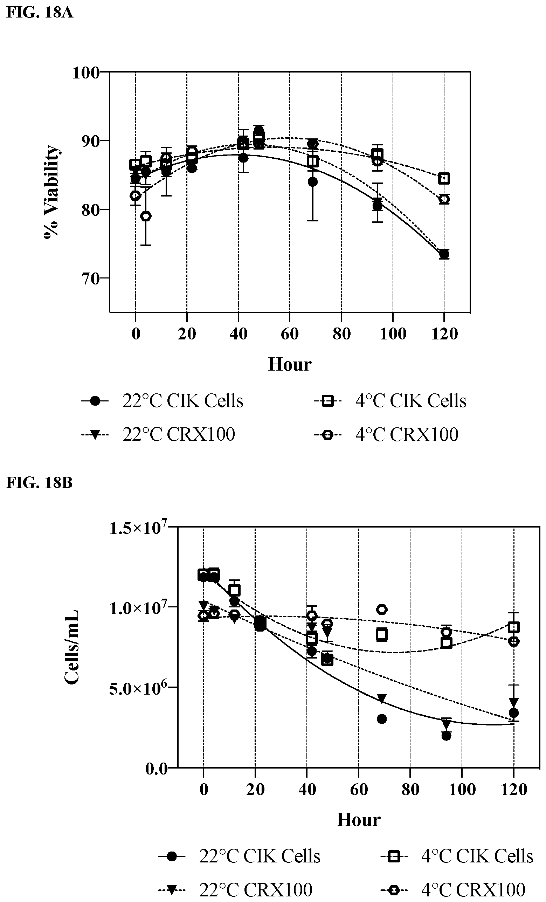

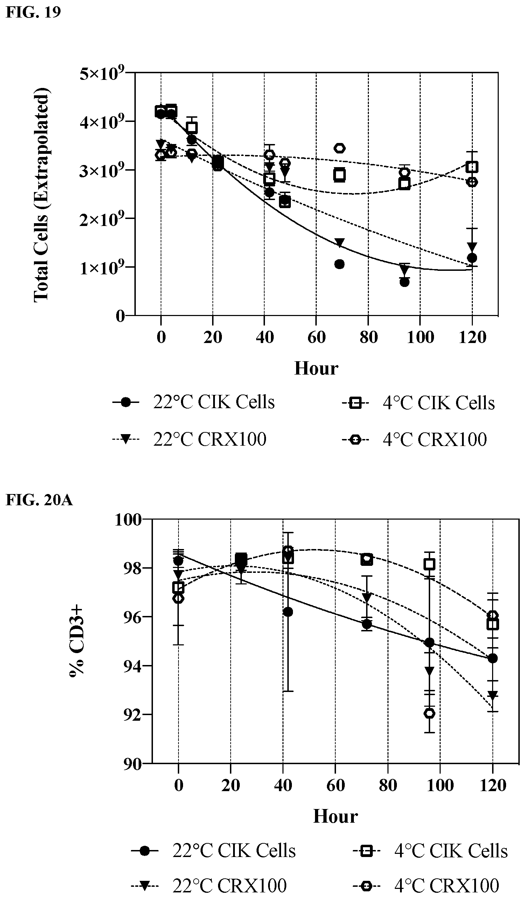

Table 28, FIGs. 18A and 18B, and FIG. 19 illustrate the percent viability (Fig. 18A), cell concentration (Fig. 18B), and total cell count (Fig. 19) for CIK and CRX100 drug substance at 4°C and 22°C. CRX100 and CIK cells stored at 4°C remained above 80% viability for 120 hours, while cell product stored at 22°C dipped below 80% viability at approximately hour 94, but remained above 70% for the 120 hour duration of the study. The extrapolated total cell population of CRX100 and CIK cells stored at 4°C remained above manufacturing specification of $2 \times 10^9$ cells for 120 hours, while cells stored at ambient temperature dipped below $2 \times 10^9$ cells between 48 and 69 hours. No significant difference in viability or cell count was observed between CRX100 and CIK cells stored at the same temperature.

**Table 28.**

|  | Bag #1 [4°C CIKs] | | Bag #2 [22°C CIKs] | | Bag #3 [4°C CRX100] | | Bag #4 [22°C CRX100] | |
|---|---|---|---|---|---|---|---|---|
| Hour | [Cell] cells/mL | % Viability | [Cell] cells/ml | % Viability | [Cell] Cells/ml | % Viability | [Cell] cells/ml | % Viability |
| 0 | 1.21E7 | 87 | 1.20E7 | 84 | 9.22E6 | 83 | 1.02E7 | 85 |
| 4 | 1.23E7 | 88 | 1.18E7 | 85 | 9.38E6 | 82 | 9.67E6 | 84 |
| 12 | 1.15E7 | 87 | 1.06E7 | 85 | 9.41E6 | 87 | 9.34E6 | 88 |
| 22 | 9.09E6 | 87 | 9.25E6 | 86 | 8.64E6 | 88 | 8.80E6 | 86 |
| 42 | 7.75E6 | 88 | 6.95E6 | 89 | 9.03E6 | 89 | 8.81E6 | 90 |
| 48 | 6.60E6 | 91 | 6.50E6 | 92 | 8.93E6 | 89 | 8.01E6 | 89 |
| 69 | 8.57E6 | 88 | 3.12E6 | 88 | 9.68E6 | 89 | 4.14E6 | 89 |
| 94 | 7.52E6 | 87 | 2.01E6 | 80 | 8.11E6 | 86 | 2.95E6 | 79 |
| 120 | 9.37E6 | 85 | 3.39E6 | 73 | 7.82E6 | 82 | 4.81E6 | 73 |

*Effect of Storage Temperature on Subpopulations*

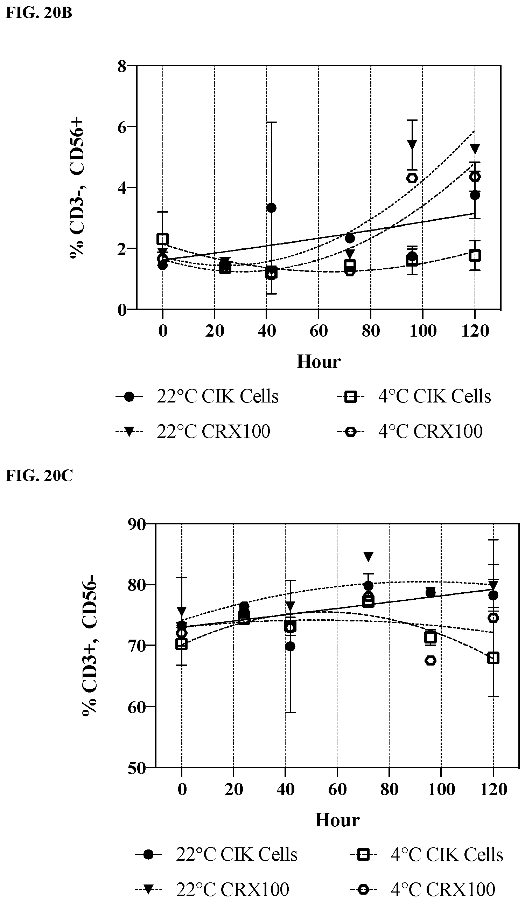

Flow cytometry surface marker characterization of CRX100 and CIK cells revealed that % CD3+ remains above 90% for all four experimental groups for the duration of the 120 hour study (FIG. 20A). At hour 120, however, CIK cells and CRX100 stored at 4°C remained above 95% CD3+ (although a slight dip under 95% CD3+ was observed at hour 96 for CRX100 stored at 4°C). Moreover, the % CD3-/CD56+ NK cell population increased across CRX100 bags at both temperatures and CIK cells stored at ambient temperature (FIG. 20B).

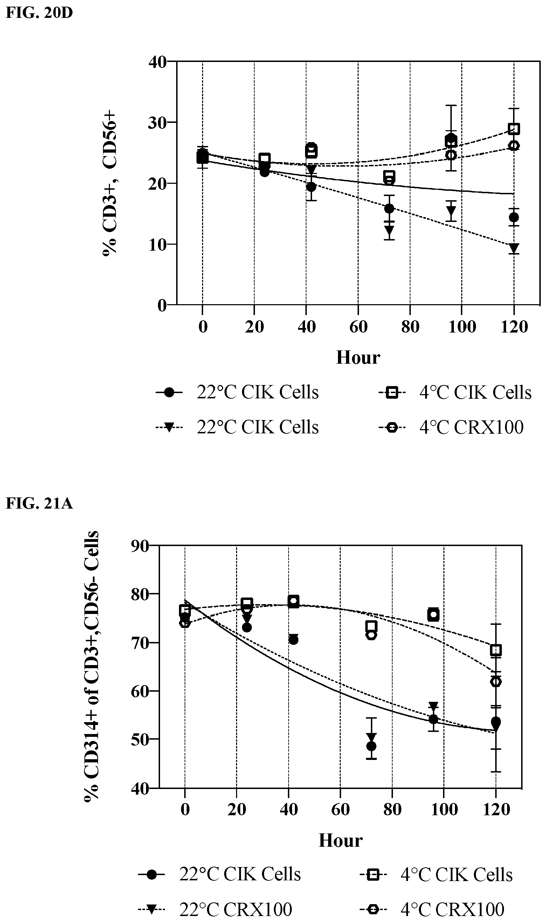

Flow cytometry surface marker expression characterization of CRX100 and CIK cells revealed dramatic noticeable difference in % CD3+/CD56+ CIK cells for bags stored at 4°C compared to bags stored at ambient temperature (FIG. 20D). By hour 94, CRX100 stored at 4°C exhibited a marked increase in % CD3+/CD56+ CIK cells as compared to CRX100 stored at 22°C. Indeed, at hour 120, CRX100 and CIK cells alone stored at 4°C were 26.2% and 28.5% CIK cells, respectively, while CRX100 and CIK cells stored at 22°C were 9.2% and 14.4% CIK cells, respectively. Finally, in FIG. 20C, the % CD3+/CD56- T cells appeared to decrease slightly for cells stored at 4°C, but slightly increased for cells stored at ambient temperature.

*Effect of Temperature and vvDD on % CD314+ Expression*

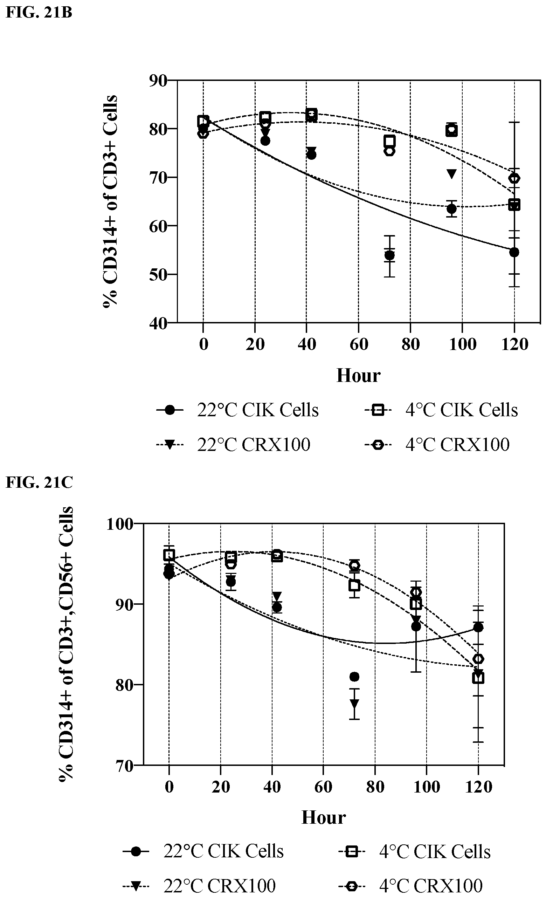

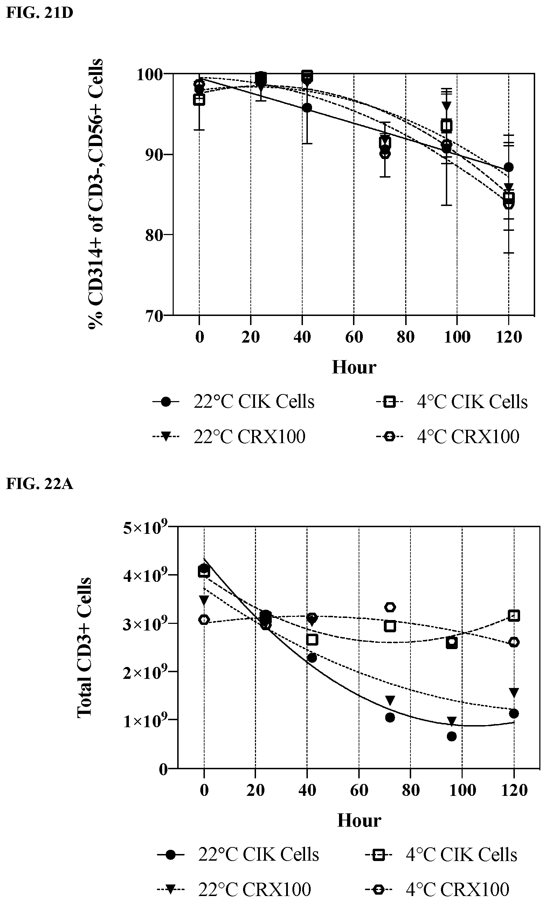

CD314 (NKG2D receptor) was assessed to characterize the potential role of storage temperature and vvDD-CDSR introduction on potency of the cell product. At hour 42, a pronounced difference in CD314 expression of CD3+ and CD3+/CD56- may be noticed between cell product stored at 4°C compared to cell product stored at ambient temperature (FIG. 20A and 21B). At hour 94, 76.4% of CD3+ cells stored at 4°C in CRX100 were CD314+, compared to 56.6% of CD3+/CD56- cells in CRX100 stored at 22°C. Infection with vvDD did not appear to have a significant effect on CD314 expression of CD3+/CD56- cells.

At hour 72, 95.3% of CD3+/CD56+ CIK cells in CRX100 at 4°C were CD314+, compared to 76.2% of CD3+/CD56+ CIK cells in CRX100 at ambient temperature (FIG. 20C). While this difference in CD314 expression is most striking at hour 96, it is not apparent by hour 120. vvDD infection did not appear to affect CD314 expression of CD3+/CD56+ CIK cells. Neither temperature nor vvDD introduction at MOI 0.1 appeared to affect CD314 expression of NK cells, as all four bags exhibited near identical CD314 expression profiles on NK cells (FIG. 20D).

*Total Cell Count of Flow Cytometry Cell Subpopulations*

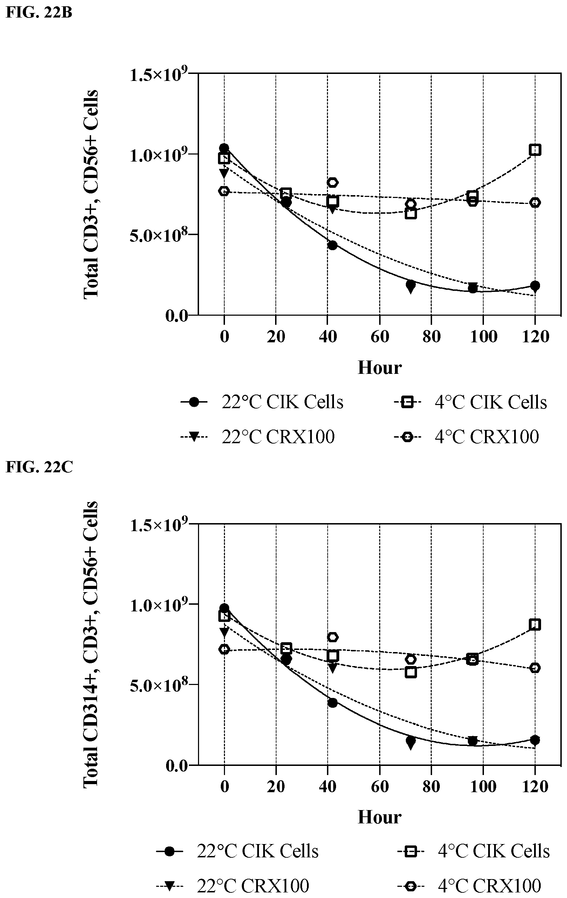

The total cell count (FIG. 19) was multiplied by cell surface marker subpopulations as characterized by flow cytometry (FIGs. 20A-20D, 21A-21D) to provide the total CD3+ cell count, total CD3+/CD56+ cell count, and total CD314+/CD3+/CD56+ cell count (FIGs. 22A-22C). The total population of CD3+ cells stored at 4°C remained above $2.5\times10^9$ cells for the entire 120 hour hold study, while cells stored at 22°C fell below $1.5\times10^9$ total cells. Importantly, CRX100 and CIK cells alone stored at 4°C had $7.5\times10^8$ and $1\times10^9$ CD3+/CD56+ cells, respectively, of which about 83% were CD314+.

*Viral Shedding Profile*

To characterize the eclipse period of CRX100 and the viral shedding profile over time, samples were collected from CRX100 and CIK bags at 4°C and 22°C temperature at T=0 (at the end of two hours viral infection at 37°C, 5.0% $CO_2$) and every 24 hours throughout the duration of the hold study. To quantify the viral titer, 1.5 mL of the samples was spun down from each bag at $1000\times$ g for 10 mins and supernatant (cell-free) was separated from the cell pellet (cell-associated). Next, the supernatant was serially 10-fold diluted in Eagle's minimal essential media and 1 mL of each dilution was used to infect confluent Vero E6 monolayers in a six well plate. Vero E6 cells were incubated with virus inoculum for 2 hours at 37°C, 5.0% $CO_2$ incubator. At the end of incubation, virus inoculum was removed and 3 mL of fresh media was added. The plates were incubated at 37°C, 5.0% $CO_2$ for 3-4 days to allow plaque formation.

The cell pellet was resuspended in 1 mL of Eagle's minimal essential media and freeze-thawed for 60 secs each in dry ice/ethanol and 37°C water bath five times to release membrane-associated and intracellular virus. Cell lysate was vortexed gently at the end of each freeze-thaw and dilutions were similarly plaqued on Vero E6 cells. Plaques were visualized by staining with 0.1% crystal violet after 3-4 days.

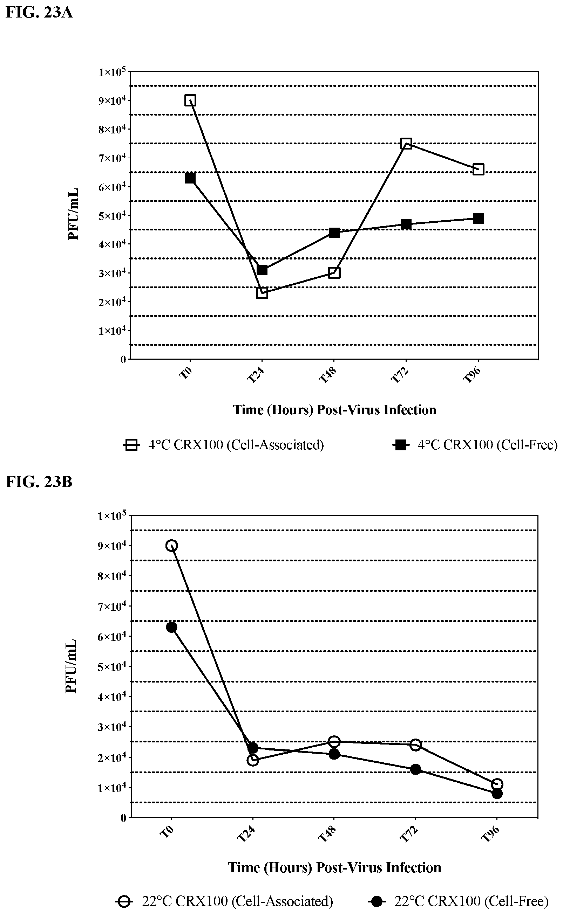

Cell-free (supernatant) and cell-associated (cell pellet) vaccinia virus titer were quantified via plaque assay and recorded. Immediately after CRX100 formulation (T0), a cell-free viral titer of $6.3\times10^4$ PFU/mL was observed while cell-associated virus was found to be higher ($9\times10^4$ PFU/mL). This data suggests approximately 58.8% of virus is adsorbed by CIK cells during the 2 hour infection at 37°C. At 24 hours post-viral infection, no significant differences in cell-free and cell-associated viral titer for CRX100 at 4°C and 22°C were observed (FIGs 23A and 23B).

An increase in both cell-associated and cell-free vaccinia virus was observed after 48 hours for CRX100 stored at 4°C (FIG. 23A). By 72 hours post-virus infection, cell-associated virus titer in CRX100 at 4°C had increased to $7.5\times10^4$ PFU/mL while free virus titer had increased to $4.7\times10^4$ PFU/mL. Surprisingly, no such increase in either cell-associated or free virus titer was observed in CRX100 at room temperature, as the cell-associated and free virus titer remained around $2.0\times10^4$ PFU/mL (FIG. 23B). No plaques were observed in samples from CIK bags at 4°C and 22°C temperature at any time point assayed. These results demonstrate that not only is CRX100 stable for extended periods of time at 4°C, but appears to be replicating in the cells at 4°C and infecting other CIK cells as noted by the increase in both cell-associated titer and cell-free titer thereby extending the eclipse period of the vvDD in CRX100 cells.

*Tumor Killing Profile Post-120 Hour Hold*

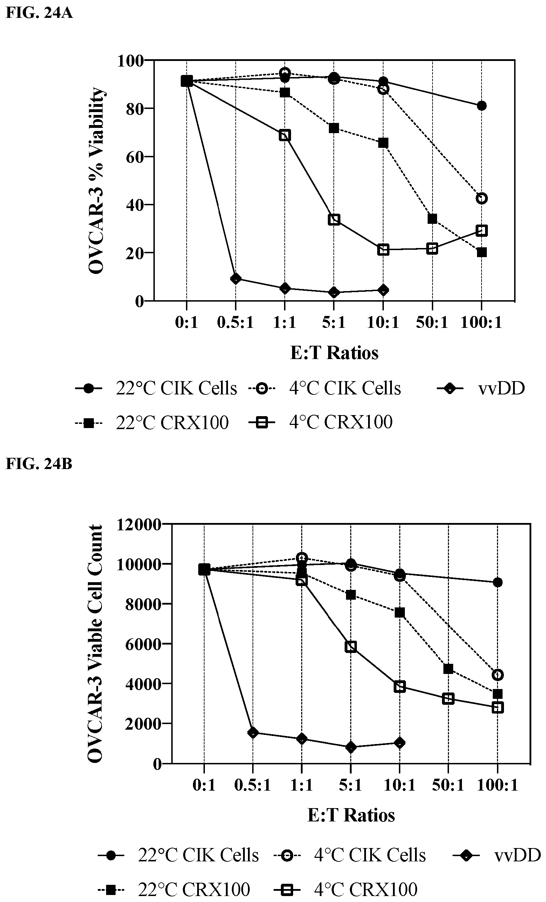

Immediately following the 120 hour hold, CRX100 and CIK cells from both storage temperatures were taken, spun down, and resuspended to various concentrations in 100$\mu$L of AIM-V 5.0% human AB serum media supplemented with IL-2 (300 IU/mL). CRX100 and CIK cells were co-incubated with OVCAR-3 target cells ($2.0\times10^5$ target cells) at various effector-to-target ratios (FIGs. 24A and 24B). vvDD virus was also added to confluent OVCAR-3 cells at different multiplicity of infections (MOIs). Effector and target cells were allowed to co-incubate at 37°C, 5.0% $CO_2$ incubator for 96 hours. After 96 hours, supernatant was removed, OVCAR-3 tumor cells were washed 1X with PBS and trypsinized. The cells were stained with Zombie-NIR™ viability dye. Percent viability of tumor cells and total viable cell count were assessed via flow cytometer.

Assessment of OVCAR-3 cells viability across the experimental groups revealed that vvDD efficiently lysed OVCAR-3 cells starting at MOI of 0.5:1. More importantly, it was observed that CRX100 cells stored at 4°C resulted in more tumor cell death than CRX100 stored at 22°C for E:T ratios between 1: 1 and 50: 1. Furthermore, CRX100 stored at either temperature resulted in more tumor-killing than CIK cells alone. While CRX100 stored at 4°C appeared to result in less tumor cell death than vvDD, the CRX100 was formulated at a low MOI of 0.1. Finally, CIK cells stored at 4°C also appeared to kill a higher number of OVCAR-3 tumor cells at a E:T ratio of 100: 1 compared to CIK cells stored at ambient temperature.

*Tumor Killing Profile: Comparison to Fresh CRX100*

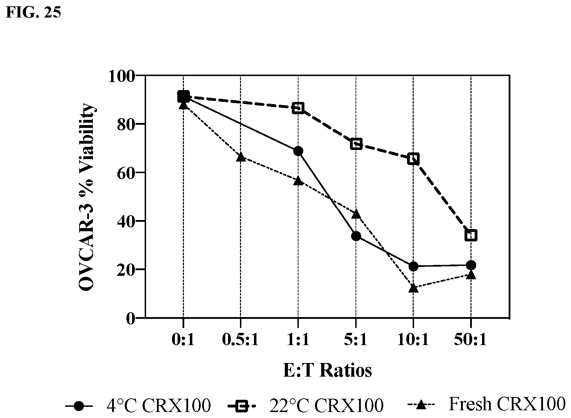

CRX100 formulated and used fresh exhibited a similar tumor-killing profile to CRX100 formulated and stored at 4°C for 120 hours (FIG. 25). At an MOI of 0.1 and an E:T ratio of 10:1, almost all OVCAR-3 cells were completely killed in both fresh and 4°C held CRX100. CRX100 held at ambient temperature, meanwhile, was markedly worse at tumor-killing between E:T ratios of 0.5:1 and 50:1. The increase in OVCAR-3 tumor viability for fresh CRX100 at an E:T ratio of 100:1 is likely an artifact of flow cytometry, as OVCAR-3 cells die they become detached from the plate and are removed from the flow cytometry sample staining. Consequently, remaining cells are more likely to be viable. This artifact is less apparent when a larger target cell number is used, as was the case for the post-120 hour study potency assay.

**Conclusions**

*CIK Cells Alone & CRX100 Remain Above 70% for At Least 120 hours at 4°C*

As demonstrated in FIGs. 17A, 18B and 19, cell product stored at 4°C remains above 80% viability for at least 120 hours. Moreover, cell product stored at ambient temperature remains above the manufacturing specification of 70% viability for at least 120 hours. This has direct implications for shipping of the final drug product, as some shipping containers have a listed temperature range of 15-30°C. These data indicate that while storage at lower temperatures (room temperature or 4°C) result in improved viability, holds at 22°C for 120 hours would not reduce viability below manufacturing specifications. Furthermore, this hold study has indicated that cell product stored at lower temperatures are more stable than cell product stored at either 30°C or 37°C. Finally, extrapolated total cell count data indicates that cell product stored at 4°C remained above the manufacturing specification of $2\times10^9$ effector cells for the duration of the 120 hour hold study. As cell count is directly correlated to the potency of the final product (FIGs. 24A, 24B, 25), these data suggest that storage at 4°C could yield a more potent product.

*Effect of vvDD Introduction on Viability, Cell Count, and Surface Marker Expression*

Introduction of vvDD-CDSR at an MOI of 0.1 did not have a significant effect on either viability, cell concentration, or total cell count (FIGs. 18A, 18B and 19). This is important to note due to the implications for shipping. Addition of vvDD-CDSR at this particular dosage prior to shipping would not decrease viability of the cell product if held at 4°C for 120 hours

Furthermore, introduction of vvDD to CIK cells at an MOI of 0.1 did not appear to have a major effect on CD314 expression or cell subpopulations, though a slight increase in NK cell population was observed across CRX100 bags. This suggests that vvDD introduction does not appear to affect stability or viability of the drug product over time.

*Effect of Temperature on Viability, Cell Count, and Surface Marker Expression*

Cell product stored at 4°C ultimately had a higher percentage of % CD3+/CD56+ CIK cells by hour 120 compared to cells stored at ambient temperature. Inversely, a slight decrease in % CD3+/CD56- T Cells was observed in cells stored at 4°C, possibly indicating that cells are transitioning from CD3+/CD56- to CD3+/CD56+ over time in bags stored at lower temperatures. This phenomenon could also have direct implications for potency of the final drug product, as CD3+/CD56+ cells are currently hypothesized to contribute the most tumor-killing ability of all cell subpopulations.

Moreover, it was found that storage at 4°C resulted in higher NKG2D (CD314) expression over time for CD3+/CD56- T cells and CD3+/CD56+ CIK cells up to 69 hours. As NKG2D is believed to be the primary binding site between CIK cells and tumor cells, it is possible that increased NKG2D receptor expression could produce more tumor-killing.

*Viral Shedding Profile & Potency*

While the cell-associated virus and cell-free virus profile of cell product stored at 4°C was unsurprising (markedly increasing after 48 hours), it was surprising to see no similar increase in either cell-associated or free virus in CRX100 stored at 22°C. Cell-associated and cell-free vaccinia viral titer increased after 48 hours post-infection, suggesting that the virus eclipse period (48 hours) and viral replication kinetics were maintained in CRX100 held at 4°C. This also suggested that vaccinia virus was spreading systemically after initial infection both as free virus and cell-to-cell within infected cells in CRX100 at 4°C.

Cell-free and cell-associated viral titer dropped significantly in CRX100 held at room temperature and no burst of viral replication 48 hours post-infection was observed. This suggested that at room temperature, either the vaccinia virus did not undergo complete replication process cycle in the cells or the infected cells died rapidly at room temperature. A drop in total cell count was also observed between 48 to 72 hours (FIG. 19) for CRX100 held at 22°C. Hence, a low number of virions which formed areas of clear growth (plaque) on Vero cells were observed. It is also possible that at room temperature, the vaccinia virus particles are highly unstable to undergo replication-lysis-infection process in Vero cells and form plaques, resulting in low titer of cell-free and cell-associated virus at room temperature at all time points post-infection.

This lack of viral shedding or viral replication could also play a significant role in the differences of observed OVCAR-3 killing between CRX100 held at 4°C or ambient temperature. These data importantly indicate that CRX100 stored at 4°C for 120 hours exhibits the same cytotoxic profile as CRX100 used immediately after COBE wash. This suggests that CRX100 may be safely used for a significant period of time after washing (120 hours) and retains their ability to kill tumor cells if maintained at 4°C for the duration of its hold.

### Example 6

The purpose of the study was to investigate the stability and potency of CIK-DS and CRX100 after prolonged storage at 4°C immediately after COBE washing.

CIK cells were expanded as described above. Cells were seeded at a density of $2 \times 10^6$ cell/mL seed density in 200 mL of AIM-V media in a 5L expansion bag. CIK cells were harvested on Day 21 of expansion and resuspended in Plasma-Lyte© + 0.5% Human AB serum. Cells were infected with vvDD at an MOI of 0.01 for two hours at 37°C to generate CRX100.

The CRX100 was maintained at 4°C and shipped from a manufacturing plant to the analytical laboratory. Cells were received at the laboratory about ~36 hours after harvest. A sample was removed from the bag, washed in Plasma-Lyte, and used for potency assays as described above. vvDD was thawed from -80°C and used directly for potency using OVCAR-3 tumor cells as described above.

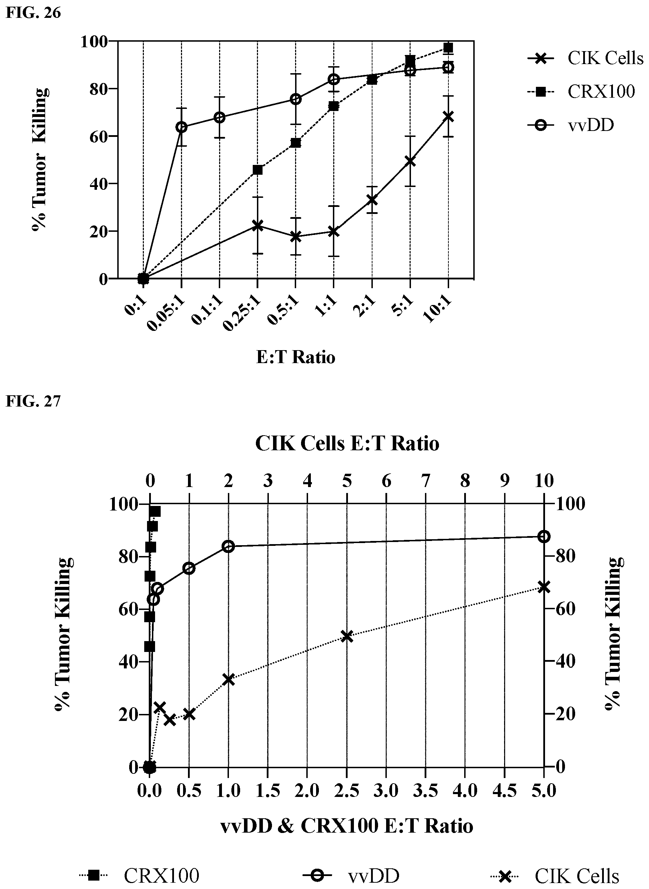

Results are shown in FIGs. 26 and 27 and show that immune cells (CIK and CRX100 maintained potency during an extended hold at 4°C. Notably, cells maintained their potency following shipping. Titers are shown in Table 29.

**Table 29.**

| MOI = 0.01 E:T Ratios | | | | |
|---|---|---|---|---|
| E:T Ratio | CIK Cells | CRX100 (Cells) | CRX100 (vvDD) | vvDD pfu |
| 0:00 | 0 | 0 | 0 | 0 |
| 0.05 | N/A | N/A | N/A | 1000 |
| 0.1 | N/A | N/A | N/A | 2000 |
| 0.25 | 5000 | 5000 | 37.5 | N/A |
| 0.5 | 10000 | 10000 | 75 | 10000 |
| 1 | 20000 | 20000 | 150 | 20000 |
| 2 | 40000 | 40000 | 300 | N/A |
| 5 | 100000 | 100000 | 750 | 100000 |
| 10 | 200000 | 200000 | 1500 | 200000 |

### Example 7

The purpose of the study was to investigate the stability and potency of CIK-DS and CRX100 after prolonged storage at 4°C, 22°C, and 37°C.

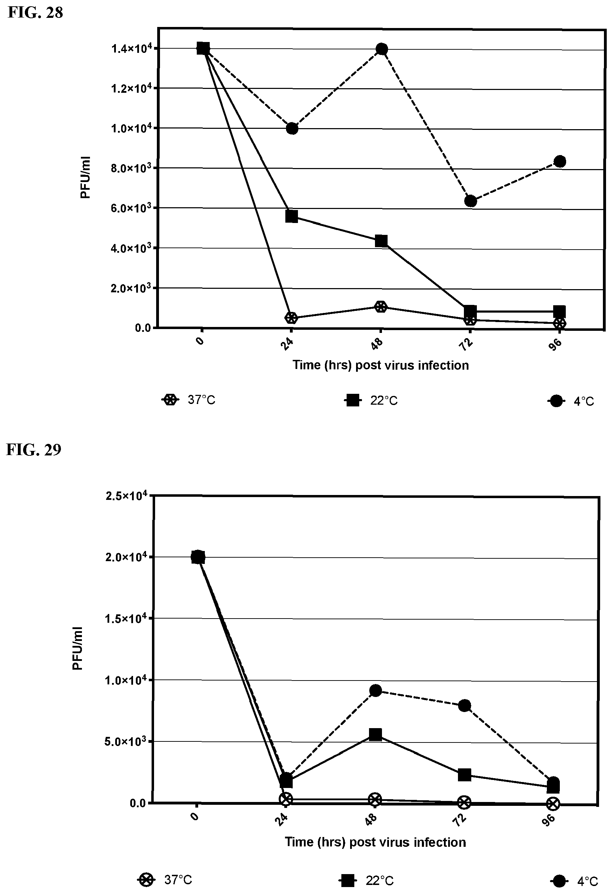

CIK cells were expanded from human patient apheresis product for 25 days, washed with Plasma-Lyte© +0.5% Human AB serum, and infected with vvDD virus (MOI 0.1) at 37°C for 2 hours. Next, CRX100 cells were held in Plasma-Lyte© at different temperatures for 96 hours post virus infection. $3 \times 10^6$ CRX100 cells from each time point and temperature were centrifuged and the cell-pellet was freeze-thawed $5\times$ to titer cell-associated virus on Vero E6 cells. Plaques were quantified after 3-4 days using 0.1% crystal violet staining. An increase in viral titer after 24 hours eclipse period was observed in CRX1000 cells held at 4°C, however, no cell-associated viral titer increase was observed for cells held at 22° and 37°C (FIG. 28). At 96 hours post-infection, an increase in viral titer was again observed in CRX100 cells held at 4°C indicating that the virus can re-infect CIK cells and replicate to increase the cell-associated viral titer of CRX100 product at 4°C.

CIK cells were expanded from patient apheresis product at BioEclipse for 25 days, washed with Plasma-Lyte© + 0.5% Human AB serum, and infected with vvDD virus at 37°C (MOI 0.1) for 2 hours. Next, CRX100 cells were held in Plasma-Lyte© at different temperatures for 96 hours post virus infection. $3 \times 10^6$ CRX100 cells from each time point and temperature were spun at 1000g for 10 mins. The supernatant was serially diluted in serum-free media to titer cell-free virus on Vero E6 cells. Plaques were quantified after 3-4 days using 0.1% crystal violet staining. CRX100 cells held at 4°C showed an increased cell-free viral titer as compared to cells held at 22° and 4°C (FIG. 29).

### Claims

1. A method of producing a stabilized population of immune cells comprising cytokine-induced killer (CIK) cells, wherein the immune cells comprise an oncolytic virus, the method comprising

a) culturing immune cells under conditions to select and enhance cytokine-induced killer cells,

b) contacting the cells with the oncolytic virus, and

c) maintaining the cells at 2°C to 8°C for more than 6 hours and up to 120 hours.

2. The method of claim 1, wherein

i) the cells of step c) are maintained at 2°C to 8°C or 4°C for 24 hours; and/or

ii) wherein the cells are removed from the culture and maintained at 2°C to 8°C prior to contacting the cells with the oncolytic virus, optionally

wherein the cells are maintained at 2°C to 8°C, or 4°C, for up to 120 hours prior to contacting the cells with the oncolytic virus.

3. The method of claim 1 or 2, wherein the immune cells are contacted with the oncolytic virus at a multiplicity of infection (MOI) of 0.1 to an MOI of 10, optionally wherein the immune cells are contacted with the oncolytic virus at 37°C for 2 hours.

4. The method of any one of any one of claims 1-3, wherein

a) the cells of step c) are maintained in <1% $CO_2$; and/or

b) wherein the cells of step c) are maintained in a salt solution, optionally wherein the salt solution is essentially free of serum and/or growth factors; and/or

c) wherein the cells of step c) are maintained in the dark.

5. The method of any one of claims 1-4, wherein

a) the stabilized population of immune cells comprise NKG2D+ cells; optionally wherein the percentage of NKG2D+ cells in the population is maintained or enhanced; and/or

b) wherein the viability of the stable population of immune cells is greater than 70%, optionally wherein the viability of said immune cells is measured by trypan blue exclusion or by flow cytometry.

6. The method of any one of claims 1-5,

a) wherein the immune cells are derived from peripheral blood mononuclear cells (PBMCs); and/or

b) wherein the immune cells of step a) are cultured in 1 liter to 10 liters of culture media, optionally in 1 liter or 5 liters of culture media; and/or

c) wherein the immune cells of step a) are cultured for 28 days, 32 days, 36 days, or 40 days.

7. The method of any one of claims 1-6, wherein the oncolytic virus is a vaccinia virus.

8. The method of claim 7,

a) wherein the vaccinia virus comprises a mutation in the viral thymidine kinase (TK) gene and/or the viral growth factor (VGF) gene; and/or

b) wherein the oncolytic virus is replication competent; and/or

(c) wherein the vaccinia virus is an EEV virus.

**Patentansprüche**

1. Verfahren zum Herstellen einer stabilisierten Population von Immunzellen, die cytokininduzierte Killer(CIK)-Zellen umfassen, wobei die Immunzellen ein onkolytisches Virus umfassen, wobei das Verfahren umfasst:

a) Kultivieren der Immunzellen unter Bedingungen, um cytokininduzierte Killerzellen auszuwählen und zu verstärken,

b) Inberührungbringen der Zellen mit dem onkolytischen Virus und

c) Halten der Zellen bei 2 °C bis 8 °C für mehr als 6 Stunden und bis zu 120 Stunden.

**2.** Verfahren nach Anspruch 1, wobei

i) die Zellen aus Schritt c) 24 Stunden lang bei 2 °C bis 8 °C oder 4 °C gehalten werden; und/oder
ii) wobei die Zellen aus der Kultur entnommen und bei 2 °C bis 8 °C gehalten werden, bevor sie mit dem onkolytischen Virus in Berührung gebracht werden, wobei optional die Zellen bei 2 °C bis 8 °C oder 4 °C bis zu 120 Stunden lang gehalten werden, bevor sie mit dem onkolytischen Virus in Berührung gebracht werden.

**3.** Verfahren nach Anspruch 1 oder 2, wobei die Immunzellen mit dem onkolytischen Virus bei einer Multiplizität der Infektion (MOI) von 0,1 bis zu einer MOI von 10 in Berührung gebracht werden, wobei optional die Immunzellen 2 Stunden lang bei 37 °C mit dem onkolytischen Virus in Berührung gebracht werden.

**4.** Verfahren nach einem der Ansprüche 1-3, wobei

a) die Zellen aus Schritt c) bei <1 % $CO_2$ gehalten werden; und/oder
b) wobei die Zellen aus Schritt c) in einer Salzlösung gehalten werden, wobei die Salzlösung optional im Wesentlichen frei von Serum und/oder Wachstumsfaktoren ist; und/oder
c) die Zellen aus Schritt c) im Dunkeln gehalten werden.

**5.** Verfahren nach einem der Ansprüche 1-4, wobei

a) die stabilisierte Population von Immunzellen NKG2D+ Zellen umfasst; optional wobei der Prozentsatz an NKG2D+ Zellen in der Population gehalten oder erhöht wird; und/oder
b) wobei die Lebensfähigkeit der stabilen Population von Immunzellen größer als 70 % ist, optional wobei die Lebensfähigkeit der Immunzellen durch Trypanblau-Ausschluss oder durch Durchflusszytometrie gemessen wird.

**6.** Verfahren nach einem der Ansprüche 1-5,

a) wobei die Immunzellen aus peripheren mononukleären Blutzellen (PBMCs) abgeleitet werden; und/oder
b) wobei die Immunzellen aus Schritt a) in 1 Liter bis 10 Liter Kulturmedium kultiviert werden, optional in 1 Liter oder 5 Liter Kulturmedium; und/oder
c) wobei die Immunzellen aus Schritt a) 28 Tage, 32 Tage, 36 Tage oder 40 Tage lang kultiviert werden.

**7.** Verfahren nach einem der Ansprüche 1-6, wobei das onkolytische Virus ein Vacciniavirus ist.

**8.** Verfahren nach Anspruch 7,

a) wobei das Vacciniavirus eine Mutation in dem viralen Thymidinkinase(TK)-Gen und/oder in dem viralen Wachstumsfaktor(VGF)-Gen umfasst; und/oder
b) wobei das onkolytische Virus replikationskompetent ist; und/oder
(c) wobei das Vacciniavirus ein EEV-Virus ist.

**Revendications**

**1.** Procédé de production d'une population stabilisée de cellules immunitaires comprenant des cellules tueuses induites par les cytokines (CIK), lesdites cellules immunitaires comprenant un virus oncolytique, ledit procédé comprenant :

a) la mise en culture de cellules immunitaires dans des conditions permettant de sélectionner et de développer des cellules tueuses induites par la cytokine,
b) la mise en contact des cellules avec le virus oncolytique, et
c) le maintien des cellules à une température de 2 °C à 8 °C pendant plus de 6 heures et jusqu'à 120 heures.

**2.** Procédé selon la revendication 1, dans lequel

i) les cellules de l'étape c) sont maintenues à une température de 2 °C à 8 °C, ou de 4°C, pendant 24 heures, et/ou
ii) lesdites cellules sont retirées de la culture et maintenues à une température de 2° C à 8° C avant d'être mises en contact avec le virus oncolytique,

lesdites cellules étant facultativement maintenues à une température de 2 °C à 8 °C, ou de 4 °C, pendant une période allant jusqu'à 120 heures avant d'être mises en contact avec le virus oncolytique.

3. Procédé selon la revendication 1 ou 2, dans lequel les cellules immunitaires sont mises en contact avec le virus selon une multiplicité d'infection (MOI, multiplicity of infection) allant de 0,1 à 10, lesdites cellules immunitaires étant mises en contact avec le virus oncolytique à une température de 37 °C pendant 2 heures.

4. Procédé selon l'une quelconque des revendications 1 à 3, dans lequel

   a) les cellules de l'étape c) sont maintenues dans <1 % de $CO_2$, et/ou
   b) les cellules de l'étape c) sont maintenues dans une solution saline, ladite solution saline étant facultativement essentiellement exempte de sérum et/ou de facteurs de croissance, et/ou
   c) les cellules de l'étape c) sont maintenues dans le noir.

5. Procédé selon l'une quelconque des revendications 1 à 4, dans lequel

   a) la population stabilisée de cellules immunitaires comprend des cellules NKG2D+, le pourcentage desdites cellules NKG2D+ de ladite population étant éventuellement conservé ou augmenté, et/ou
   b) la viabilité de la population stable de cellules immunitaires est supérieure à 70 %, ladite viabilité desdites cellules immunitaires étant facultativement mesurée par exclusion au bleu de trypan ou par cytométrie en flux.

6. Procédé selon l'une quelconque des revendications 1 à 5,

   a) dans lequel les cellules immunitaires sont dérivées de cellules mononucléées du sang périphérique (PBMC, peripheral blood mononuclear cells),
   et/ou
   b) dans lequel les cellules immunitaires de l'étape a) sont mises en culture dans un volume allant de 1 litre à 10 litres de milieu de culture, facultativement de 1 litre à 5 litres de milieu de culture, et/ou
   c) dans lequel les cellules immunitaires de l'étape a) sont mises en culture pendant 28 jours, 32 jours, 36 jours ou 40 jours.

7. Procédé selon l'une quelconque des revendications 1 à 6, dans lequel le virus oncolytique est un virus de la vaccine.

8. Procédé selon la revendication 7,

   a) dans lequel le virus de la vaccine comprend une mutation dans le gène de la thymidine-kinase (TK) virale et/ou le gène du facteur de croissance viral (VGF, viral growth factor), et/ou
   b) dans lequel le virus oncolytique est compétent pour la réplication, et/ou
   c) dans lequel le virus de la vaccine est un virus EEV.

**FIG. 1**

**FIG. 2**

## CIK Cell Substance Stored at Room Temperature

| Time Point (hours) | Viable Cell Density (cells/mL) | Viability (%) | Flow Viability (%) |
|---|---|---|---|
| 0 | 1.01E+07 | 96 | 96.19 |
| 6 | 9.76E+06 | 96 | 96.26 |
| 22 | 8.58E+06 | 93 | 95.40 |
| 53 | 5.38E+06 | 92 | 87.05 |
| 77 | 6.06E+06 | 85.8 | 61.80 |

**FIG. 3**

CIK Cell Substance Stored at 37°C

| Time Point (hours) | Viable Cell Density (cells/mL) | Viability (%) | Flow Viability (%) |
|---|---|---|---|
| 0 | 1.03E+07 | 97 | 96.19 |
| 53 | 2.81E+06 | 27 | 0.21 |
| 77 | 3.78E+06 | 51.8 | 0.02 |

**FIG. 4**

## CIK Cell Substance Stored at Room Temperature

| Time Point (hours) | Viability (%) | CD3+/CD56+ (%-#) | CD3+ (%-#) |
|---|---|---|---|
| 0 | 96 | 5.11 | 98.32 |
| 6 | 96 | 6.47 | 98.15 |
| 22 | 93 | 6.27 | 98.58 |
| 53 | 92 | 4.52 | 98.0 |
| 77 | 86 | 3.62 | 98.02 |

FIG. 5

**CIK Cell Substance Stored at Room Temperature**

| Time Point (hours) | Viability (%) | CD3+/CD56+ (%-#) | CD3+ (%-#) |
|---|---|---|---|
| 0 | 96 | 5.11 | 98.32 |
| 6 | 96 | 6.47 | 98.15 |
| 22 | 93 | 6.27 | 98.58 |
| 53 | 92 | 4.52 | 98.0 |
| 77 | 86 | 3.62 | 98.02 |

**FIG. 6**

**CIK Cell Substance Stored at 37°C**

| Time Point (hours) | CD3+/CD56+ (%) | CD3+ (%) |
|---|---|---|
| 0 | 5.11 | 98.32 |
| 53 | 7.34 | 96.0 |
| 77 | 5.56 | 61.12 |

**FIG. 7**

**CIK Cells Infected with Low Dose of vvDD Stored at Room Temperature**

| Time Point (hours) | Viability (%) | CD3+/CD56+ (%) | CD3+ (%) |
|---|---|---|---|
| 0 | 95 | 5.69 | 98.28 |
| 6 | 95 | 6.03 | 98.12 |
| 17 | 95 | 6.22 | 98.61 |
| 23 | 91 | 5.90 | 98.12 |
| 46 | 85.5 | 6.66 | 98.62 |
| 70 | 83.5 | 3.07 | 97.92 |

**FIG. 8**

**CIK Cells Infected with High Dose of vvDD Stored at Room Temperature**

| Time Point (hours) | Viability (%) | CD3+/CD56+ (%) | CD3+ (%) |
|---|---|---|---|
| 0 | 94 | 5.49 | 98.36 |
| 6 | 96 | 5.86 | 98.0 |
| 17 | 94 | 6.09 | 98.83 |
| 23 | 91 | 5.78 | 98.27 |
| 46 | 90.5 | 4.67 | 98.37 |
| 70 | 71.5 | 4.41 | 96.72 |

**FIG. 9**

**CIK Cells Infected with High Dose of vvDD Stored at 37°C**

| Time Point (hours) | Viability (%) | CD3+/CD56+ (%) | CD3+ (%) |
|---|---|---|---|
| 0 | 96 | 6.14 | 98.16 |
| 17 | 56 | 16.40 | 93.28 |
| 23 | 41 | 40.00 | 82.63 |
| 46 | 37.5 | | |
| 70 | 43.5 | | |

FIG. 10

Degranulation of CIK Cells Co-Incubated with Tumor Cells

| Time point (hours) | Total Cell Density (cells/mL) | Flow Viability (%) | CD107a+ (%) |
|---|---|---|---|
| 0 | 0.5E+06 | 95.19% | 0.55% |
| | 20E+06 | 57.06% | 0.00% |
| 24 | 0.5E+06 | 94.83% | 0.47% |
| | 20E+06 | 26.35% | 4.1% |

**FIG. 11A**

**FIG. 11B**

FIG. 11C

**FIG. 12A**

Pooled Samples

**FIG. 12B**

Pooled Samples

**FIG. 12C**

**FIG. 13A**

Pooled Samples

**FIG. 13B**

Pooled Samples

**FIG. 13C**

Pooled Samples

**FIG. 13D**

Pooled Samples

**FIG. 14A**

**FIG. 14B**

**FIG. 15A**

**FIG. 15B**

FIG. 16A

FIG. 16B

**FIG. 16C**

**FIG. 16D**

**FIG. 17A**

**FIG. 17B**

**FIG. 17C**

**FIG. 17D**

**FIG. 18A**

**FIG. 18B**

**FIG. 19**

**FIG. 20A**

**FIG. 20B**

**FIG. 20C**

**FIG. 20D**

**FIG. 21A**

**FIG. 21B**

**FIG. 21C**

**FIG. 21D**

**FIG. 22A**

**FIG. 22B**

**FIG. 22C**

**FIG. 23A**

Time (Hours) Post-Virus Infection

⊟ 4°C CRX100 (Cell-Associated)          ■ 4°C CRX100 (Cell-Free)

**FIG. 23B**

Time (Hours) Post-Virus Infection

⊖ 22°C CRX100 (Cell-Associated)          ● 22°C CRX100 (Cell-Free)

**FIG. 24A**

**FIG. 24B**

FIG. 25

**FIG. 26**

**FIG. 27**

**FIG. 28**

Time (hrs) post virus infection

⊗ 37°C          ■ 22°C          ● 4°C

**FIG. 29**

Time (hrs) post virus infection

⊗ 37°C          ■ 22°C          ● 4°C

**EP 4 114 925 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9101658 B
- US 10064893 B
- US 2013028875 A1
- WO 2020014029 A1
- US 4603112 A, Paoletti

### Non-patent literature cited in the description

- **LAPORT, GG et al.** *Biol Blood Marrow Transplant.*, 2012, vol. 17 (11), 1679-1687
- **BREMM et al.** *Front. Immunol.*, 2019, vol. 10, 1218
- **BONANNO et al.** *J. Transl. Med.*, 2010, vol. 8, 129
- **THORNE et al.** *Science*, 2006, vol. 311 (5768), 1780-4
- **SAMBROOK et al.** Molecular Cloning: A Laboratory Manual. 1989
- Oligonucleotide Synthesis. 1984
- Animal Cell Culture. 1987
- Methods in Enzymology. Academic Press, Inc.
- Handbook of Experimental Immunology
- Gene Transfer Vectors for Mammalian Cells. 1987
- Current Protocols in Molecular Biology. 1987
- PCR: The Polymerase Chain Reaction. 1994
- Current Protocols in Immunology. 1991
- **ROIZMAN** ; **JENKINS**. *Science*, 1985, vol. 229, 1208
- **AUSUBEL et al.** Current Protocols in Molecular Biology. John Wiley and Sons, Inc.
- **COEN**. Virology. Raven Press, 1990, 123-150
- **GLIENKE J. et al.** *Immunogenetics*, 1998, vol. 48, 163-173
- **ZINGONI A. et al.** *Front. Immunol.*, 12 March 2018
- **BOLLAG et al.** *Cancer Research*, 1995, vol. 55 (11), 2325-2333
- Remington's Pharmaceutical Sciences. Mack Publishing, 1990

86